# EUROPEAN PATENT APPLICATION

(11) **EP 4 810 041 A1**
(43) Date of publication of application: **23.09.2026**
(21) Application number: 24890826.1
(22) Date of filing: 15.11.2024
(51) Int. Cl.: A61K 31/4745, A61K 31/585, A61K 47/68, A61P 35/00

(54) **DOUBLE-TOXIN ANTIBODY-DRUG CONJUGATE AND USE THEREOF**

(30) Priority: 17.11.2023 CN 202311543915; 21.05.2024 CN 202410631122
(71) Applicant: Chengdu Kanghong Biotechnologies Co., Ltd., Chengdu, Sichuan 610036 (CN)
(72) Inventor: KE, Xiao, Chengdu, Sichuan 610036 (CN); ZHAO, Yonghao, Chengdu, Sichuan 610036 (CN); REN, Pengfei, Chengdu, Sichuan 610036 (CN); LEI, Gang, Chengdu, Sichuan 610036 (CN); ZUO, Teng, Chengdu, Sichuan 610036 (CN)
(74) Representative: dompatent
(86) International application number: PCT/CN2024/132357
(87) International publication number: WO 2025/103474

(57) **Abstract**

Provided are a double-toxin antibody-drug conjugate, and use of the antibody-drug conjugate in preparing a drug for treating cancer.

## Description

### CROSS-REFERENCE TO RELATED APPLICATIONS

The present application claims the priority and benefit of Chinese Patent Application No. 202311543915.2 and Chinese Patent Application No. 202410631122.4, which are incorporated herein by reference in their entireties.

### TECHNICAL FIELD

The present disclosure relates to the field of chemical medicine, in particular to a double-toxin antibody-drug conjugate and use thereof.

### BACKGROUND

As a novel targeted therapeutic drug, antibody-drug conjugates (ADCs) combine the specificity of antibodies in binding to antigens on the surfaces of normal cells and tumor cells with the high potency of cytotoxins. They can effectively deliver cytotoxins to diseased sites in a targeted manner to exert efficacy, while mitigating the drawback of excessive side effects associated with cytotoxins in conventional drug forms. Accordingly, ADCs are increasingly playing an important role in the treatment of tumors and other fields.

An ADC drug consists of three components: a monoclonal antibody, a linker, and a cytotoxic drug (cytotoxin). Most ADC drugs reported to date are antibody-drug conjugates comprising a single toxin, whereas reports on antibody-drug conjugates comprising double toxins are relatively rare.

### SUMMARY OF THE INVENTION

In a first aspect, the present disclosure provides use of a combination of triptolide and a camptothecin drug in preparing a medicament for treating a tumor disease.

In some specific embodiments, triptolide and the camptothecin drug are used together as toxins in an antibody-drug conjugate.

In a second aspect, the present disclosure provides an antibody-drug conjugate comprising double toxins, which has a formula of: 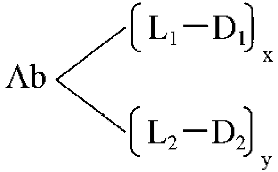
wherein Ab is an antibody or an antigen-binding fragment thereof;
D₁ and D₂ are selected from the group consisting of triptolide and a camptothecin drug, and D₁ and D₂ are different;
L₁ and L₂ are linking units;
x and y are 0-8.

In some specific embodiments, L₁ is linked to Ab via a sulfhydryl or amide group. In some other specific embodiments, L₂ is linked to Ab via glycosyl.

In some specific embodiments, L₁ has a structure represented by [H₁-L₁ₐ-L_{1b}-L_{1c}-D₁]ₓ. In some other specific embodiments, L₂ has a structure represented by [H₂-L₂ₐ-L_{2b}-L_{2c}-D₂]_{y}. In some specific embodiments, H₁ and H₂ are linker moieties for Ab or groups capable of reacting with Ab. In some other specific embodiments, L₁ₐ is a linking unit that links H₁ and L_{1b}, and L₂ₐ is a linking unit that links H₂ and L_{2b}. In some specific embodiments, L_{1b} is a linker that links L₁ₐ and L_{1c}, and L_{2b} is a linker that links L₂ₐ and L_{2c}. In some specific embodiments, L_{1c} is a spacer between L_{1b} and D₁, and L_{2c} is a spacer between L_{2b} and D₂.

In some specific embodiments, H₁ and H₂ are selected from the group consisting of: 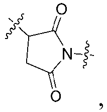 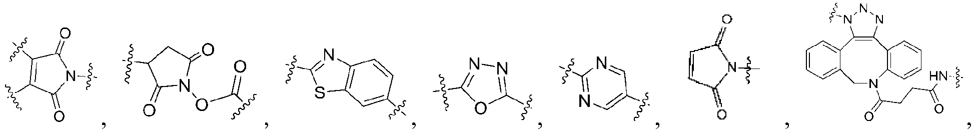 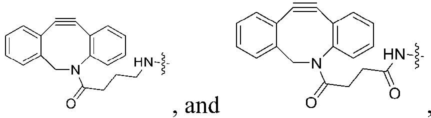 wherein 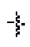 represents a linking site.

In some other specific embodiments, H₁ is selected from the group consisting of: 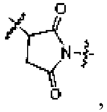 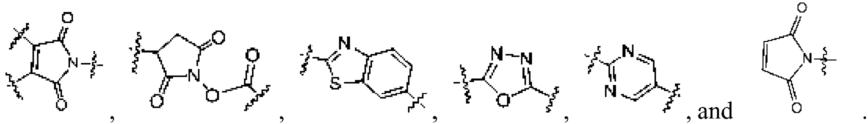 In a preferred embodiment H₁ is 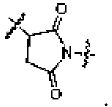 In some specific embodiments. H₂ is selected from the group consisting of:

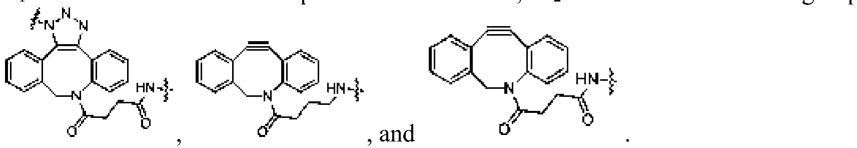

In some specific embodiments, H₁ and H₂ are selected from the group consisting of: 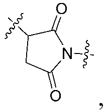 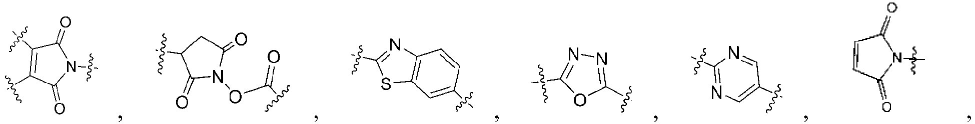 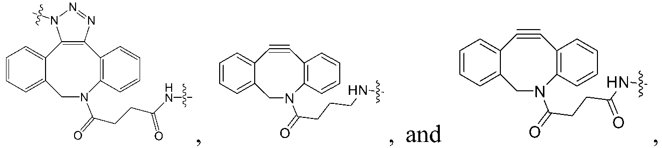 wherein 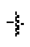 represents a linking site. In some other specific embodiments, H₂ is selected from the group consisting of:

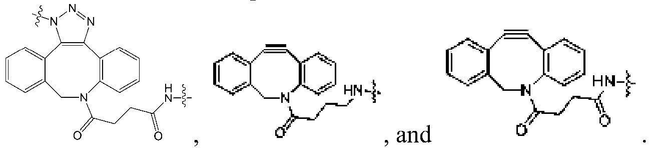

In some specific embodiments, L₁ₐ or L₂ₐ comprises -L_{d}-C(O)-, wherein L_{d} is selected from the group consisting of optionally substituted alkylene, an optionally substituted polyethylene glycol group, optionally substituted alkenylene, optionally substituted alkynylene, optionally substituted aliphatic cyclylene, optionally substituted aliphatic heterocyclylene, optionally substituted arylene, optionally substituted heteroarylene, and a combination thereof. In some other specific embodiments, L_{d} is selected from the group consisting of optionally substituted C₁₋₃₀ alkylene, an optionally substituted polyethylene glycol group, optionally substituted C₂₋₃₀ alkenylene, optionally substituted C₂₋₃₀ alkynylene, optionally substituted C₃₋₃₀ aliphatic cyclylene, optionally substituted C₁₋₃₀ aliphatic heterocyclylene, optionally substituted C₆₋₃₀ arylene, optionally substituted C₅₋₃₀ heteroarylene, and a combination thereof. In some specific embodiments, L_{d} is selected from the group consisting of - (CH₂)m-, -(PEG)n-, and -(CH₂)ₘ-(PEG)ₙ-(CH₂)_{z}-, wherein m, n, and z are integers of 0-10, and preferably, m, n, and z are integers of 0-8.

In some specific embodiments, L_{1b} or L_{2b} is a cleavable linker or a non-cleavable linker. In some specific embodiments, L_{1b} or L_{2b} is a cleavable peptide chain consisting of 2-10 amino acids. In a preferred embodiment, L_{1b} or L_{2b} is selected from the group consisting of Gly-Gly-Phe-Gly, Val-Cit, Val-Ala, Val-Lys, Val-Arg, AcLys-Val-Cit, AcLys-Val-Ala, Phe-Cit, Phe-Ala, Phe-Lys, Phe-Arg, Glu-Val-Ala, Glu-Val-Cit, Ala-Lys, Leu-Cit, Ile-Cit, Trp-Cit, Ala-Ala-Asn, Ala-Ala-Ala, Ala-Asn, and Lys. In a further preferred embodiment, L_{1b} or L_{2b} is selected from the group consisting of Gly-Gly-Phe-Gly, Val-Cit, Val-Ala, Val-Lys, Phe-Cit, Phe-Ala, Phe-Lys, and Ala-Ala-Asn.

In some specific embodiments, L_{1c} or L_{2c} is selected from the group consisting of: 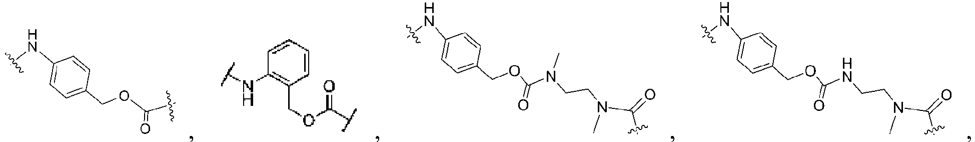 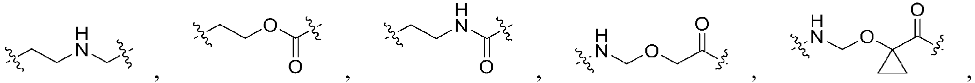 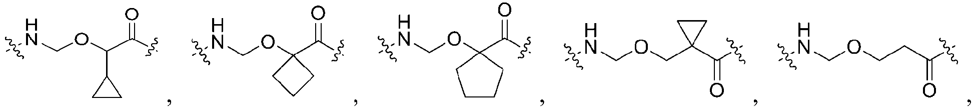 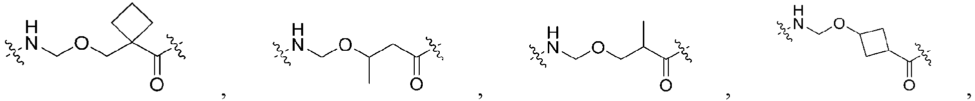 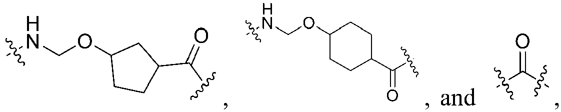 wherein 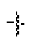 represents a linking site. In a preferred embodiment, L_{1c} is selected from the group consisting of 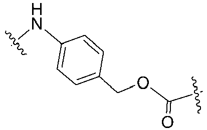 and

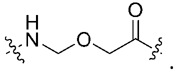

In some specific embodiments, L₁-D₁ is 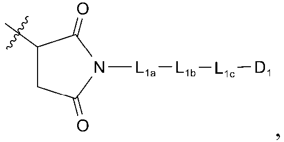 wherein L₁ₐ is -(CH₂)ₘ₁-C(O)- or -(PEG)ₙ₁-(CH₂)_{z1}-C(O)-, wherein m₁, n₁, and z₁ are integers of 2-8; L_{1b} is Gly-Gly-Phe-Gly, Val-Cit, or Val-Ala; L_{1c} is 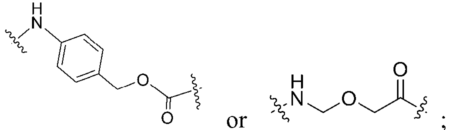 D₁ is triptolide or a camptothecin drug.

In some specific embodiments, L₂-D₂ is 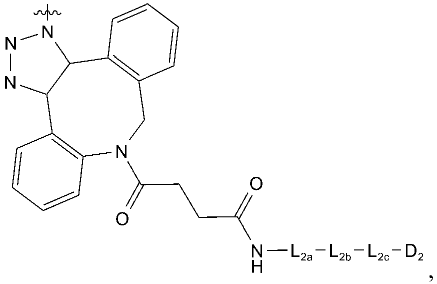 wherein: L₂ₐ is -(PEG)ₙ₂-(CH₂)_{z2}-C(O)-, wherein n₂ and z₂ are integers of 2-8; L_{2b} is Gly-Gly-Phe-Gly, Val-Cit, or Val-Ala; L_{2c} is 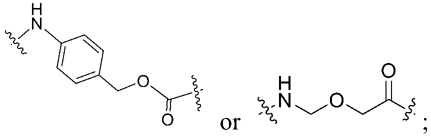 D₂ is triptolide or a camptothecin drug.

In some specific embodiments, L₂-D₂ is 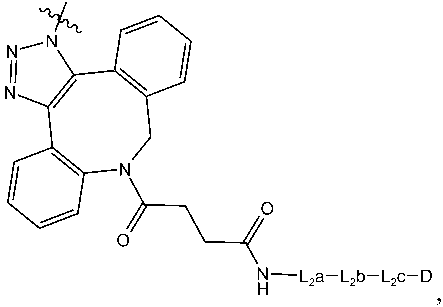 wherein: L₂ₐ is -(PEG)ₙ₂-(CH₂)_{z2}-C(O)-, wherein n₂ and z₂ are integers of 2-8; L_{2b} is Gly-Gly-Phe-Gly, Val-Cit, or Val-Ala; L_{2c} is 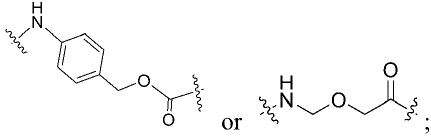 D₂ is triptolide or a camptothecin drug.

In some specific embodiments, L₁ₐ is -(CH₂)ₘ₁C(O)-, wherein m₁ is an integer of 2-6, preferably 5. In some other specific embodiments, L₁ₐ is -(PEG)ₙ₁-(CH₂)_{z1}-C(O)-, wherein n₁ and z₁ are integers of 2-6, and preferably, n₁ and z₁ are 2.

In some specific embodiments, L₂ₐ is -(PEG)ₙ₂-(CH₂)_{z2}-C(O)-, wherein n₂ and z₂ are integers of 2-6. In some other specific embodiments, L₁ₐ is -(PEG)ₙ₁-(CH₂)_{z1}-C(O)-, wherein n₁ and z₁ are integers of 2-6; preferably, n₂ is an integer of 4-6, and z₂ is 2.

In some specific embodiments, D₁ is triptolide, and D₂ is a camptothecin drug. In some other specific embodiments, D₁ is a camptothecin drug, and D₂ is triptolide. In some specific embodiments, the camptothecin drug is camptothecin, exatecan, topotecan, SN38, or a derivative thereof. In some other specific embodiments, the camptothecin drug is exatecan.

In some specific embodiments, x is selected from 2-8, preferably 3-5; y is selected from 3-4.

In some specific embodiments, x + y ≥ 4, preferably, 4 ≤ x + y ≤ 9, and more preferably 7 ≤ x + y ≤ 8.

In some specific embodiments, L₁-D₁ is selected from the group consisting of:

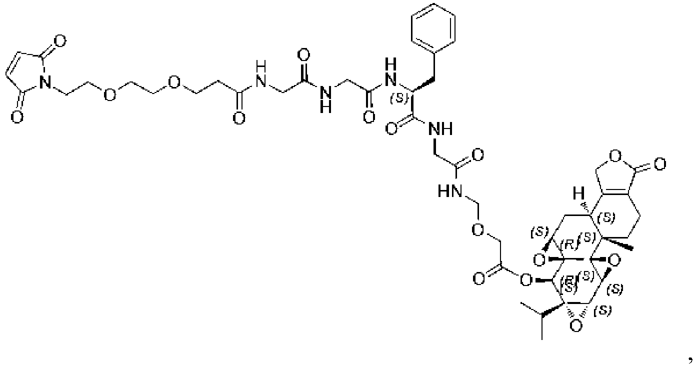

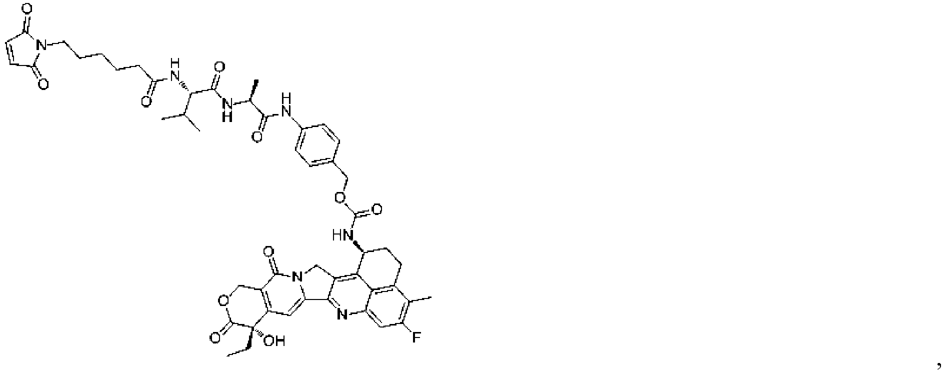

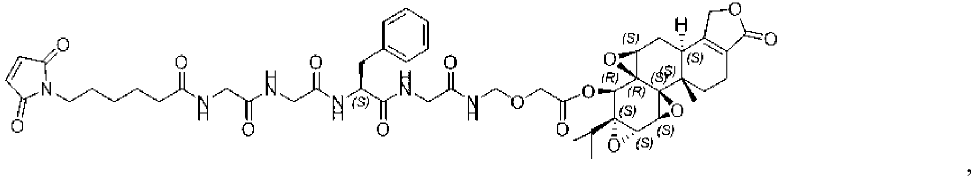

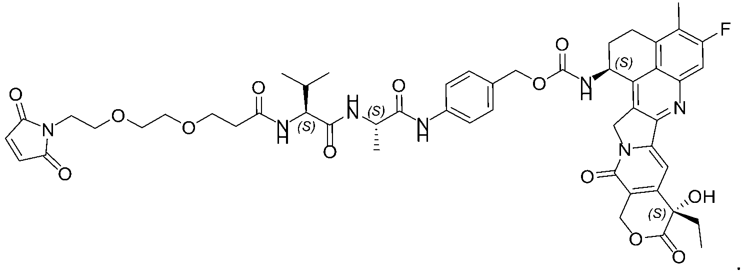

In some more specific embodiments, L₁-D₁ is selected from the group consisting of:

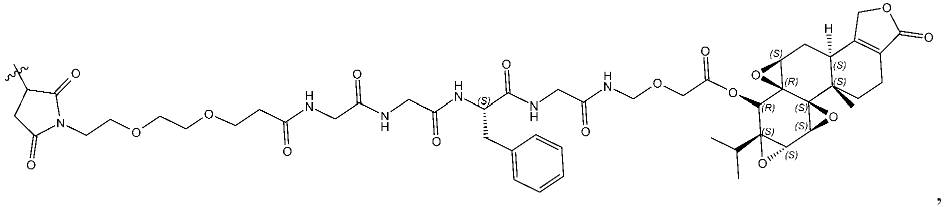

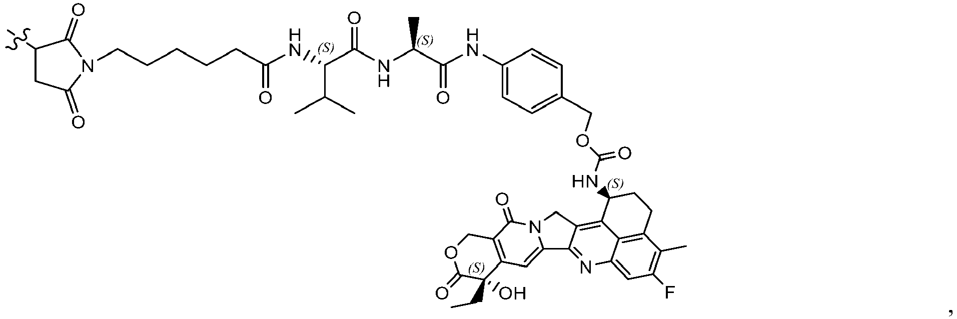

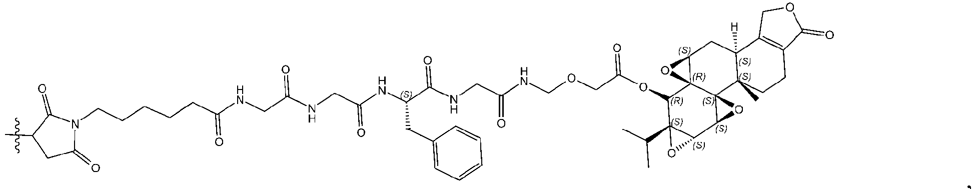

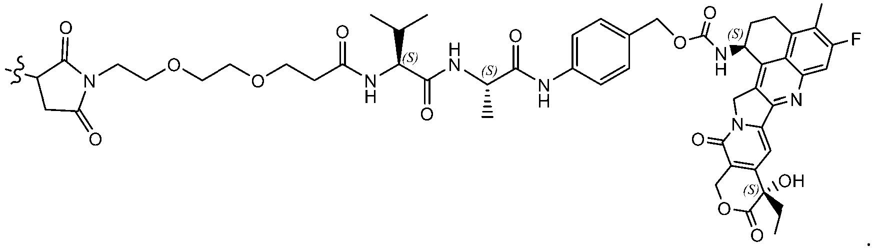

In some specific embodiments, L₂-D₂ is selected from the group consisting of:

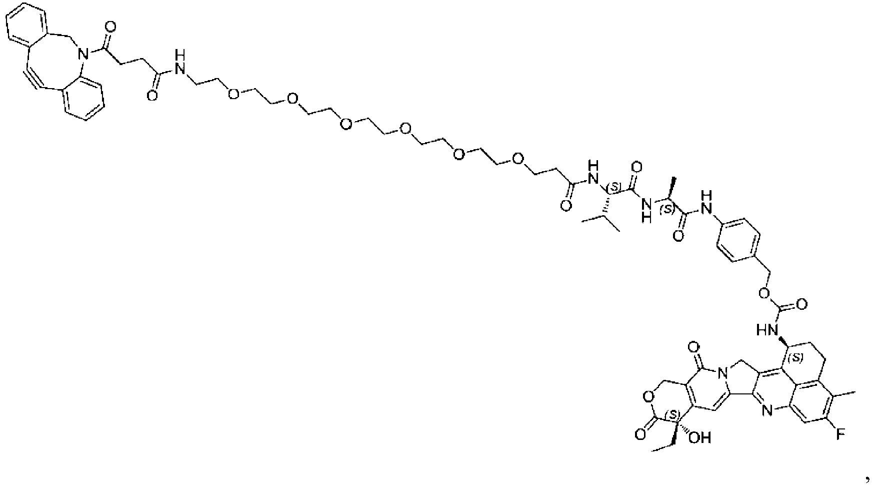

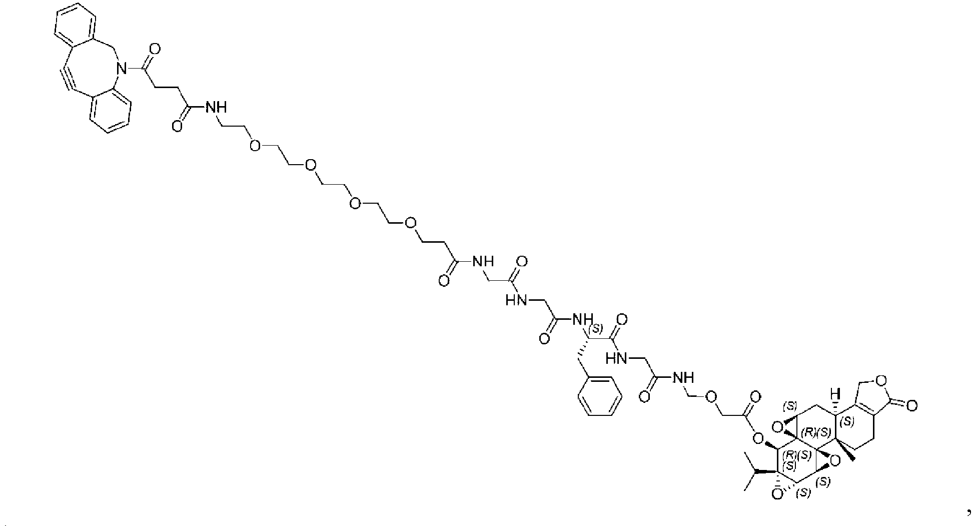

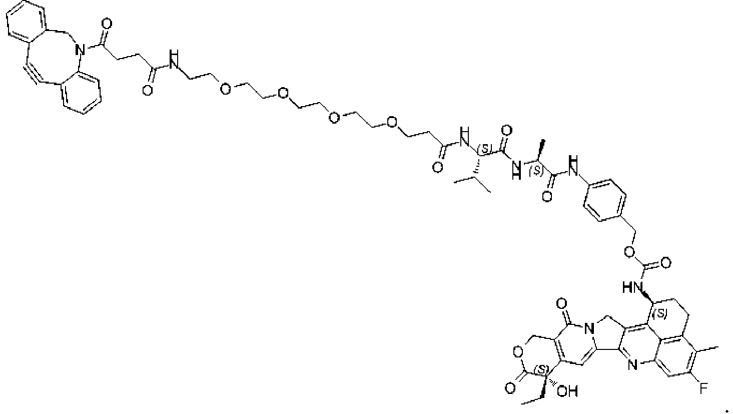

In some more specific embodiments, L₂-D₂ is selected from the group consisting of:

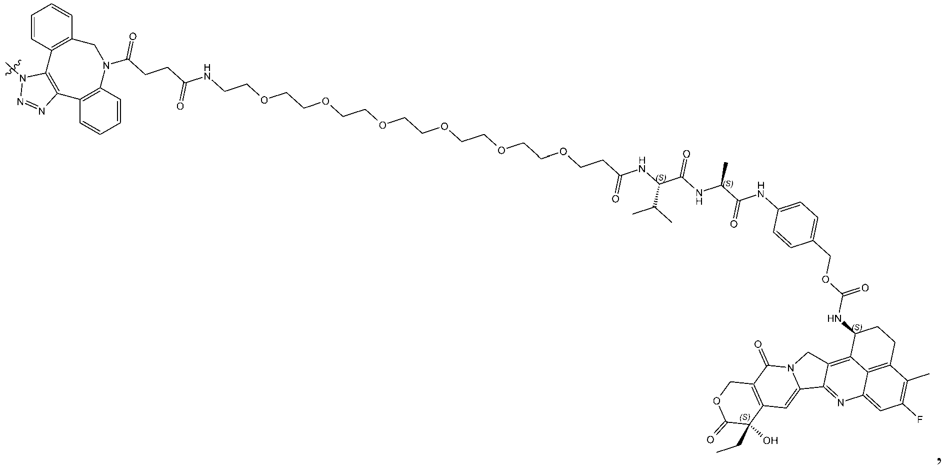

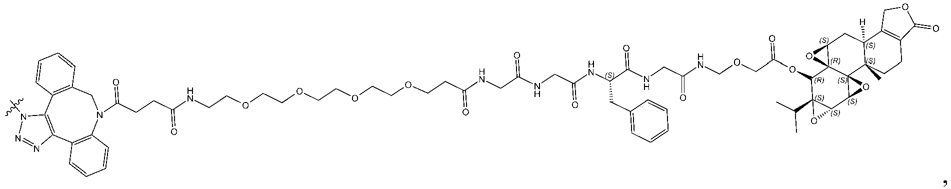

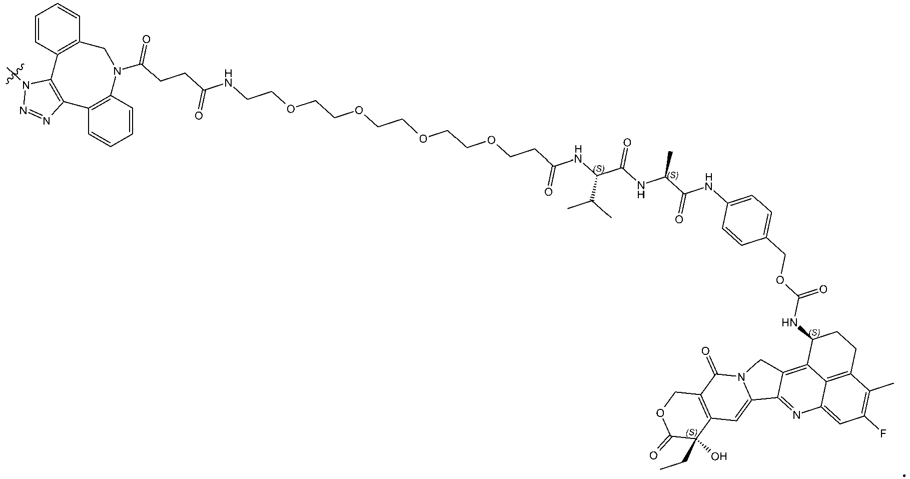

In some specific embodiments, L₁-D₁ is: 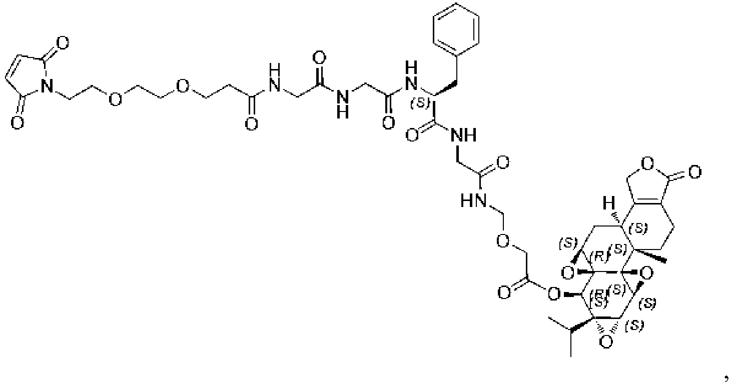 and
L₂-D₂ is:

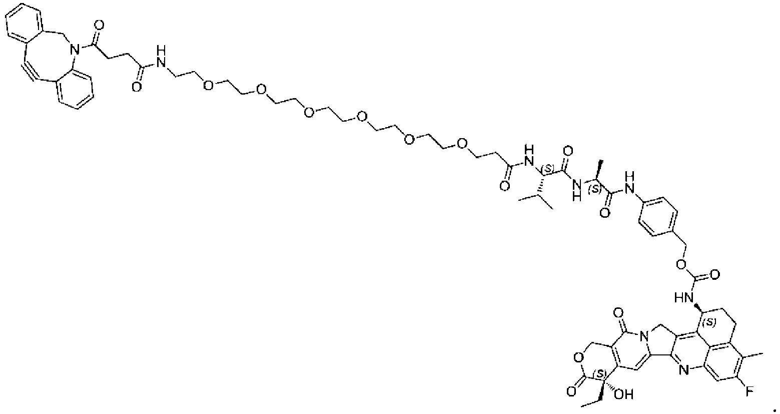

In some specific embodiments, L₁-D₁ is: 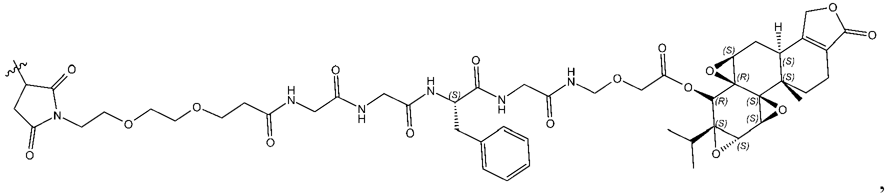 and
L₂-D₂ is:

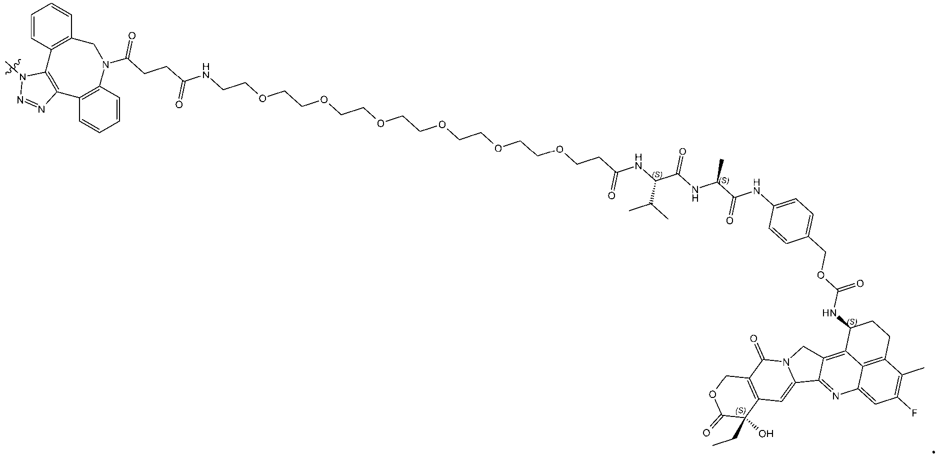

In some specific embodiments, L₁-D₁ is: 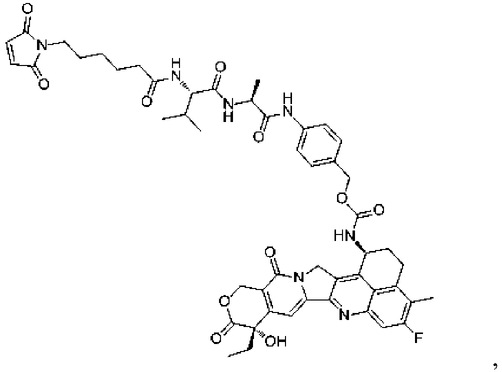 and
L₂-D₂ is:

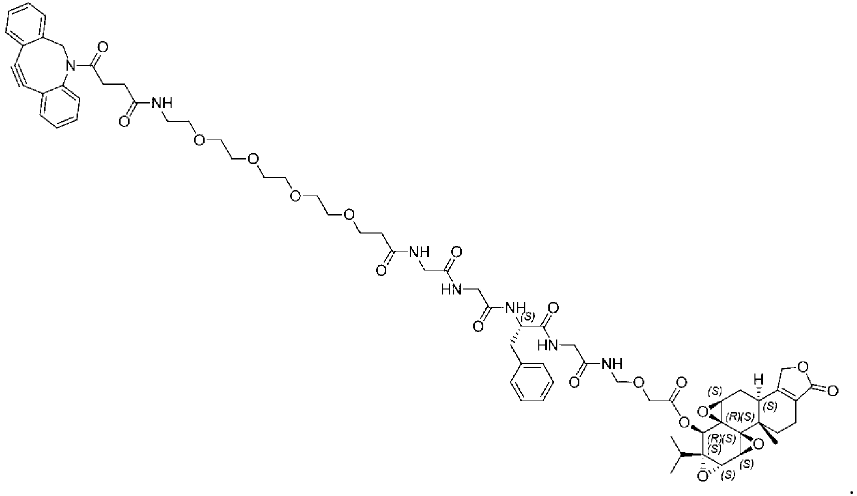

In some specific embodiments, L₁-D₁ is: 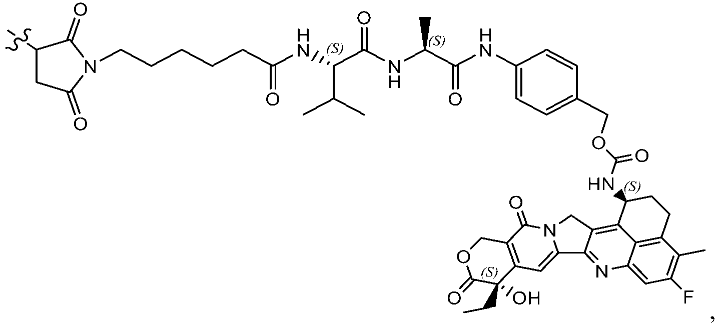 and
L₂-D₂ is:

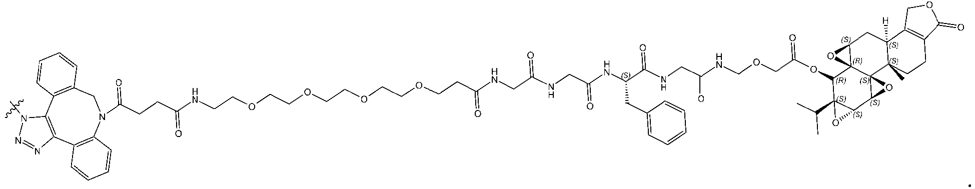

In some specific embodiments, L₁-D₁ is: 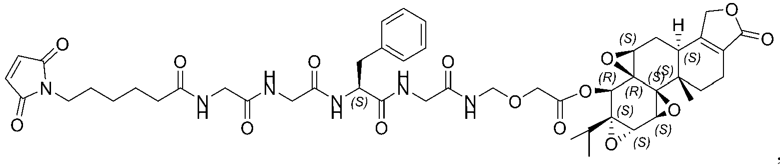 and
L₂-D₂ is:

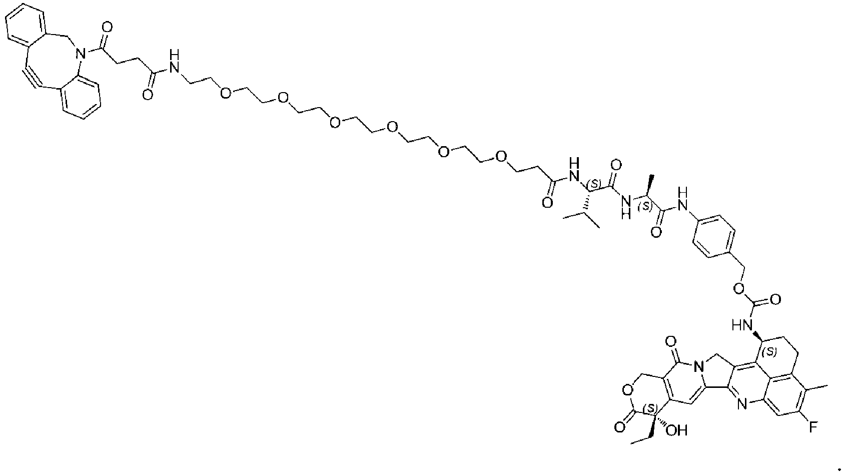

In some specific embodiments, L₁-D₁ is: 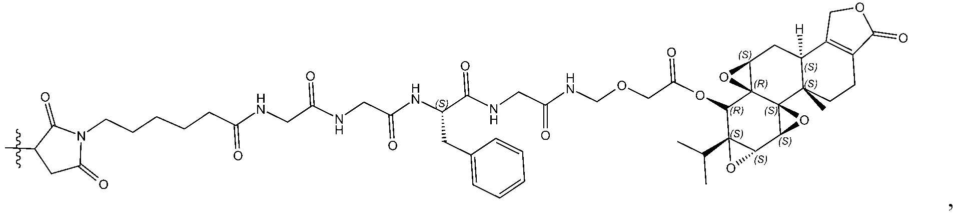 and
L₂-D₂ is:

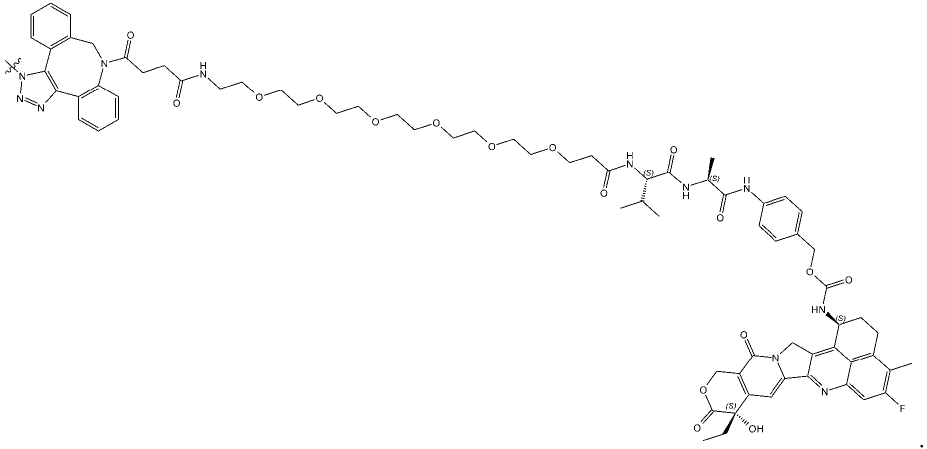

In some specific embodiments, L₁-D₁ is: 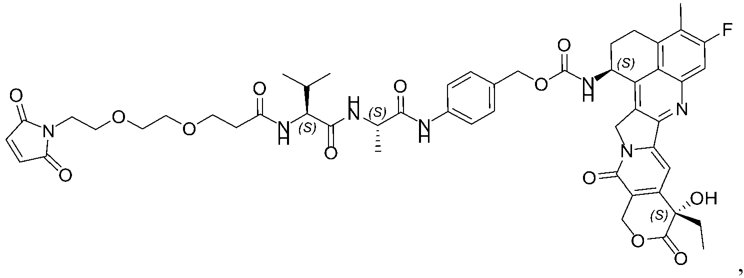 and
L₂-D₂ is:

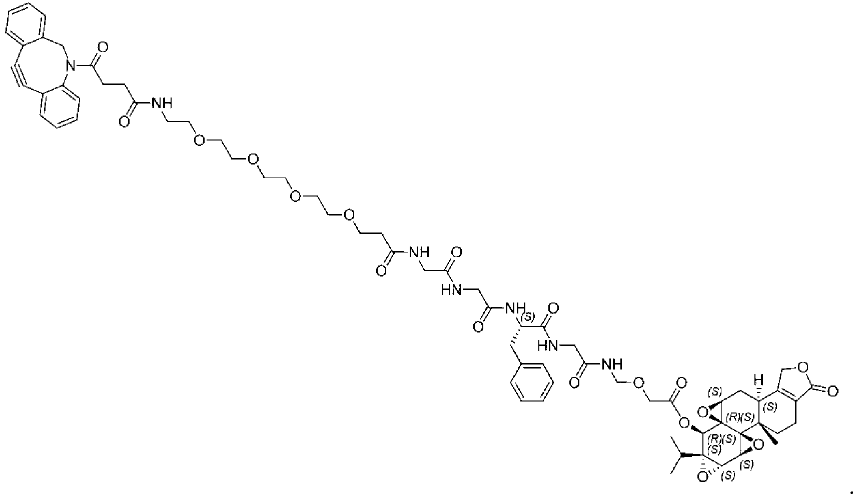

In some specific embodiments, L₁-D₁ is: 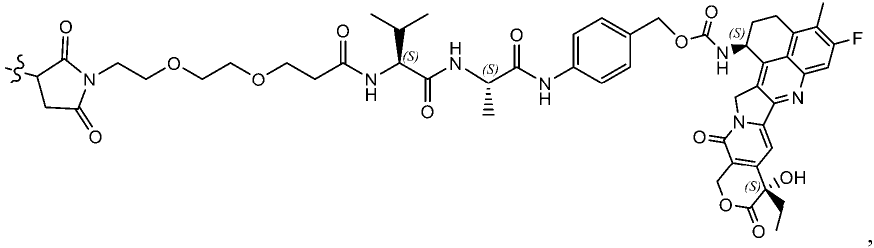 and
L₂-D₂ is:

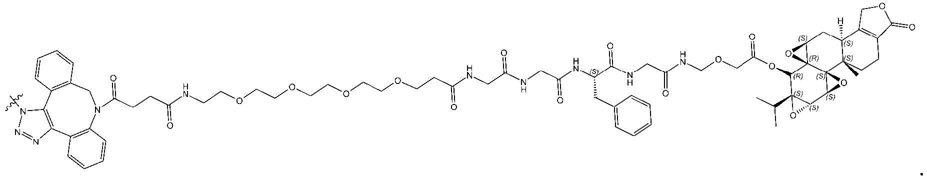

In some embodiments, L₂ is linked to the antibody via an oligosaccharide, which is located in an Fc fragment of the antibody and is specifically linked to an asparagine residue of the Fc fragment. In some embodiments, glycoforms that can be conjugated include GO-GN, G0F-GN, G0, G0F, G1, G1F, and the like. In some preferred embodiments, the glycoform of the oligosaccharide is G0, G0F, and/or G1F. In some embodiments, the oligosaccharide on the antibody is modified with azide. In some preferred embodiments, the azide modification comprises: contacting the antibody with UDP-GalNAz and GalT1. In some other embodiments, the azide-modified antibody is linked to DBCO on L₂ via a coupling reaction. In some other preferred embodiments, the coupling reaction comprises: subjecting the azide-modified antibody and DBCO on L₂ to a click chemistry reaction to accomplish glycosyl conjugation between L₂ and the antibody.

In some specific embodiments, the antibody or the antigen-binding fragment binds to one or more selected from the following: carbonic anhydrase IX, α-fetoprotein, α-actinin, A3, A33, ART 4, B7, B7H3, B7H4, BAGE, a BrE3 antigen, CA125, CAMEL, CAP", CASP-8/m, CCL19, CCL21, CD1, CDla.CD2, CD3, CD4, CD5, CD8, CD11A, CD14, CD15, CD16, CD18, CD19, CD20, CD21, CD22, CD23, CD24, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4, CDKN2A, HIF-Iα, colon-specific antigen p (CSAp), CEA, CEACAM5, CEACAM6, oMet, DAM, EGFR, EGFRvIII, cMet, EGP-1 (Trop-2), EGP-2, ELF2-M, Ep-CAM, Her2, Her3, Claudin 18.2, ROR1, ROR2, dll3, marcl7, a fibroblast growth factor (FGF), Flt-1, Flt-3, a folate receptor, a G250 antigen, GAGE, gp100, GRO-β, HLA-DR.HML24, HMGB-1, HSP70-2M., IGF-1R, IGR1R, MAGE, MAGE-3, MART-1, MART-2, NY-ESO-1, TRAG3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, a mucin in pancreatic cancer, a PD-1 receptor, a PD-L1 receptor, a placental growth factor, PLAGL2, prostatic acid phosphatase, PSA, PRAME, PSMA, P1GF, ILGF, ILGF-1R, RS5, RANTES, T101, SAGE, S100, survivin, survivin-2B, TAG, TAG-72, tenascin, a TRAIL receptor, a Tn antigen, ED-B, WT-1, and a 17-1A antigen. In some other specific embodiments, the antibody or the antigen-binding fragment binds to one or more selected from the following: Her2, Her3, B7H3, Claudin 18.2, DLL-3, and EGP-1 (Trop-2).

In some specific embodiments, the antibody or the antigen-binding fragment is selected from the group consisting of epratuzumab, veltuzumab, sacituzumab, patritumab, trastuzumab, pertuzumab, abciximab, alemtuzumab, cetuximab, gemtuzumab, ibritumomab, panitumumab, rituximab, tositumomab, abagovomab, atlizumab, benralizumab, obinutuzumab, basiliximab, dadizumab, efalizumab, muromomab, natlizumab, omalizumab, gaiitenemmab, solanezumab, tisotumab, brentuximab, inotuzumab, moxetumomab, polatuzumab, enfortumab, belantamab, cetuximab, loncastuximab, daratumumab, nimotuzumab, zolbetuximab, omburtamab, and rovalpituzumab. In some other specific embodiments, the antibody or the antigen-binding fragment is selected from the group consisting of sacituzumab, daratumumab, zolbetuximab, omburtamab, patritumab, and rovalpituzumab.

In some specific embodiments, the antibody or the antigen-binding fragment comprises a heavy chain variable region and a light chain variable region, wherein the heavy chain variable region comprises three complementarity determining regions HCDR1-3, which comprise the amino acid sequences set forth in SEQ ID NOs: 1-3, respectively, and the light chain variable region comprises three complementarity determining regions LCDR1-3, which comprise the amino acid sequences set forth in SEQ ID NOs: 4-6, respectively; preferably, the heavy chain variable region of the antibody or the antigen-binding fragment thereof comprises the amino acid sequence set forth in SEQ ID NO: 7, and the light chain variable region comprises the amino acid sequence set forth in SEQ ID NO: 8. In some other specific embodiments, the heavy chain of the antibody or the antigen-binding fragment thereof comprises the amino acid sequence set forth in SEQ ID NO: 9, and the light chain comprises the amino acid sequence set forth in SEQ ID NO: 10.

In some specific embodiments, the antibody or the antigen-binding fragment comprises a heavy chain variable region and a light chain variable region, wherein the heavy chain variable region comprises three complementarity determining regions HCDR1-3, which comprise the amino acid sequences set forth in SEQ ID NOs: 11-13, respectively, and the light chain variable region comprises three complementarity determining regions LCDR1-3, which comprise the amino acid sequences set forth in SEQ ID NOs: 14-16, respectively. In some other specific embodiments, the heavy chain variable region of the antibody or the antigen-binding fragment thereof comprises the amino acid sequence set forth in SEQ ID NO: 17, and the light chain variable region comprises the amino acid sequence set forth in SEQ ID NO: 18; more preferably, the heavy chain of the antibody or the antigen-binding fragment thereof comprises the amino acid sequence set forth in SEQ ID NO: 19, and the light chain comprises the amino acid sequence set forth in SEQ ID NO: 20.

In a third aspect, the present disclosure provides a pharmaceutical composition, which comprises the antibody-drug conjugate described herein. In some embodiments, the pharmaceutical composition further comprises a pharmaceutically acceptable carrier, excipient, or diluent.

In a fourth aspect, the present disclosure provides use of the antibody-drug conjugate described herein in preparing a medicament for treating and/or preventing a tumor.

In some specific embodiments, the tumor is selected from tumors associated with the expression of one or more of the following molecules: Trop-2, Her2, Her3, B7H3, Claudin 18.2, CD30, CD38, CD33, CD70, and EGFR. In some other specific embodiments, the tumor includes a solid tumor or a hematological tumor. In some specific embodiments, the tumor is selected from the group consisting of: lung cancer, kidney cancer, urethral cancer, colorectal cancer, prostate cancer, glioblastoma multiforme, ovarian cancer, pancreatic cancer, breast cancer, melanoma, liver cancer, bladder cancer, gastric cancer, leukemia, malignant lymphoma, cervical cancer, squamous cell carcinoma, penile cancer, esophageal cancer, and salivary gland cancer.

In a fifth aspect, the present disclosure provides the antibody-drug conjugate described herein for use in treating a tumor disease.

In some specific embodiments, triptolide and a camptothecin drug are used together as toxins in the antibody-drug conjugate.

In some specific embodiments, the tumor is selected from tumors associated with the expression of one or more of the following molecules: Trop-2, Her2, Her3, B7H3, Claudin 18.2, CD30, CD38, CD33, CD70, and EGFR. In some other specific embodiments, the tumor includes a solid tumor or a hematological tumor. In some specific embodiments, the tumor is selected from the group consisting of: lung cancer, kidney cancer, urethral cancer, colorectal cancer, prostate cancer, glioblastoma multiforme, ovarian cancer, pancreatic cancer, breast cancer, melanoma, liver cancer, bladder cancer, gastric cancer, leukemia, malignant lymphoma, cervical cancer, squamous cell carcinoma, penile cancer, esophageal cancer, and salivary gland cancer.

In a sixth aspect, the present disclosure provides a method for treating and/or preventing a tumor, which comprises administering to an individual in need thereof a therapeutically effective amount of the antibody-drug conjugate described herein.

In some specific embodiments, triptolide and a camptothecin drug are used together as toxins in the antibody-drug conjugate.

In some specific embodiments, the tumor is selected from tumors associated with the expression of one or more of the following molecules: Trop-2, Her2, Her3, B7H3, Claudin 18.2, CD30, CD38, CD33, CD70, and EGFR. In some other specific embodiments, the tumor includes a solid tumor or a hematological tumor. In some specific embodiments, the tumor is selected from the group consisting of: lung cancer, kidney cancer, urethral cancer, colorectal cancer, prostate cancer, glioblastoma multiforme, ovarian cancer, pancreatic cancer, breast cancer, melanoma, liver cancer, bladder cancer, gastric cancer, leukemia, malignant lymphoma, cervical cancer, squamous cell carcinoma, penile cancer, esophageal cancer, and salivary gland cancer.

In a seventh aspect, the present disclosure provides use of the antibody-drug conjugate described herein in treating and/or preventing a tumor.

In some specific embodiments, the tumor is selected from tumors associated with the expression of one or more of the following molecules: Trop-2, Her2, Her3, B7H3, Claudin 18.2, CD30, CD38, CD33, CD70, and EGFR. In some other specific embodiments, the tumor includes a solid tumor or a hematological tumor. In some specific embodiments, the tumor is selected from the group consisting of: lung cancer, kidney cancer, urethral cancer, colorectal cancer, prostate cancer, glioblastoma multiforme, ovarian cancer, pancreatic cancer, breast cancer, melanoma, liver cancer, bladder cancer, gastric cancer, leukemia, malignant lymphoma, cervical cancer, squamous cell carcinoma, penile cancer, esophageal cancer, and salivary gland cancer.

### BRIEF DESCRIPTION OF THE DRAWINGS

FIG. 1 shows a schematic diagram of the structure of the ADC comprising double toxins.
FIG. 2A shows the expression of HSP70 in HCT-15 (human colorectal cancer) cells used in Example 12.
FIG. 2B shows the expression of the efflux pump gene ABAC1 in HCT-15 (human colorectal cancer) cells used in Example 12.
FIG. 3A shows changes in tumor volume in mice in the human colorectal cancer mouse model (COLO205 cells) used in Example 13.
FIG. 3B shows changes in tumor weight in mice in the human colorectal cancer mouse model (COLO205 cells) used in Example 13.
FIG. 4 shows changes in tumor volume in mice in the human pancreatic cancer mouse model (Bxpc-3 cells) used in Example 14.
FIG. 5 shows changes in tumor volume in mice in the human colorectal cancer drug-resistant mouse model (HCT-15-TROP2 cells) used in Example 15.
FIG. 6A shows the effect of the small-molecule toxins used in Example 16 on the confluency of MX-1 cells (human breast cancer cells).
FIG. 6B shows the effect of the small-molecule toxins used in Example 16 on the confluency of WiDr cells (human colorectal cancer cells).
FIG. 6C shows the effect of the small-molecule toxins used in Example 16 on the confluency of HeLa cells (human cervical cancer cells).
FIG. 6D shows the effect of the small-molecule toxins used in Example 16 on the confluency of MFE-280 cells (human endometrial cancer cells).
FIG. 7A shows the effect of the small-molecule toxins used in Example 16 on the viability of HuH-7 cells (human liver cancer cells).
FIG. 7B shows the effect of the small-molecule toxins used in Example 16 on the viability of NUGC-4 cells (human gastric cancer cells).
FIG. 7C shows the effect of the small-molecule toxins used in Example 16 on the viability of Calu-6 cells (human anaplastic carcinoma cells).
FIG. 8A shows changes in tumor volume in mice in the human colorectal cancer drug-resistant model (HCT-15-TROP2 cells) used in Example 17.
FIG. 8B shows changes in tumor weight in mice in the human colorectal cancer drug-resistant model (HCT-15-TROP2 cells) used in Example 17.
FIG. 9A shows changes in tumor volume in mice in the human lung cancer model (NCI-H2170 cells) used in Example 18.
FIG. 9B shows changes in tumor weight in mice in the human lung cancer model (NCI-H2170 cells) used in Example 18.
FIG. 10 shows changes in tumor volume in mice in the human NCI-H292 CDX model used in Example 22.
FIG. 11 shows changes in tumor volume in mice in the human gastric cancer (NUGC-4 cells) subcutaneous tumor-bearing model used in Example 27.
FIG. 12A shows changes in tumor volume in mice in the human colon cancer (COLO205 cells) subcutaneous tumor-bearing model used in Example 28.
FIG. 12B shows changes in tumor volume in mice in the human colon cancer (COLO205 cells) subcutaneous tumor-bearing model used in Example 28.
FIG. 13A shows changes in tumor volume in mice in the human lung squamous cell carcinoma (NCI-H2170 cells) subcutaneous tumor-bearing model used in Example 29.
FIG. 13B shows changes in tumor volume in mice in the human colon cancer (NCI-H2170 cells) subcutaneous tumor-bearing model used in Example 29.

### DETAILED DESCRIPTION OF THE INVENTION

In the following description, certain specific details are included to provide a thorough understanding of various disclosed embodiments. However, one skilled in the relevant art will recognize that embodiments may be practiced without one or more of these specific details, or with other methods, components, materials, and so forth.

Unless otherwise required in the context of the present disclosure, throughout the specification and claims, the words "include" and "comprise" are to be construed in an open, inclusive sense, that is, as "including, but not limited to".

Reference throughout this specification to "one embodiment" or "another embodiment" or "an embodiment" or "some embodiments" means that a particular referenced element, structure, or characteristic described in connection with the embodiment is included in at least one embodiment. Thus, the appearances of the phrases "one embodiment" or "an embodiment" or "another embodiment" in various places throughout this specification are not necessarily all referring to the same embodiment. Furthermore, the particular elements, structures, or characteristics may be combined in any suitable manner in one or more embodiments.

It should be understood that the singular articles "a", "an", and "the" as used in the specification and claims of the present disclosure include the plural unless the context clearly dictates otherwise.

### Definition of Terms

Unless otherwise stated to the contrary, the following terms used in the specification and claims have the meanings set forth below:
The abbreviated symbols preceding certain chemical groups named in the present disclosure indicate the total number of carbon atoms present in the indicated chemical groups. For example, C₁-C₃₀ alkyl refers to an alkyl group having a total of 1 to 30 carbon atoms as defined below, and C₃-C₃₀ cycloalkyl refers to a cycloalkyl group having a total of 3 to 30 carbon atoms as defined below. The total number of carbons in the abbreviated symbols does not include carbons that may be present in substituents of the groups.

In the present disclosure, the range of carbon numbers preceding a substituent is intended to include all subranges within that range as well as individual values. For example, "C₁₋₃₀" is deemed to include "C₁₋₂₀", "C₁₋₁₀", "C₂₋₃₀", "C₂₋₂₀", "C₂₋₁₀", "C₃₋₃₀", "C₃₋₂₀", "C₃₋₁₀", and the like. For example, "C₆₋₃₀" is deemed to include "C₆₋₂₄", "C₆₋₁₈", "C₆₋₁₅", "C₆₋₁₂", and the like. In addition, "C₂₋₃₀" and "C₅₋₃₀" are also construed accordingly.

In the present disclosure, the term "halogen" refers to fluorine, chlorine, bromine, or iodine.

In the present disclosure, the term "hydroxy" refers to an -OH group.

In the present disclosure, the term "amino" refers to an -NH₂ group.

In the present disclosure, the term "carboxyl" refers to a -COOH group.

In the present disclosure, the term "cyano" refers to a -CN group.

In the present disclosure, the term "nitro" refers to an -NO₂ group.

In the present disclosure, the term "hydrocarbyl" refers to an aliphatic hydrocarbon group. The hydrocarbyl moiety may be a "saturated hydrocarbyl" group, i.e., an alkyl group, which does not comprise any alkene or alkyne moieties. The hydrocarbyl moiety may also be an "unsaturated hydrocarbyl" moiety, i.e., an alkenyl group or an alkynyl group, which comprises at least one alkene or alkyne moiety. "Alkyl" refers to a saturated hydrocarbon group consisting of the specified number of carbon atoms. "Alkenyl" refers to a linear or branched hydrocarbon chain group consisting of the specified number of carbon atoms and at least one carbon-carbon double bond, and attached to the rest of the molecule by a single bond, such as vinyl, prop-1-enyl, but-1-enyl, pent-1-enyl, and penta-1,4-dienyl. "Alkynyl" refers to a linear or branched hydrocarbon chain group consisting of the specified number of carbon atoms and at least one carbon-carbon triple bond, and attached to the rest of the molecule by a single bond. Hydrocarbyl moieties, whether saturated or unsaturated, may be branched or linear. In the present disclosure, the term "hydrocarbylene", such as "alkylene", "alkenylene", and "alkynylene", refers to the corresponding divalent hydrocarbyl group.

In the present disclosure, the term "hydrocarbyloxy" refers to a group of the formula of -O-hydrocarbyl, wherein the hydrocarbyl is as defined in the present disclosure. Illustrative examples of hydrocarbyloxy include, but are not limited to, methoxy, ethoxy, n-propoxy, 1-methylethoxy (isopropoxy), n-butoxy, isobutoxy, sec-butoxy, tert-butoxy, pentyloxy, and tert-pentyloxy.

In the present disclosure, the term "aryl" refers to a carbocyclic ring (all-carbon) or two or more fused rings (rings sharing two adjacent carbon atoms) having a fully delocalized Pi-electron system. Aryl groups include, but are not limited to, fluorenyl, phenyl, biphenyl, and naphthyl. In the present disclosure, the term "arylene" refers to a divalent form of the aryl group described above.

In the present disclosure, the term "heteroaryl" refers to an aromatic cyclic group consisting of the specified number of carbon atoms (e.g., 3 to 30 carbon atoms) and 1 to 5 heteroatoms selected from nitrogen, oxygen, and sulfur. Heteroaryl may be a monocyclic, bicyclic, tricyclic, or tetracyclic ring system, which may include fused or bridged ring systems. Illustrative examples of heteroaryl include, but are not limited to, azepinyl, acridinyl, benzimidazolyl, benzothiazolyl, benzindolyl, benzodioxolyl, benzofuranyl, benzoxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl, benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolinyl, indolizinyl, isoxazolyl, naphthyridinyl, oxadiazolyl, 2-oxoazepinyl, oxazolyl, oxiranyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, pyrrolyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thienyl. The term "heteroarylene" refers to a divalent form of the heteroaryl group described above.

In the present disclosure, the term "aliphatic cyclic group" refers to a stable non-aromatic monocyclic hydrocarbon group consisting solely of carbon and hydrogen atoms, saturated or unsaturated, and attached to the rest of the molecule by a single bond, such as cyclopropyl, cyclobutyl, cyclopentyl, cyclohexyl, and cyclodecyl. The term "aliphatic heterocyclylene" refers to a divalent form of the aliphatic heterocyclyl group described above.

In the present disclosure, "substituted" refers to being substituted with one or more substituents selected from the following: deuterium, halogen, an amino group, a cyano group, a nitro group, a C₁-C₁₀ alkyl group, a C₁-C₁₀ alkoxy group, a C₁-C₁₀ alkylamino group, a C₂-C₁₀ alkenyl group, a C₂-C₁₀ alkynyl group, a C₃-C₁₀ cycloalkyl group, a C₆-C₂₀ aryl group, and a C₂-C₂₀ heteroaryl group.

In the present disclosure, the terms "individual" and "patient" are used interchangeably and refer to animals (e.g., humans), companion animals (e.g., dogs, cats, or horses), and livestock (e.g., cattle, pigs, and sheep). In certain embodiments, the individual is a mammal including males and females. In certain embodiments, the individual is a human.

In the present disclosure, the term "mammal" refers to animals including, such as dogs, cats, cattle, sheep, horses, humans, and the like. In certain embodiments, the mammal includes humans.

In the present disclosure, the term "pharmaceutically acceptable" refers to carriers, vehicles, diluents, excipients, and/or salts that must be compatible with other ingredients of the formulation and not deleterious to a recipient thereof.

In the present disclosure, the term "optional" or "optionally" means that the subsequently described event or circumstance may or may not occur, and the specification includes instances where the event or circumstance occurs and instances where it does not.

In the present disclosure, the term "pharmaceutically acceptable excipient" includes, but is not limited to, any auxiliary substance of various forms approved by the U.S. Food and Drug Administration for use in humans or animals without adverse effects on the composition of pharmaceutical compositions, such as carriers, glidants, sweeteners, diluents, preservatives, dyes/colorants, flavor enhancers, surfactants, wetting agents, dispersants, suspending agents, stabilizers, isotonic agents, solvents, or emulsifiers.

In the present disclosure, the term "carrier" is defined as a compound that facilitates the introduction of a compound into cells or tissues. For example, dimethyl sulfoxide (DMSO) is commonly used as a carrier because it readily facilitates the introduction of certain organic compounds into cells or tissues of organisms.

In the present disclosure, the term "diluent" is defined as a substance used to dilute the primary pharmaceutical ingredient in pharmaceutical formulations, the main function of which is to uniformly mix a trace amount of the primary pharmaceutical ingredient with other ingredients so as to improve the content uniformity of the drug in the formulation, or improve the formability of the formulation to meet the requirements of the preparation process.

In the present disclosure, the term "pharmaceutically acceptable salt" includes "acceptable acid addition salts" and "acceptable base addition salts".

In the present disclosure, the term "acceptable acid addition salts" refers to salts that retain the biological effectiveness and properties of the free bases, and the acid addition salts are biologically or otherwise suitable and formed from inorganic acids or organic acids. The inorganic acids include, but are not limited to, hydrochloric acid, hydrobromic acid, sulfuric acid, nitric acid, phosphoric acid, and the like. The organic acids include, but are not limited to, acetic acid, 2,2-dichloroacetic acid, adipic acid, alginic acid, ascorbic acid, aspartic acid, benzenesulfonic acid, benzene carboxylic acid, 4-acetamidobenzene carboxylic acid, camphoric acid, camphor-10-sulfonic acid, decanoic acid, caproic acid, caprylic acid, carbonic acid, cinnamic acid, citric acid, cyclohexylsulfamic acid, dodecyl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

In the present disclosure, the term "acceptable base addition salts" refers to salts that retain the biological effectiveness and properties of the free acids, and the acceptable base addition salts are biologically or otherwise suitable. Such salts are prepared by adding inorganic bases or organic bases to the free acids. Salts derived from inorganic bases include, but are not limited to, sodium, potassium, lithium, ammonium, calcium, magnesium, iron, zinc, copper, manganese, aluminium salts, and the like. In certain embodiments, the inorganic salts are ammonium, sodium, potassium, calcium, and magnesium salts. Salts derived from organic bases include, but are not limited to, salts of primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, diethanolamine, ethanolamine, 2-dimethylaminoethanol, 2-diethylaminoethanol, dicyclohexylamine, lysine, arginine, histidine, caffeine, procaine, hydrabamine, choline, betaine, benzylamine, phenethylenediamine, ethylenediamine, glucosamine, methylglucamine, theobromine, triethanolamine, tromethamine, purine, piperazine, piperidine, N-ethylpiperidine, polyamine resins, and the like. In certain embodiments, the organic bases are isopropylamine, diethylamine, ethanolamine, trimethylamine, dicyclohexylamine, choline, and caffeine.

In the present disclosure, the term "solvent or solvent mixture" refers to any and all solvents. In certain embodiments, the solvent or solvent mixture comprises organic solvents and water, including, but not limited to, methanol, ethanol, 2-propanol, n-butanol, isobutanol, acetone, methyl ethyl ketone, ethyl acetate, 1,4-dioxane, diethyl ether, methyl tert-butyl ether, tetrahydrofuran, acetonitrile, dichloromethane, chloroform, N,N-dimethylformamide, cyclohexane, cyclopentane, n-hexane, n-heptane, n-pentane, toluene, o-xylene, p-xylene, dimethyl sulfoxide (DMSO), pyridine, acetic acid, anisole, butyl acetate, cumene, ethyl formate, formic acid, isobutyl acetate, isopropyl acetate, methyl acetate, 3-methyl-1-butanol, methyl isobutyl ketone, 2-methyl-1-propanol, 1-pentanol, propyl acetate, ethylene glycol, and 1-methyl-2-pyrrolidinone, as well as any and all mixtures of two or more such solvents. In certain embodiments, the solvent or solvent mixture is a single solvent or a binary mixture. In certain embodiments, the solvent or solvent mixture is a single solvent of water or an organic solvent, or a binary mixture of water and an organic solvent.

In the present disclosure, the term "pharmaceutical composition" refers to a formulation formed by the compound described in the present disclosure and a medium generally accepted in the art for delivering biologically active compounds to mammals such as humans. Such a medium includes all pharmaceutically acceptable carriers, diluents, or excipients.

In the present disclosure, the terms "therapeutically effective amount" and "effective amount" are used interchangeably, and refer to an amount of a compound or a combination of compounds that ameliorates, alleviates, or eliminates a specific disease or condition and symptoms of the specific disease or condition, or prevents or delays the onset of a specific disease or condition or symptoms of the specific disease or condition. The amount of the compound described in the present disclosure that constitutes a "therapeutically effective amount" will vary depending on the compound, the disease state and its severity, and the age, body weight, and the like of the mammal to be treated, but can be determined routinely by those skilled in the art in accordance with their knowledge and the present disclosure.

As used in the present disclosure, "treat", "treating", or "treatment" refers to treating a related disease or disease state in a mammal, such as a human, suffering from the relevant disease or condition, and includes:
(i) preventing the disease or disease state from occurring in a mammal, particularly when the mammal is susceptible to the disease state but has not been diagnosed with it;
(ii) inhibiting the disease or disease state, i.e., preventing its occurrence; or
(iii) alleviating the disease or disease state, such that the disease or disease state regresses or ceases to progress.

As used in the present disclosure, the terms "disease", "condition", and "disease state" may be used interchangeably, or may be distinct. This is because a particular disease or disease state may have no known causative factor (and thus cannot be explained etiologically), and is therefore not recognized as a disease but as an undesirable disease state or condition for which clinicians have identified a more or less specific set of symptoms.

In the present disclosure, the term "physiologically acceptable" refers to carriers or diluents that do not abolish the biological activity and properties of the compound.

### Exemplary Embodiments

The present disclosure unexpectedly finds that triptolide in combination with camptothecin drugs exerts a synergistic effect and can significantly enhance the killing effect on tumor cells, which is expected to provide a novel therapeutic strategy for tumor diseases.

Therefore, the present disclosure provides use of triptolide in combination with a camptothecin drug in preparing a medicament for treating a tumor disease.

In some specific embodiments, the present disclosure finds that triptolide and a camptothecin drug can be used together as toxins in an antibody-drug conjugate for preparing a medicament for treating a tumor disease.

In some specific embodiments, the camptothecin drug is illustratively camptothecin (CPT), exatecan, topotecan, SN38, or a derivative thereof. In some preferred embodiments, the camptothecin drug is exatecan.

In some specific embodiments, the molar ratio of triptolide to the camptothecin drug is 0.1:1 to 10:1. In some other preferred embodiments, the molar ratio of triptolide to the camptothecin drug is 1:0.5 to 1:2. In some other preferred embodiments, the molar ratio of triptolide to the camptothecin drug is about 1:1.

In some more specific embodiments, the present disclosure provides an antibody-drug conjugate comprising double toxins, which has a formula of: 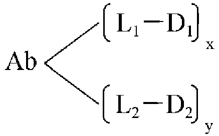
wherein Ab is an antibody or an antigen-binding fragment thereof;
D₁ and D₂ are toxins having anti-tumor activity, D₁ and D₂ are selected from the group consisting of triptolide and a camptothecin drug, and D₁ and D₂ are different;
L₁ and L₂ are linking units that link the toxin and the antibody;
x and y are 0-8, and x and y are integers or non-integers.

In some more specific embodiments, x and y may be 0, 1, 2, 3, 4, 5, 6, 7, or 8, and other values between these values. In some more specific embodiments, the sum of x and y is greater than or equal to 4.

In some more specific embodiments, the present disclosure provides an antibody-drug conjugate comprising double toxins, which has a formula of:

Ab-[(L₁-D₁-L₂-D₂)ₓ

wherein Ab is an antibody or an antigen-binding fragment thereof;
D₁ and D₂ are toxins having anti-tumor activity, D₁ and D₂ are selected from the group consisting of triptolide and a camptothecin drug, and D₁ and D₂ are different;
L₁ and L₂ are linking units that link the toxin and the antibody;
x is 2-8, and x and y are integers or non-integers.

In some more specific embodiments, x and y may be 2, 3, 4, 5, 6, 7, or 8, and other values between these values.

In some embodiments, L₁ is linked to Ab via a sulfhydryl or amide group. In some preferred embodiments, L₁ is linked to a Fab fragment of Ab. One of the most common methods for conjugating antibodies to toxins involves utilizing lysine residues of antibodies, wherein the nucleophilic NH₂ group of amino acids reacts with the electrophilic N-hydroxysuccinimide (NHS) group on the payload. In addition, a disulfide rebridging strategy may be adopted. The four interchain disulfide bonds contained in IgG antibodies are reduced to generate eight sulfhydryl groups, which react with maleimide-containing linkers. Furthermore, the conjugation of toxins to antibodies may also be performed via divinylpyrimidine rebridging technology, dibromopyridinedione bridging technology, bis-sulfone rebridging conjugation technology, engineered unnatural amino acid bioconjugation, and the like.

In some embodiments, L₂ is linked to Ab via glycosyl. In some preferred embodiments, L₂ is linked to a Fc fragment of Ab. Since IgG is a glycoprotein, it contains an N-glycan at the asparagine residue position of the CH₂ domain of each heavy chain in the Fc fragment, and such glycosylation can serve as a point of attachment for conjugation of payloads. In some embodiments, L₂ is linked to the antibody via an oligosaccharide, which is located in the Fc fragment of the antibody and is specifically linked to an asparagine residue of the Fc fragment. In some embodiments, glycoforms that can be conjugated include GO-GN, G0F-GN, G0, G0F, G1, G1F, and the like. In some preferred embodiments, the glycoform of the oligosaccharide is G0, G0F, and/or G1F. In some preferred embodiments, the oligosaccharide on the antibody is modified with azide. In some preferred embodiments, the azide modification comprises: contacting the antibody with UDP-GalNAz and GalT1. In some other embodiments, the azide-modified antibody is linked to DBCO on L₂ via a coupling reaction. L₂ is linked to asparagine (Asn residue) on the antibody via an oligosaccharide, and more preferably, L₂ is linked to the asparagine on the antibody via GlcNac on the oligosaccharide. In some specific embodiments, the oligosaccharide is linked to a Fc fragment of the antibody. In some specific embodiments, the oligosaccharide is linked to a CH₂ domain of the Fc fragment. In some more specific embodiments, the oligosaccharide is linked to Asn of the Fc fragment. In some other preferred embodiments, the coupling reaction comprises: subjecting the azide-modified antibody and DBCO on L₂ to a click chemistry reaction to accomplish glycosyl conjugation between L₂ and the antibody. For the glycosyl conjugation approach, reference may be made to the literature: Chemoenzymatic Conjugation of Toxic Payloads to the Globally Conserved N-Glycan of Native mAbs Provides Homogeneous and Highly Efficacious Antibody-Drug Conjugate, RV Geel et al., Bioconjug Chem. 2015 Nov 18; 26(11):2233-42.

In some embodiments:
L₁ has a structure represented by [H₁-L₁ₐ-L_{1b}-L_{1c}-D₁]ₓ;
L₂ has a structure represented by [H₂-L₂ₐ-L_{2b}-L_{2c}-D₂]_{y};
H₁ and H₂ are linker moieties for the antibody or the antigen-binding fragment thereof or groups capable of reacting with Ab;
L₁ₐ is a linking unit that links the linker moiety H₁ and L_{1b}, and L₂ₐ is a linking unit that links the linker moiety H₂ and L_{2b};
L_{1b} is a linker that links L₁, and L_{1c}, and L_{2b} is a linker that links L₂ₐ and L_{2c};
L_{1c} is a spacer between L_{1b} and D₁, and L_{2c} is a spacer between L_{2b} and D₂.

In some embodiments, H₁ and H₂ are selected from the group consisting of:

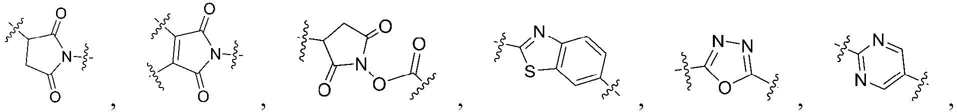

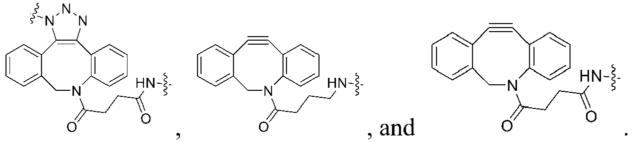

In some embodiments, H₁ is selected from the group consisting of: 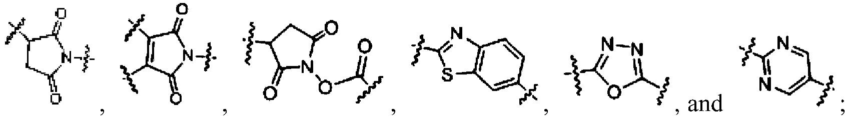 preferably, H₁ is

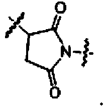

In some embodiments, H₂ is selected from the group consisting of:

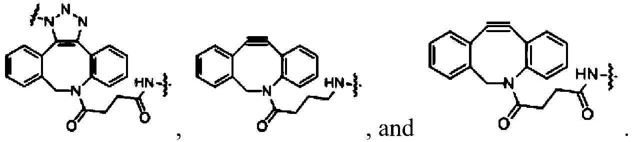

In some embodiments, L₁ₐ or L₂ₐ comprises -L_{d}-C(O)-, wherein L_{d} is selected from the group consisting of optionally substituted alkylene, an optionally substituted polyethylene glycol group, optionally substituted alkenylene, optionally substituted alkynylene, optionally substituted aliphatic cyclylene, optionally substituted aliphatic heterocyclylene, optionally substituted arylene, optionally substituted heteroarylene, and a combination thereof.

In some embodiments, L_{d} is selected from the group consisting of -(CH₂)m-, -(PEG)n-, and - (CH₂)ₘ-(PEG)ₙ-(CH₂)_{z}-, wherein m, n, and z are integers of 0-10, and preferably, m, n, and z are integers of 0-8. For example, m, n, and z are 0, 1, 2, 3, 4, 5, 6, 7, 8, 9, 10, or a range between any two of these values.

In some embodiments, L_{1b} or L_{2b} is a cleavable linker or a non-cleavable linker. ADCs based on non-cleavable linkers must be internalized, and the antibody moiety needs to be degraded by lysosomal proteases to release active molecules. A representative non-cleavable linker is N-succinimidyl-4-(N-maleimidomethyl)cyclohexane-1-carboxylate (SMCC). Cleavable linkers can be classified into enzyme-dependent linkers and non-enzymatic (chemical-)dependent linkers. A typical chemical-dependent linker is a linker containing a disulfide bond, which is subjected to nucleophilic attack by thiols to release active carriers. Enzymatic linkers are the most promising type of linkers in current applications, as they can be cleaved by enzymes such as cathepsins, phosphatases, pyrophosphatases, β-glucuronidases, β-galactosidases, and sulfatases.

In some embodiments, L_{1b} or L_{2b} is a cleavable peptide chain consisting of 2-10 amino acids; preferably, L₂ or L₅ is selected from the group consisting of Gly-Gly-Phe-Gly (abbreviated as GGFG), Val-Cit (abbreviated as VC), Val-Ala (abbreviated as VA), Val-Lys, Val-Arg, AcLys-Val-Cit, AcLys-Val-Ala, Phe-Cit, Phe-Ala, Phe-Lys, Phe-Arg, Glu-Val-Ala, Glu-Val-Cit, Ala-Lys, Leu-Cit, Ile-Cit, Trp-Cit, Ala-Ala-Asn, Ala-Ala-Ala, Ala-Asn, and Lys; preferably, L_{1b} or L_{2b} is selected from the group consisting of Gly-Gly-Phe-Gly, Val-Cit, Val-Ala, Val-Lys, Phe-Cit, Phe-Ala, Phe-Lys, and Ala-Ala-Asn.

In some embodiments, L_{1c} or L_{2c} is selected from the group consisting of:

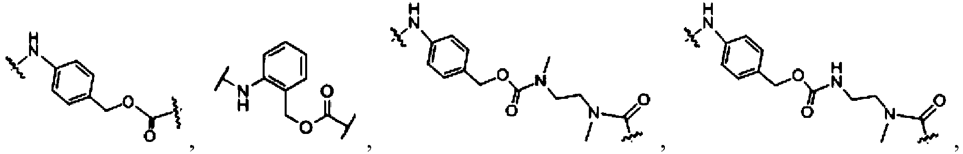

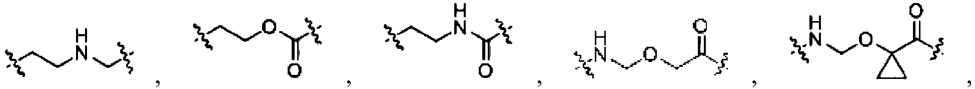

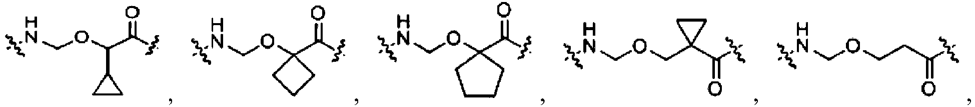

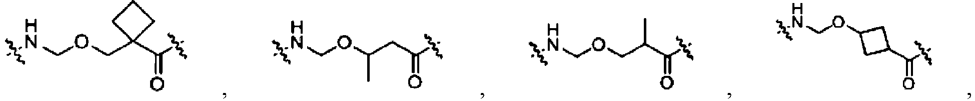

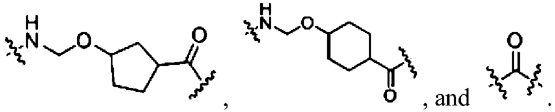

In some preferred embodiments, L_{1c} is selected from the group consisting of

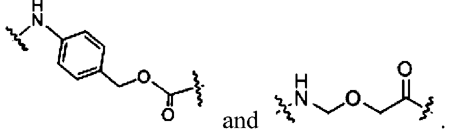

In some specific embodiments, L₁-D₁ is: 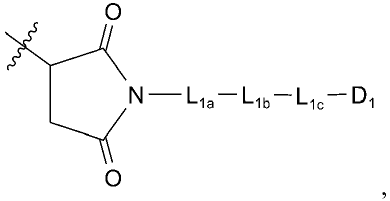 wherein:
L₁ₐ is -(CH₂)ₘ₁-C(O)- or -(PEG)ₙ₁-(CH₂)_{z1}-C(O)-, wherein m₁, n₁, and z₁ are integers of 2-8;
L_{1b} is Gly-Gly-Phe-Gly, Val-Cit, or Val-Ala;
L_{1c} is 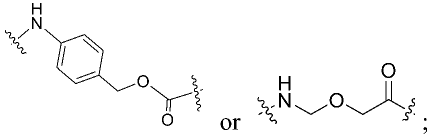
D₁ is triptolide or a camptothecin drug.

In a more specific embodiment, m₁, n₁, and z₁ are 2, 3, 4, 5, 6, 7, 8, or a range between any two of these values.

In some preferred embodiments, L₁ₐ is -(CH₂)ₘ₁-C(O)-, wherein m₁ is an integer of 2-6, preferably 5; or L₁ₐ is -(PEG)ₙ₁-(CH₂)_{z1}-C(O)-, wherein n₁ and z₁ are integers of 2-6, and preferably, n₁ and z₁ are 2.

In some specific embodiments, L₂-D₂ is: 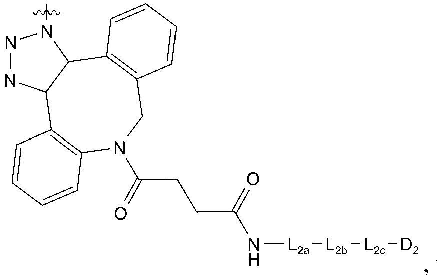 wherein:
L₂ₐ is -(PEG)ₙ₂-(CH₂)_{z2}-C(O)-, wherein n₂ and z₂ are integers of 2-8;
L_{2b} is Gly-Gly-Phe-Gly, Val-Cit, or Val-Ala;
L_{2c} is 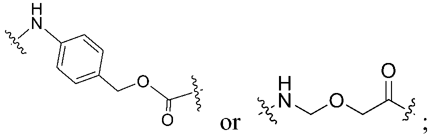
D₂ is triptolide or a camptothecin drug.

In a more specific embodiment, n₂ and z₂ are 2, 3, 4, 5, 6, 7, 8, or a range between any two of these values.

In some preferred embodiments, L₂ₐ is -(PEG)ₙ₂-(CH₂)_{z2}-C(O)-, wherein n₂ and z₂ are integers of 2-6; or L₁ₐ is -(PEG)ₙ₁-(CH₂)_{z1}-C(O)-, wherein n₁ and z₁ are integers of 2-6; preferably, n₂ is an integer of 4-6, and z₂ is 2.

In some embodiments, D₁ is triptolide, and D₂ is a camptothecin drug.

In some preferred embodiments, D₁ is a camptothecin drug, and D₂ is triptolide.

In some embodiments, the camptothecin drug is camptothecin (CPT), exatecan, topotecan, SN38, or a derivative thereof; preferably, the camptothecin drug is exatecan.

Illustratively, the camptothecin drug or the derivative has the following structures:

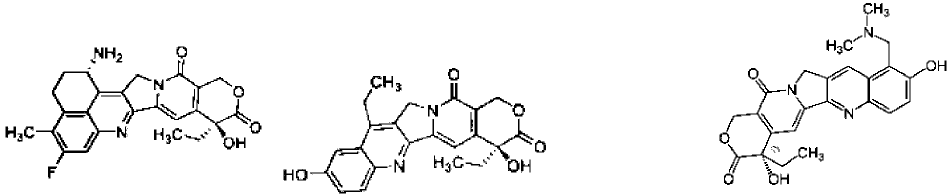

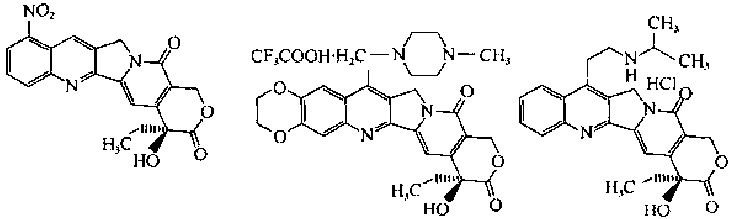

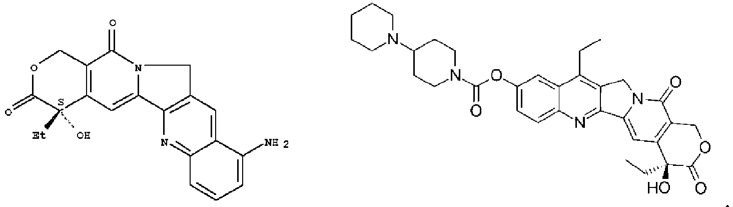

In some preferred embodiments, the camptothecin drug is exatecan.

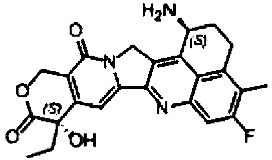

In some embodiments, x is selected from 2-8, preferably 3-5; y is selected from 3-4.

In some embodiments, D₁ is

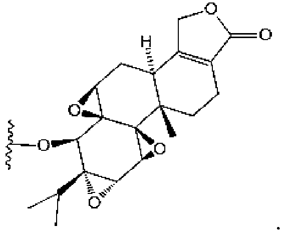

In some embodiments, D₂ is

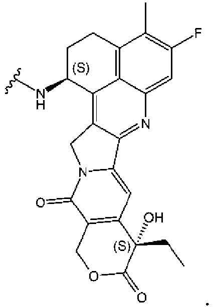

In some embodiments, D₁ is

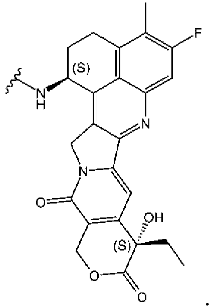

In some embodiments, D₂ is

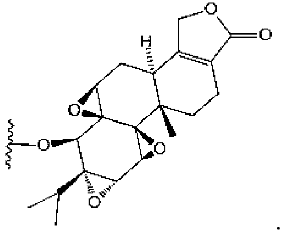

In some embodiments, the L₁-D₁ structure is selected from the group consisting of:

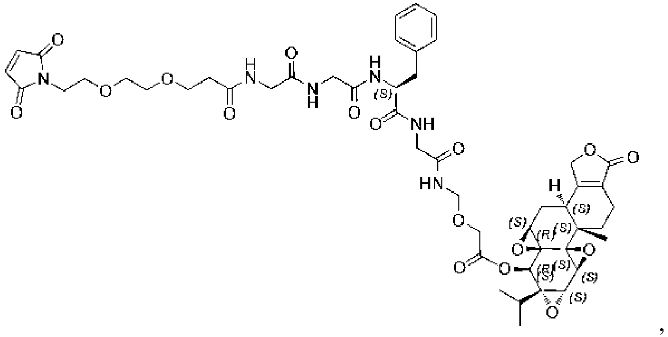

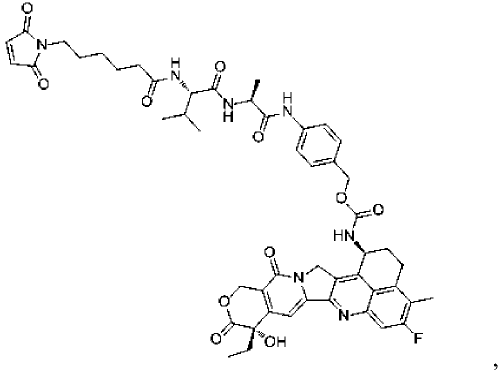

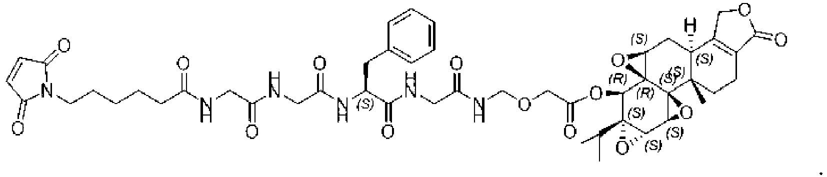

In some embodiments, the L₂-D₂ structure is selected from the group consisting of:

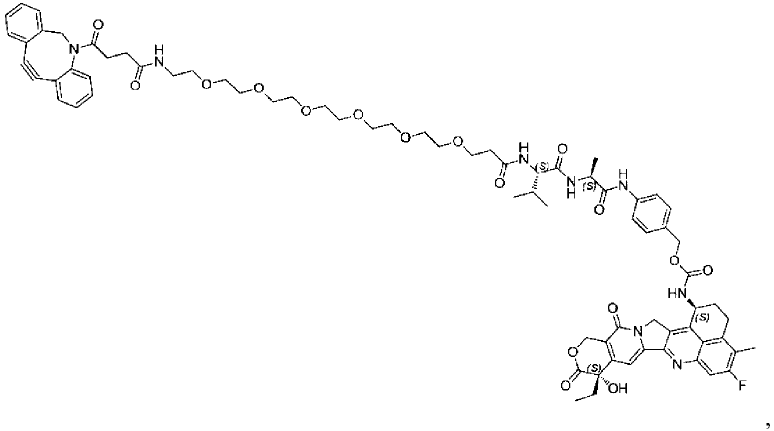

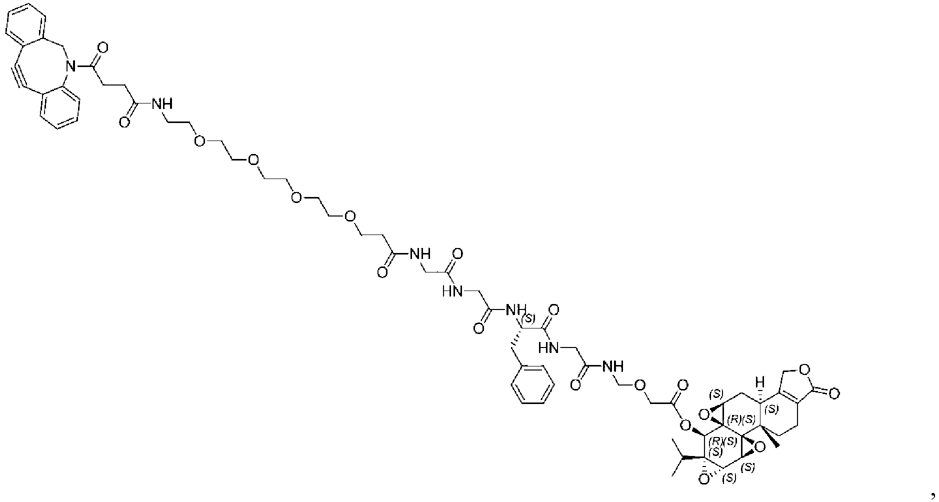

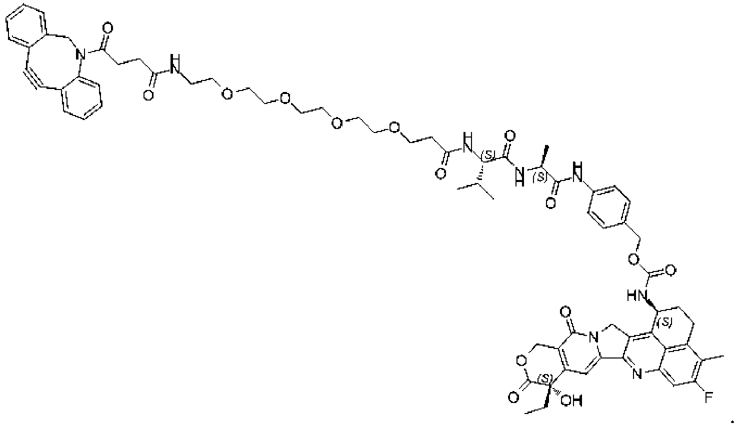

In some specific embodiments, the L₁-D₁ structure is: 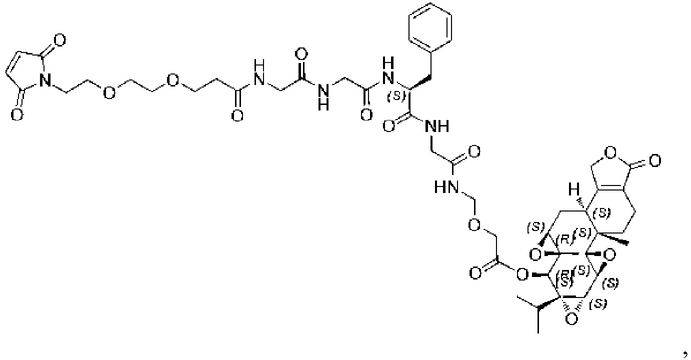 and
the L₂-D₂ structure is:

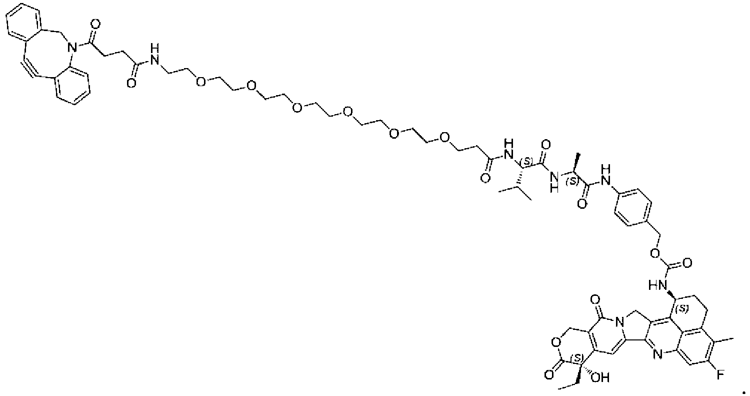

In some other specific embodiments, the L₁-D₁ structure is: 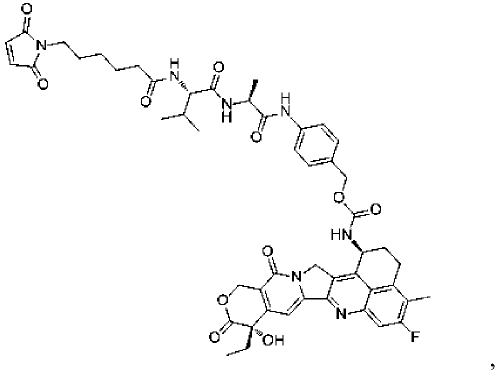 and
the L₂-D₂ structure is:

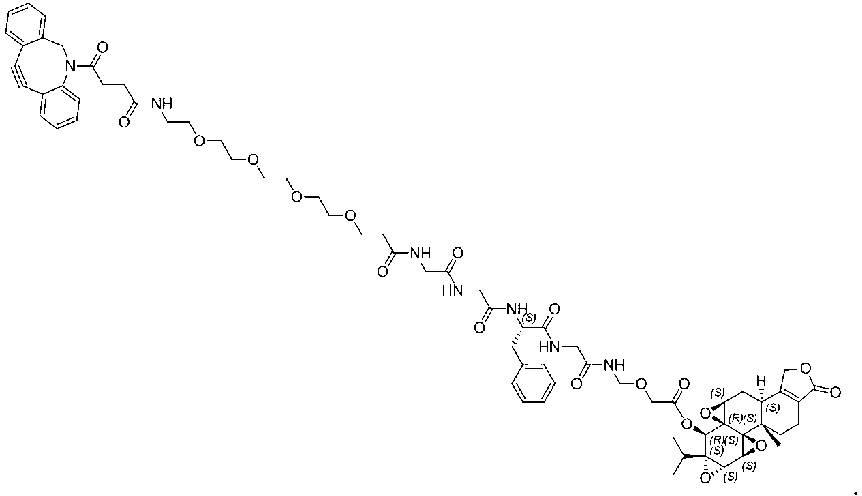

In some other specific embodiments, the L₁-D₁ structure is: 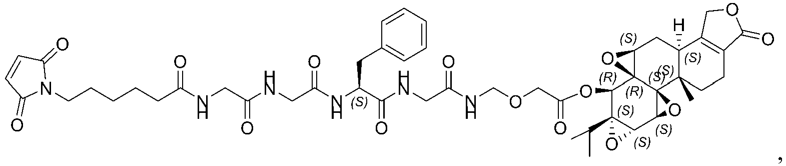 and
the L₂-D₂ structure is:

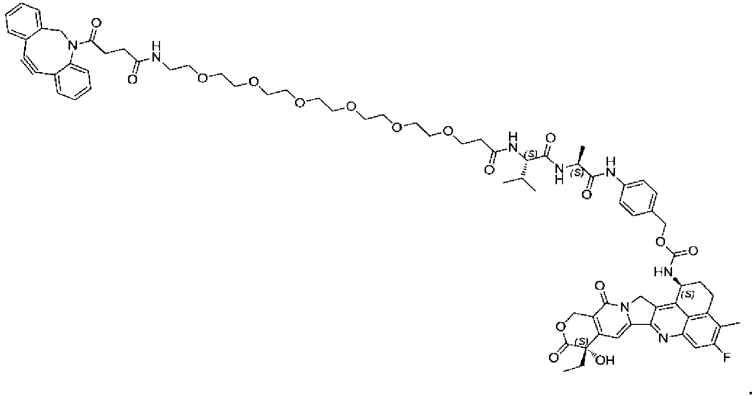

In some other specific embodiments, the L₁-D₁ structure is: 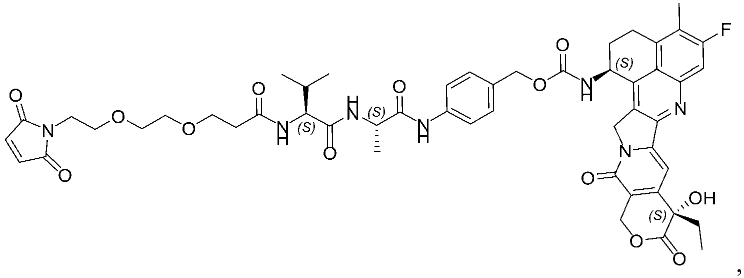 and
the L₂-D₂ structure is:

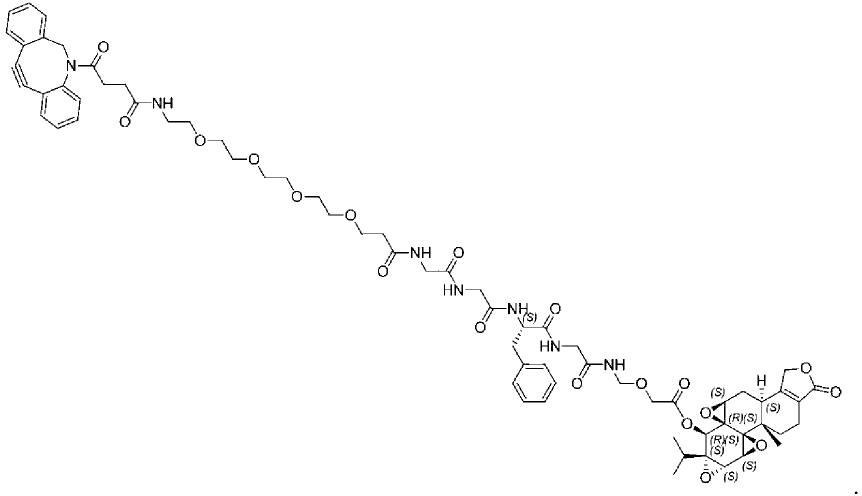

In some embodiments, L₂ is linked to the antibody via an oligosaccharide, which is located in a Fc fragment of the antibody and is specifically linked to an asparagine residue of the Fc fragment. In some embodiments, glycoforms that can be conjugated include GO-GN, G0F-GN, G0, G0F, G1, G1F, and the like. In some preferred embodiments, the glycoform of the oligosaccharide is G0, G0F, and/or G1F. In some embodiments, the oligosaccharide on the antibody is modified with azide. In some preferred embodiments, the azide modification comprises: contacting the antibody with UDP-GalNAz and GalT1. In some other embodiments, the azide-modified antibody is linked to DBCO on L2 via a coupling reaction. In some other preferred embodiments, the coupling reaction comprises: subjecting the azide-modified antibody and DBCO on L2 to a click chemistry reaction to accomplish glycosyl conjugation between L2 and the antibody.

In some embodiments, the antibody or the antigen-binding fragment binds to one or more selected from the following: carbonic anhydrase IX, α-fetoprotein, α-actinin, A3, A33, ART 4, B7, B7H3, B7H4, BAGE, a BrE3 antigen, CA125, CAMEL, CAP", CASP-8/m, CCL19, CCL21, CD1, CDla.CD2, CD3, CD4, CD5, CD8, CD11A, CD14, CD15, CD16, CD18, CD19, CD20, CD21, CD22, CD23, CD24, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4, CDKN2A, HIF-Iα, colon-specific antigen p (CSAp), CEA, CEACAM5, CEACAM6, oMet, DAM, EGFR, EGFRvIII, cMet, EGP-1 (Trop-2), EGP-2, ELF2-M, Ep-CAM, Her2, Her3, Claudin 18.2, ROR1, ROR2, dll3, marcl7, a fibroblast growth factor (FGF), Flt-1, Flt-3, a folate receptor, a G250 antigen, GAGE, gp100, GRO-β, HLA-DR.HML24, HMGB-1, HSP70-2M, IGF-1R, IGR1R, MAGE, MAGE-3, MART-1, MART-2, NY-ESO-1, TRAG3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, a mucin in pancreatic cancer, a PD-1 receptor, a PD-L1 receptor, a placental growth factor, PLAGL2, prostatic acid phosphatase, PSA, PRAME, PSMA, P1GF, ILGF, ILGF-1R, RS5, RANTES, T101, SAGE, S100, survivin, survivin-2B, TAG, TAG-72, tenascin, a TRAIL receptor, a Tn antigen, ED-B, WT-1, and a 17-1A antigen. In a preferred embodiment, the antibody or the antigen-binding fragment binds to one or more selected from the group consisting of Her2, Her3, B7H3, Claudin 18.2, DLL-3, and EGP-1 (Trop-2).

In some specific embodiments, the antibody or the antigen-binding fragment is selected from the group consisting of epratuzumab, veltuzumab, sacituzumab, patritumab, trastuzumab, pertuzumab, abciximab, alemtuzumab, cetuximab, gemtuzumab, ibritumomab, panitumumab, rituximab, tositumomab, abagovomab, atlizumab, benralizumab, obinutuzumab, basiliximab, dadizumab, efalizumab, muromomab, natlizumab, omalizumab, gaiitenemmab, solanezumab, tisotumab, brentuximab, inotuzumab, moxetumomab, polatuzumab, enfortumab, belantamab, cetuximab, loncastuximab, daratumumab, nimotuzumab, zolbetuximab, omburtamab, and rovalpituzumab.

In some preferred embodiments, the antibody or the antigen-binding fragment is selected from the group consisting of sacituzumab, daratumumab, zolbetuximab, omburtamab, patritumab, and rovalpituzumab.

In some embodiments, the antibody or the antigen-binding fragment is a sacituzumab antibody or an antigen-binding fragment thereof, wherein the sacituzumab antibody or the antigen-binding fragment comprises a heavy chain variable region and a light chain variable region, wherein the heavy chain variable region comprises three complementarity determining regions HCDR1-3, which comprise the amino acid sequences set forth in SEQ ID NOs: 1-3, respectively, and the light chain variable region comprises three complementarity determining regions LCDR1-3, which comprise the amino acid sequences set forth in SEQ ID NOs: 4-6, respectively.

In some specific embodiments, the amino acid sequences of the HCDR1-3 of the antibody or the antigen-binding fragment are set forth in SEQ ID NOs: 1-3, respectively. In some other specific embodiments, the amino acid sequences of the LCDR1-3 of the antibody or the antigen-binding fragment are set forth in SEQ ID NOs: 4-6, respectively.

In some more specific embodiments, the heavy chain variable region of the sacituzumab antibody or the antigen-binding fragment thereof comprises the amino acid sequence set forth in SEQ ID NO: 7, and the light chain variable region comprises the amino acid sequence set forth in SEQ ID NO: 8. For example, in some embodiments, the amino acid sequence of the heavy chain variable region of the antibody or the antigen-binding fragment is set forth in SEQ ID NO: 7. In some other embodiments, the amino acid sequence of the light chain variable region of the antibody or the antigen-binding fragment is set forth in SEQ ID NO: 8.

In some more specific embodiments, a heavy chain of the sacituzumab antibody or the antigen-binding fragment thereof comprises the amino acid sequence set forth in SEQ ID NO: 9, and a light chain comprises the amino acid sequence set forth in SEQ ID NO: 10. For example, in some embodiments, the amino acid sequence of the heavy chain of the antibody or the antigen-binding fragment is set forth in SEQ ID NO: 9. In some other embodiments, the amino acid sequence of the light chain of the antibody or the antigen-binding fragment is set forth in SEQ ID NO: 10.

In some embodiments, the antibody or the antigen-binding fragment is a patritumab antibody or an antigen-binding fragment thereof, wherein the patritumab antibody or the antigen-binding fragment comprises a heavy chain variable region and a light chain variable region, wherein the heavy chain variable region comprises three complementarity determining regions HCDR1-3, which comprise the amino acid sequences set forth in SEQ ID NOs: 11-13, respectively, and the light chain variable region comprises three complementarity determining regions LCDR1-3, which comprise the amino acid sequences set forth in SEQ ID NOs: 14-16, respectively.

In some specific embodiments, the amino acid sequences of the HCDR1-3 of the antibody or the antigen-binding fragment are set forth in SEQ ID NOs: 11-13, respectively. In some other specific embodiments, the amino acid sequences of the LCDR1-3 of the antibody or the antigen-binding fragment are set forth in SEQ ID NOs: 14-16, respectively.

In some more specific embodiments, the heavy chain variable region of the patritumab antibody or the antigen-binding fragment thereof comprises the amino acid sequence set forth in SEQ ID NO: 17, and the light chain variable region comprises the amino acid sequence set forth in SEQ ID NO: 18. For example, in some embodiments, the amino acid sequence of the heavy chain variable region of the antibody or the antigen-binding fragment is set forth in SEQ ID NO: 17. In some other embodiments, the amino acid sequence of the light chain variable region of the antibody or the antigen-binding fragment is set forth in SEQ ID NO: 18.

In some more specific embodiments, a heavy chain of the patritumab antibody or the antigen-binding fragment thereof comprises the amino acid sequence set forth in SEQ ID NO: 19, and a light chain comprises the amino acid sequence set forth in SEQ ID NO: 20. For example, in some embodiments, the amino acid sequence of the heavy chain of the antibody or the antigen-binding fragment is set forth in SEQ ID NO: 19. In some other embodiments, the amino acid sequence of the light chain of the antibody or the antigen-binding fragment is set forth in SEQ ID NO: 20.

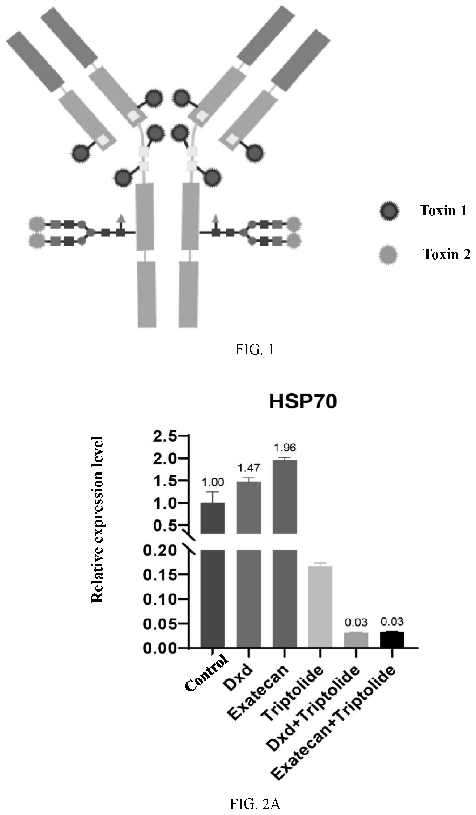

In some embodiments, the method for preparing the double-toxin antibody-drug conjugate described in the present disclosure comprises: first, subjecting an antibody to an azidation reaction, followed by glycosyl conjugation of toxin D₂ (or the aforementioned [H₂-L₂ₐ₋L_{2b}-L_{2c}-D₂]_{y} structural unit) to the antibody; then, conjugating toxin D₁ (or the aforementioned [H₁-L₁ₐ-L_{1b}-L_{1c}-D₁]ₓ structural unit) to the antibody via sulfhydryl, thereby preparing an antibody-drug conjugate comprising double toxins, the structural schematic diagram of which is shown in FIG. 1. In FIG. 1, toxin 1 is conjugated to the antibody via a sulfhydryl linking unit, and the four interchain disulfide bonds contained in the antibody are reduced to generate eight sulfhydryl groups, thereby allowing 2-8 toxin 1 molecules to be loaded via the linking unit. Toxin 2 is conjugated to the antibody via a glycosyl linking unit, allowing 2-4 toxin 2 molecules to be loaded via the linking unit.

In another aspect, the present disclosure is to provide a pharmaceutical composition, which comprises the antibody-drug conjugate described in the present disclosure, and a pharmaceutically acceptable carrier, excipient, or diluent.

In another aspect, the present disclosure is to provide use of the antibody-drug conjugate described in the present disclosure in preparing a medicament for treating and/or preventing a tumor.

In some preferred embodiments, the tumor is selected from tumors associated with the expression of one or more of the following molecules: Trop-2, Her2, Her3, B7H3, Claudin 18.2, CD30, CD38, CD33, CD70, and EGFR.

In some specific embodiments, the tumor includes a solid tumor or a hematological tumor.

In some more specific embodiments, the tumor is selected from the group consisting of: lung cancer, kidney cancer, urethral cancer, colorectal cancer, prostate cancer, glioblastoma multiforme, ovarian cancer, pancreatic cancer, breast cancer, melanoma, liver cancer, bladder cancer, gastric cancer, leukemia, malignant lymphoma, cervical cancer, squamous cell carcinoma, penile cancer, esophageal cancer, and salivary gland cancer.

### Examples

The present disclosure is further illustrated with reference to the following specific examples. It should be understood that these examples are merely intended to illustrate the present disclosure rather than limit the scope of the present disclosure, and the following examples do not represent that the following experiments are all or the only experiments of the present disclosure.

An exemplary antibody used in the present disclosure is sacituzumab (also known as certolizumab pegol/sacituzumab govitecan/hRS7), which is described in detail in WO2014057687A. Specifically, the heavy chain variable region of the sacituzumab antibody comprises three complementarity determining regions HCDR1-3, which comprise the amino acid sequences set forth in SEQ ID NOs: 1-3, respectively; the light chain variable region comprises three complementarity determining regions LCDR1-3, which comprise the amino acid sequences set forth in SEQ ID NOs: 4-6, respectively. More specifically, the heavy chain variable region of the sacituzumab antibody comprises the amino acid sequence set forth in SEQ ID NO: 7, and the light chain variable region comprises the amino acid sequence set forth in SEQ ID NO: 8; more specifically, the heavy chain of the sacituzumab antibody comprises the amino acid sequence set forth in SEQ ID NO: 9, and the light chain comprises the amino acid sequence set forth in SEQ ID NO: 10. Antibodies can be constructed using conventional biological methods in the art or are commercially available.

Another exemplary antibody used in the present disclosure is patritumab, which is described in detail in WO2007077028A2. Specifically, the heavy chain variable region of the patritumab antibody comprises three complementarity determining regions HCDR1-3, which comprise the amino acid sequences set forth in SEQ ID NOs: 11-13, respectively; the light chain variable region comprises three complementarity determining regions LCDR1-3, which comprise the amino acid sequences set forth in SEQ ID NOs: 14-16, respectively. More specifically, the heavy chain variable region of the patritumab antibody comprises the amino acid sequence set forth in SEQ ID NO: 17, and the light chain variable region comprises the amino acid sequence set forth in SEQ ID NO: 18; more specifically, the heavy chain of the patritumab antibody comprises the amino acid sequence set forth in SEQ ID NO: 19, and the light chain comprises the amino acid sequence set forth in SEQ ID NO: 20. Antibodies can be constructed using conventional biological methods in the art or are commercially available.

The reference drug (positive control drug) Dato-Dxd (datopotamab deruxtecan, DS-1062a) is an antibody-drug conjugate developed based on Daiichi Sankyo's Dxd-ADC platform, using Trop-2 antibody (datopotamab) and topoisomerase inhibitor (Dxd). Its structure and preparation method are described in detail in WO2014057687A, and it can be prepared according to the method of that patent or is commercially available.

The reference drug (positive control drug) Trodelvy is an antibody-drug conjugate developed by Gilead using the Trop-2 antibody (sacituzumab) and SN38. A similar control drug is commercially available.

Other starting materials of the present disclosure are commercially available or can be prepared by methods known in the art. For example, the toxin used in the present disclosure is triptolide, which was purchased from the National Institutes for Food and Drug Control, CAT#111567-201404, ID: DP03-BYVB, M.wt: 360.4. Exatecan of the present disclosure is commercially available, CAS No.: 171335-80-1. Exatecan mesylate is commercially available, CAS No.: 169869-90-3.

Compound 7 (147270-MC-VA-PAB-Exatecan) is commercially available, CAS No.: 2680543-57-9, with the structure shown below:

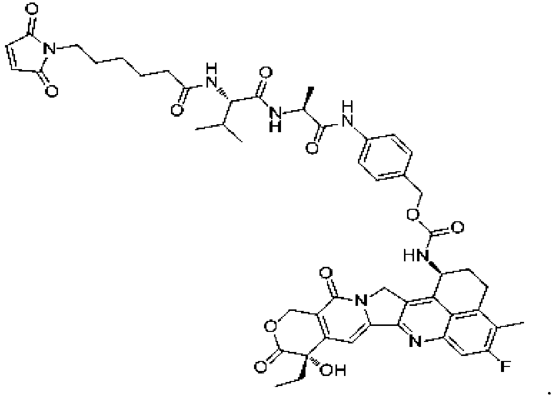

Compound 8 (HY-13631E) is commercially available, CAS No.: 1599440-13-7, with the structure shown below:

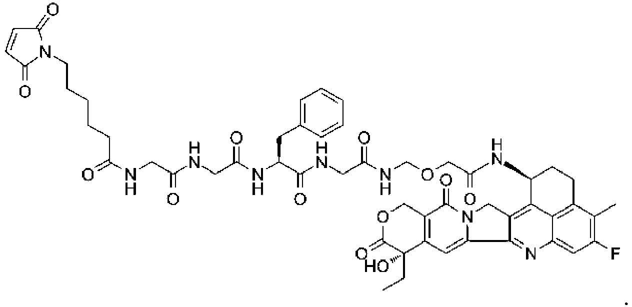

### Example 1. Preparation of Compound 1 (DBCO-PEG6-VA-PAB-Exatecan)

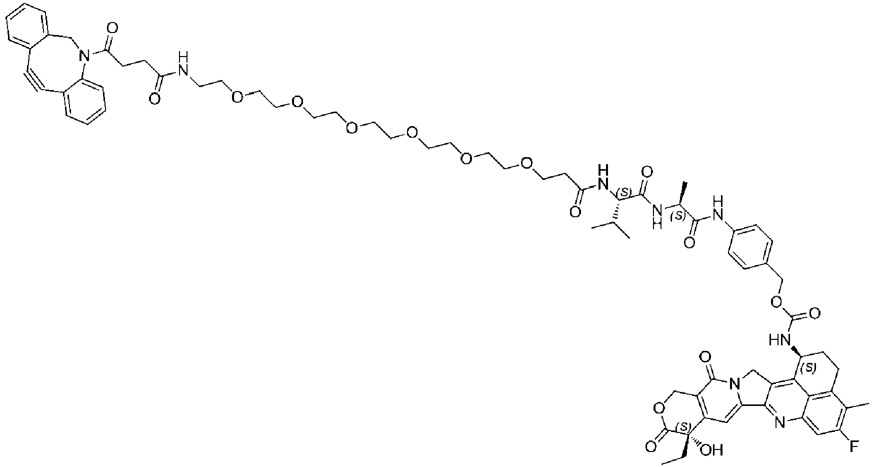

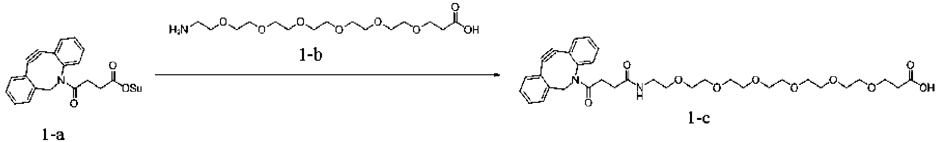

Compound 1-a (150 mg, 0.37 mmol) and compound 1-b (132 mg, 0.37 mmol) were dissolved in 1.5 mL of DMF, and then, TEA (75 mg, 0.74 mmol) was slowly added dropwise. After the addition, the mixture was reacted at room temperature for 2 h. Post-treatment: The reaction liquid was directly purified by a reversed-phase column (H₂O:ACN = 60%:40%) and lyophilized to give a yellow oil (compound 1-c, 300 mg, yield: 94%).

Compound 1-c (135 mg, 0.21 mmol) and HATU (96 mg, 0.25 mmol) were dissolved in 4 mL of DMF, and then, DIEA (40 mg, 0.31 mol) was added. The mixture was stirred at room temperature for 15 min, and compound 1-d (162 mg, 0.21 mmol) was added. The mixture was stirred at room temperature for 1 h. Post-treatment: The reaction liquid was directly subjected to reversed-phase HPLC preparation and lyophilized to give an off-white solid (130 mg, yield: 43%).

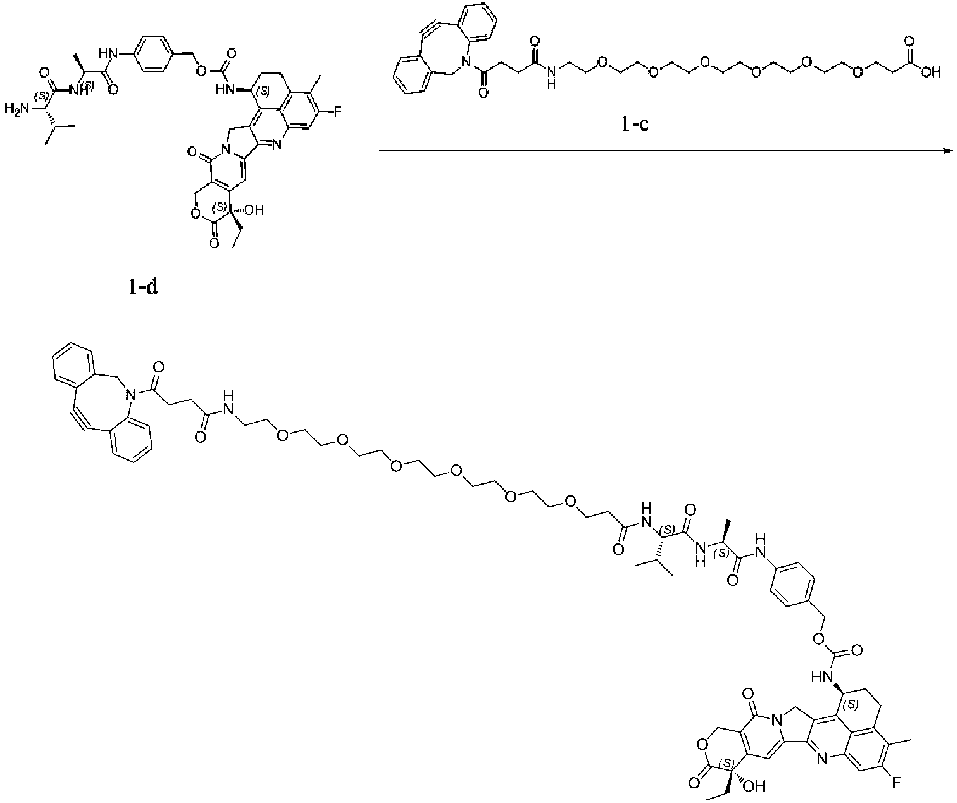

NMR: ¹H NMR (400 MHz, DMSO) δ 9.92 (s, 1H), 8.17 (d, *J* = 6.7 Hz, 1H), 8.06 (d, *J* = 7.9 Hz, 1H), 7.87 (d, *J =* 8.6 Hz, 1H), 7.79-7.72 (m, 2H), 7.67 (d, *J =* 6.7 Hz, 1H), 7.60 (d, *J =* 8.5 Hz, 3H), 7.47 (dd, *J* = 15.6, 7.0 Hz, 3H), 7.36 (d, *J =* 7.8 Hz, 3H), 7.33-7.26 (m, 3H), 6.51 (s, 1H), 5.44 (s, 2H), 5.28 (s, 3H), 5.09-4.99 (m, 3H), 4.38 (t, *J* = 7.1 Hz, 1H), 4.23-4.17 (m, 1H), 3.61-3.55 (m, 3H), 3.49-3.43 (m, 22H), 3.28 (d, *J =* 5.8 Hz, 2H), 3.10-3.04 (m, 2H), 2.61-2.54 (m, 1H), 2.44 (d, *J =* 6.7 Hz, 1H), 2.37 (s, 3H), 2.27-1.70 (m, 9H), 1.30 (d, *J =* 7.1 Hz, 3H), 0.88 (t, *J =* 6.2 Hz, 6H), 0.83 (d, *J =* 6.7 Hz, 3H).

### Example 2. Preparation of Compound 2 (MC-PEG2-GGFG-Triptolide)

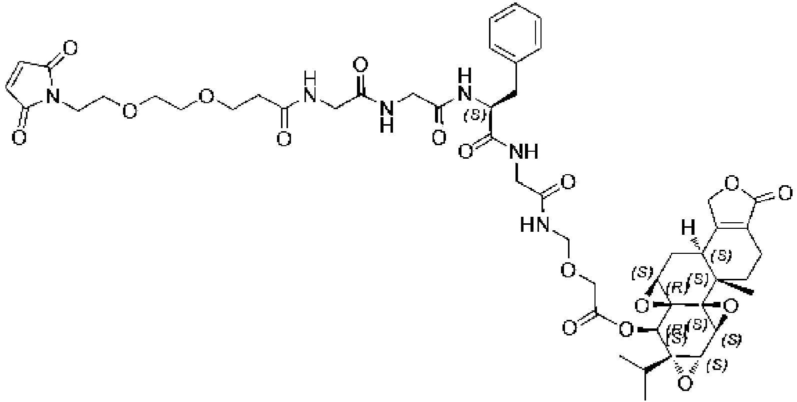

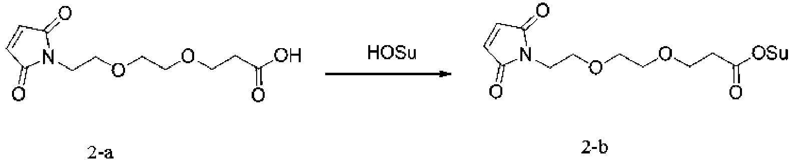

Compound 2-a (10 g, 38.9 mmol) and HOSU (5.37 g, 46.7 mmol) were dissolved in 100 mL of DCM, and EDCI (11.2 g, 58.5 mmol) was added. The mixture was stirred at room temperature for 1 h. After no starting materials were left, as detected by LCMS, the reaction liquid was concentrated, loaded onto silica gel, and purified by column chromatography (DCM:EA = 10:1) to give a product, which was concentrated to give a light yellow oil (compound 2-b, 12 g, yield: 87%).

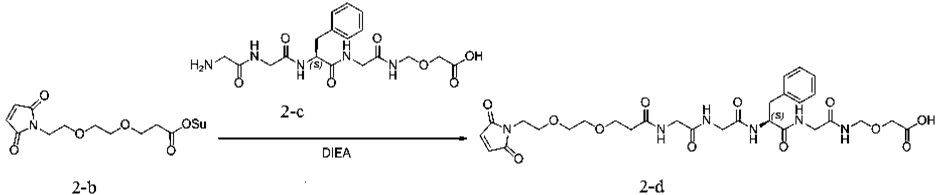

Compound 2-b (8 g, 22.6 mmol) and compound 2-c (9.56 g, 22.6 mmol) were placed in a three-necked flask and dissolved in 90 mL of (water : acetonitrile = 2:1), and DIEA (2.33 g, 18 mmol) was added at 0-10°C under a nitrogen atmosphere. After the addition, the mixture was warmed to room temperature and reacted for 16 h. After the reaction was completed, as monitored by LCMS, the reaction liquid was extracted once with EA, and the aqueous phase was adjusted to pH 2 with 0.5 mol/L HCl and extracted four times with (DCM:IPA = 4:1). The organic phases were combined, dried over anhydrous sodium sulfate, concentrated, and slurried with EA to give a white solid (10.5 g, yield: 70%).

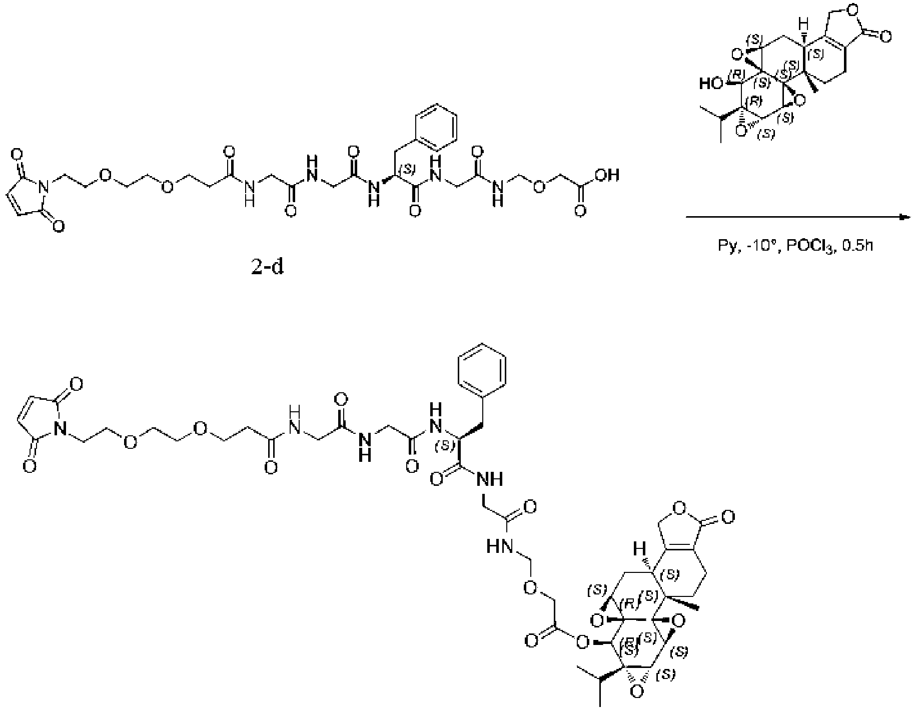

Compound 2-d (1.62 g, 2.44 mmol) and triptolide (800 mg, 2.22 mmol) were placed in a three-necked flask and dissolved in 20 mL of Py (pyridine), and POCl₃ (3.4 g, 22.2 mmol) was added at - 10°C to 5°C under a nitrogen atmosphere. The mixture was reacted for 5 min with the temperature maintained. After the reaction was completed, as monitored by LCMS, the reaction liquid was added dropwise to 400 mL of ice-cold 1 mol/L HCl, and extraction was performed with (DCM:IPA = 4:1) (200 × 3). The organic phases were combined, dried over anhydrous sodium sulfate, concentrated, and subjected to preparation. The prepared solution was lyophilized to give a yellow solid (1.1 g, yield: 44%).

NMR: ¹H NMR (400 MHz, DMSO) δ 8.61 (t, *J =* 6.7 Hz, 1H), 8.30 (t, *J =* 5.7 Hz, 1H), 8.18-8.05 (m, 2H), 7.99 (t, *J =* 5.7 Hz, 1H), 7.29-7.22 (m, 4H), 7.22-7.15 (m, 1H), 7.01 (s, 2H), 5.03 (s, 1H), 4.90-4.73 (m, 2H), 4.70-4.58 (m, 2H), 4.54-4.45 (m, 1H), 4.17 (s, 2H), 3.95 (d, *J =* 3.0 Hz, 1H), 3.80-3.66 (m, 6H), 3.63-3.48 (m, 8H), 3.47-3.43 (m, 2H), 3.43-3.39 (m, 2H), 3.06 (dd, *J* = 13.8, 4.4 Hz, 1H), 2.80 (dd, *J =* 13.9, 9.9 Hz, 1H), 2.68-2.58 (m, 1H), 2.36 (t, *J =* 6.5 Hz, 2H), 2.28-2.18 (m, 1H), 2.16-2.06 (m, 1H), 2.00-1.90 (m, 1H), 1.90-1.75 (m, 2H), 1.34-1.23 (m, 2H), 0.92-0.84 (m, 6H), 0.74 (d, *J =* 6.8 Hz, 3H).

### Example 3. Preparation of Compound 3 (DBCO-PEG4-GGFG-Triptolide)

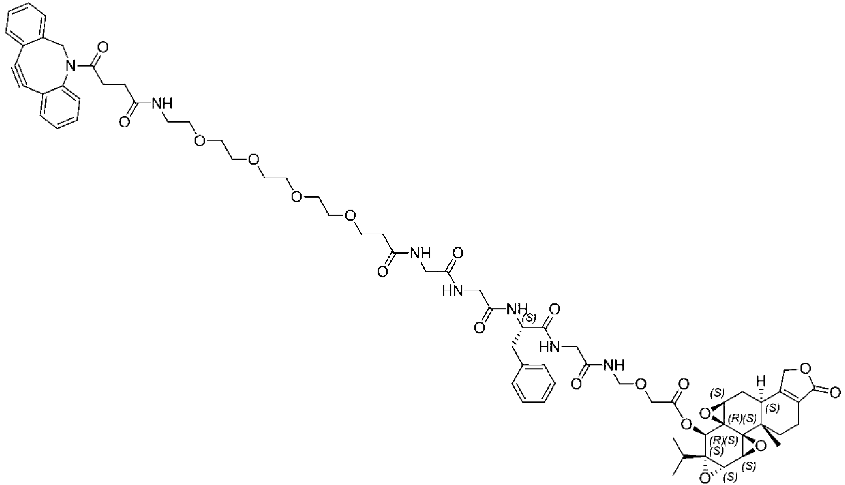

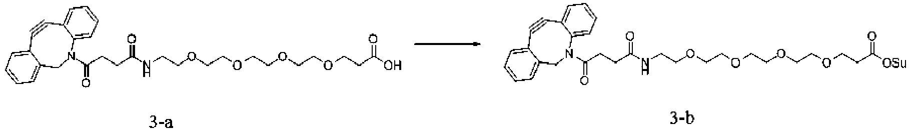

Compound 3-a (2 g, 3.62 mmol), NHS (624 mg, 5.42 mmol), and 2,6-dimethylpyridine were dissolved in 80 mL of dimethylacetamide, and then, EDCI (1.04 g, 5.43 mmol) was added at room temperature. After the addition, the mixture was reacted overnight at room temperature. Post-treatment: The reaction liquid was poured into water, and the mixture was extracted with EA. The organic phase was washed with saturated brine, dried over anhydrous sodium sulfate, and concentrated by rotary evaporation to give a yellow oil (compound 3-b, 2.4 g).

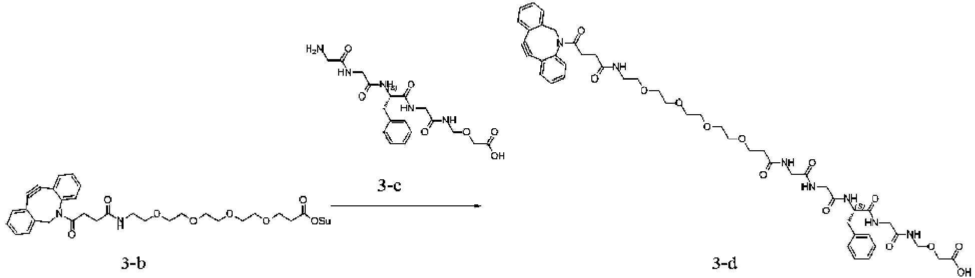

Compound 3-b (2.0 g, 3.07 mmol) and compound 3-d (1.3 g, 3.07 mmol) were dissolved in 10 mL of DMF, and then, TEA (620 mg, 6.14 mol) was added. The mixture was stirred at room temperature for 1 h. Post-treatment: The reaction liquid was directly purified by a reversed-phase column (H₂O:CAN = 60%:40%) and lyophilized to give a pale yellow hygroscopic solid (compound 3-d, 1.2, two-step yield: 50%).

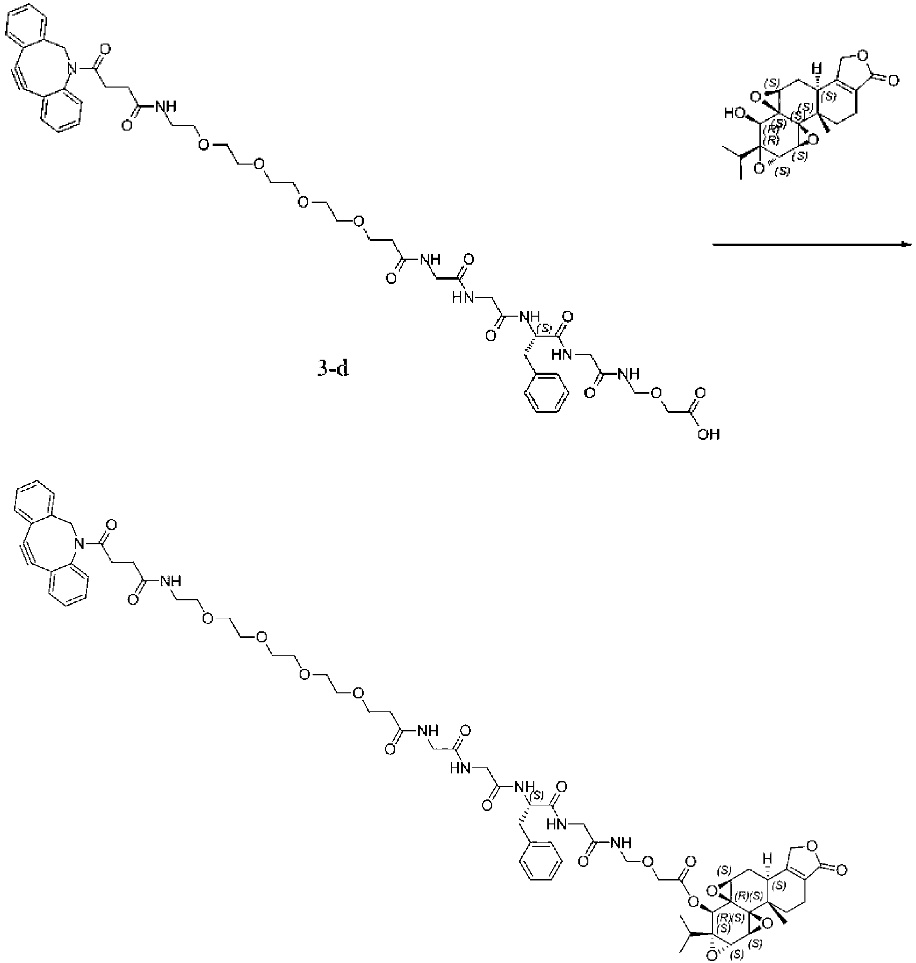

Compound 3-d (1.2 g, 1.23 mmol) and triptolide (446 mg, 1.23 mmol) were dissolved in 18 mL of pyridine, and the mixture was cooled to -30°C under a nitrogen atmosphere. POCl₃ (569 mg, 3.71 mmol) was slowly added dropwise, and after the dropwise addition, the mixture was reacted for 0.5 h with the temperature maintained at -10°C to -30°C. Post-treatment: The reaction liquid was cooled to -30°C and then quickly loaded onto a reversed-phase column for purification (H₂O:ACN = 50%:50%). The product was lyophilized to give a white solid (1.5 g), which was subjected to reversed-phase preparation to give a white solid (1.1 g, yield: 68%).

NMR: ¹H NMR (400 MHz, DMSO) δ 8.61 (t, J = 6.8 Hz, 1H), 8.30 (t, J = 5.8 Hz, 1H), 8.18-8.08 (m, 2H), 7.99 (t, J = 5.7 Hz, 1H), 7.75 (t, J = 5.5 Hz, 1H), 7.70-7.65 (m, 1H), 7.62 (d, J = 7.0 Hz, 1H), 7.52-7.42 (m, 3H), 7.40-7.28 (m, 3H), 7.27-7.22 (m, 4H), 7.18 (td, J = 5.9, 2.5 Hz, 1H), 5.03 (t, J = 7.0 Hz, 2H), 4.89-4.72 (m, 2H), 4.69-4.58 (m, 2H), 4.50 (td, J = 9.1, 4.3 Hz, 1H), 4.17 (s, 2H), 3.95 (d, J = 3.1 Hz, 1H), 3.76 (dt, J = 17.3, 5.7 Hz, 3H), 3.71-3.67 (m, 3H), 3.60 (dd, J = 12.7, 6.2 Hz, 5H), 3.48-3.43 (m, 12H), 3.30 (s, 2H), 3.13-3.02 (m, 3H), 2.80 (dd, J = 13.8, 9.7 Hz, 1H), 2.58 (dd, J = 16.1, 7.9 Hz, 2H), 2.38 (t, J = 6.5 Hz, 2H), 2.23 (ddd, J = 15.4, 10.2, 5.5 Hz, 2H), 2.16-2.06 (m, 1H), 2.03-1.92 (m, 2H), 1.89-1.72 (m, 3H), 1.29 (d, J = 5.2 Hz, 2H), 0.91-0.85 (m, 6H), 0.74 (d, J = 6.8 Hz, 3H).

### Example 4. Preparation of Compound 4 (MC-GGFG-Triptolide)

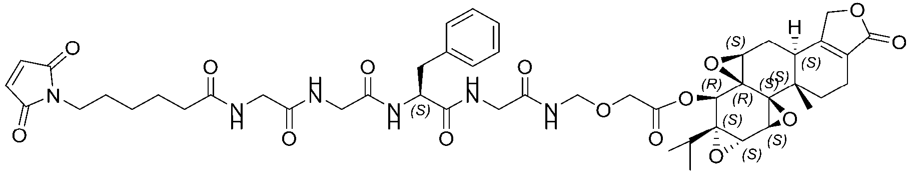

Triptolide (36 mg, 0.1 mmol) and compound 4-a (62 mg, 0.1 mmol) were added to pyridine (1.5 mL, dry). The mixture was stirred at -10°C under a nitrogen atmosphere, followed by the addition of POCl₃ (46 mg, 0.3 mmol). The mixture was then stirred at -10°C under a N₂ atmosphere for 0.5 h and purified by HPLC to give a white solid (20 mg, yield: 21%).

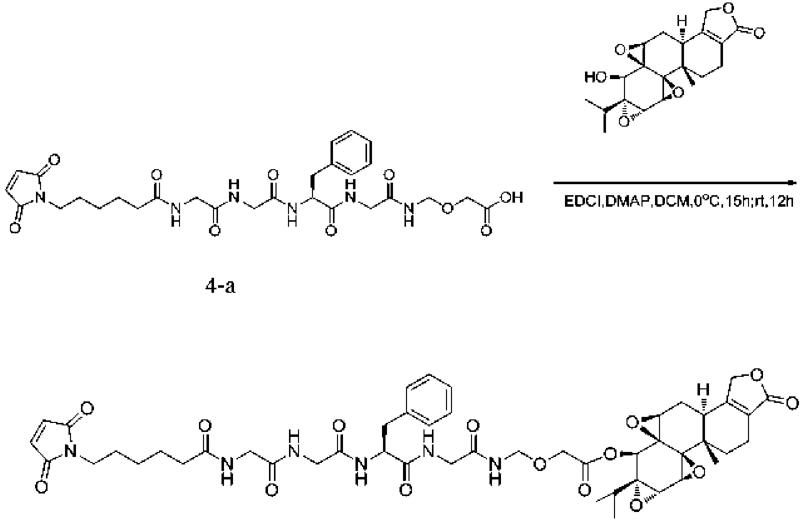

NMR: ¹H NMR (400 MHz, DMSO) δ 8.62 (d, J = 6.8 Hz, 1H), 8.32 (t, J = 5.7 Hz, 1H), 8.18-8.05 (m, 2H), 8.01 (t, J = 5.6 Hz, 1H), 7.35-7.15 (m, 5H), 7.02 (s, 2H), 5.06 (s, 1H), 4.92-4.77 (m, 2H), 4.72-4.58 (m, 2H), 4.52 (td, J = 9.2, 4.6 Hz, 1H), 4.19 (s, 2H), 3.97 (d, J = 3.2 Hz, 1H), 3.83-3.53 (m, 8H), 3.40 (d, J = 7.0 Hz, 2H), 3.08 (dd, J = 13.8, 4.5 Hz, 1H), 2.82 (dd, J = 13.7, 9.8 Hz, 1H), 2.62 (s, 1H), 2.31-2.20 (m, 1H), 2.18-2.06 (m, 3H), 1.88 (ddd, J = 33.7, 27.6, 17.1 Hz, 3H), 1.50 (dq, J = 14.6, 7.3 Hz, 4H), 1.39-1.28 (m, 2H), 1.21 (dt, J = 15.1, 7.6 Hz, 2H), 0.94-0.85 (m, 6H), 0.77 (t, J = 5.9 Hz, 3H).

### Example 5. Preparation of Compound 5 (DBCO-PEG4-VA-PAB-Exatecan)

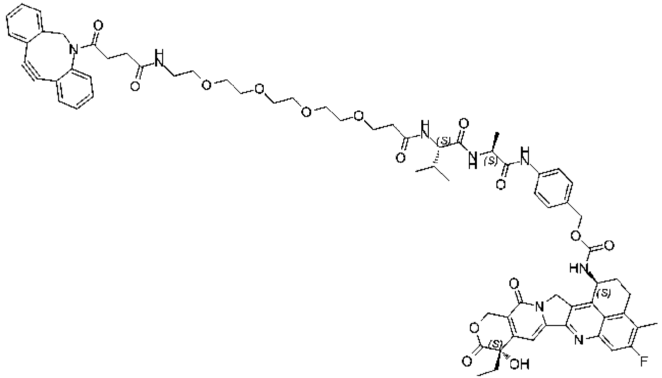

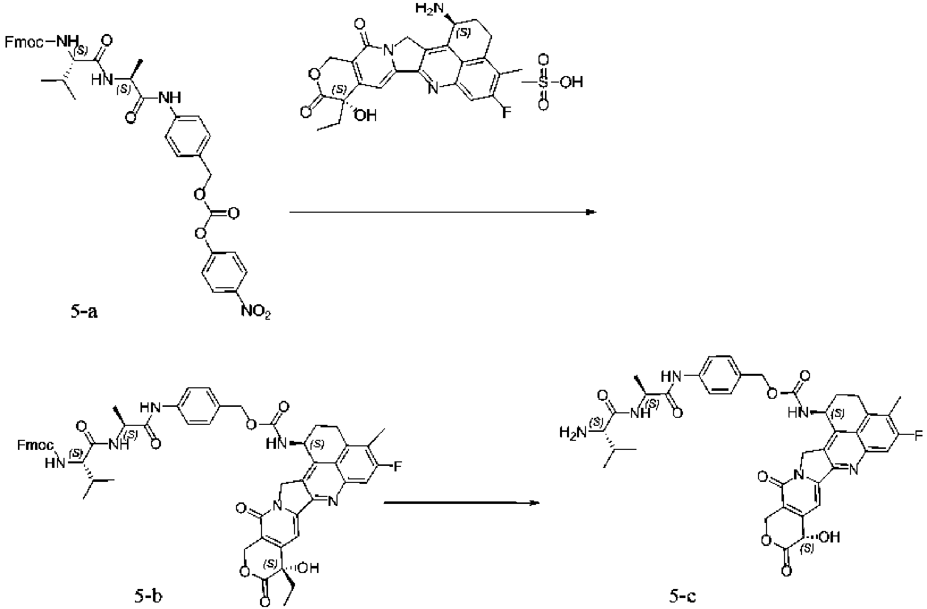

Compound 5-a (1.8 g, 2.64 mmol) and exatecan mesylate (1.54 g, 2.91 mmol) were dissolved in 18 mL of DMSO, and then, DIEA (1 g, 7.92 mmol) was slowly added. After the addition, the mixture was reacted at room temperature for 4 h. After the starting materials were consumed by monitoring the reaction, TEA (1.8 mL) was added, and the mixture was stirred at room temperature for 3 h. Post-treatment: The reaction liquid was directly purified by a reversed-phase column (H₂O:ACN = 65%:35%) and lyophilized to give a yellowish-green powder (compound 5-c, 1.98 g, yield: 99%).

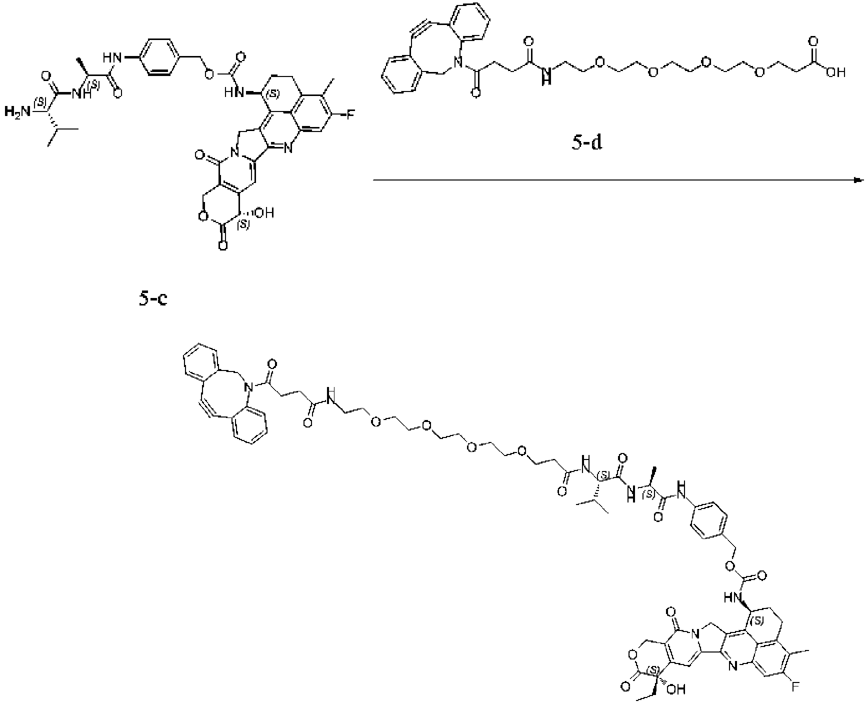

Compound 5-c (409 mg, 0.21 mmol) and HATU (247 mg, 043 mmol) were dissolved in 3 mL of DMF, and then, DIEA (105 mg, 0.54 mol) was added. The mixture was stirred at room temperature for 15 min, and compound 5-d (300 mg, 0.36 mmol) was added. The mixture was stirred at room temperature for 2 h. Post-treatment: The reaction liquid was directly subjected to reversed-phase HPLC preparation and lyophilized to give an off-white solid (120 mg, yield: 17%).

NMR: ¹H NMR (400 MHz, DMSO) δ 9.93 (s, 1H), 8.17 (d, *J =* 6.9 Hz, 1H), 8.06 (d, *J =* 8.4 Hz, 1H), 7.87 (d, *J =* 8.6 Hz, 1H), 7.76 (t, *J =* 9.9 Hz, 2H), 7.66 (d, *J =* 6.3 Hz, 1H), 7.60 (d, *J =* 8.4 Hz, 3H), 7.50-7.43 (m, 3H), 7.36 (d, *J =* 8.7 Hz, 3H), 7.33-7.26 (m, 3H), 6.51 (s, 1H), 5.44 (s, 2H), 5.28 (d, *J* = 4.3 Hz, 3H), 5.10-4.98 (m, 3H), 4.38 (t, *J* = 7.1 Hz, 1H), 4.24-4.17 (m, 1H), 3.61-3.55 (m, 3H), 3.52-3.39 (m, 14H), 3.28 (d, *J =* 5.9 Hz, 2H), 3.08 (dd, *J=* 10.0, 5.3 Hz, 2H), 2.58 (dd, *J =* 16.1, 7.9 Hz, 1H), 2.44 (t, *J* = 6.7 Hz, 1H), 2.37 (s, 3H), 2.27-1.71 (m, 9H), 1.30 (d, *J* = 7.1 Hz, 3H), 0.88 (t, *J* = 6.2 Hz, 6H), 0.83 (d, *J =* 6.8 Hz, 3H).

### Example 6. Preparation of Compound 6 (MC-PEG2-VA-PAB-Exatecan)

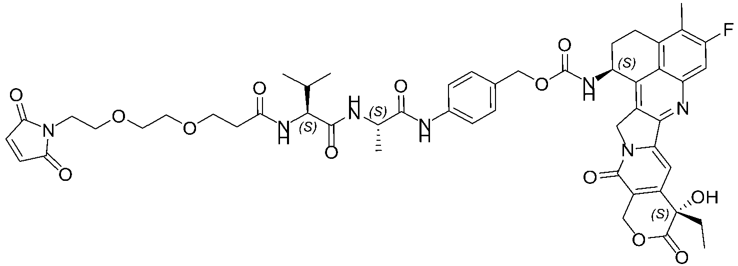

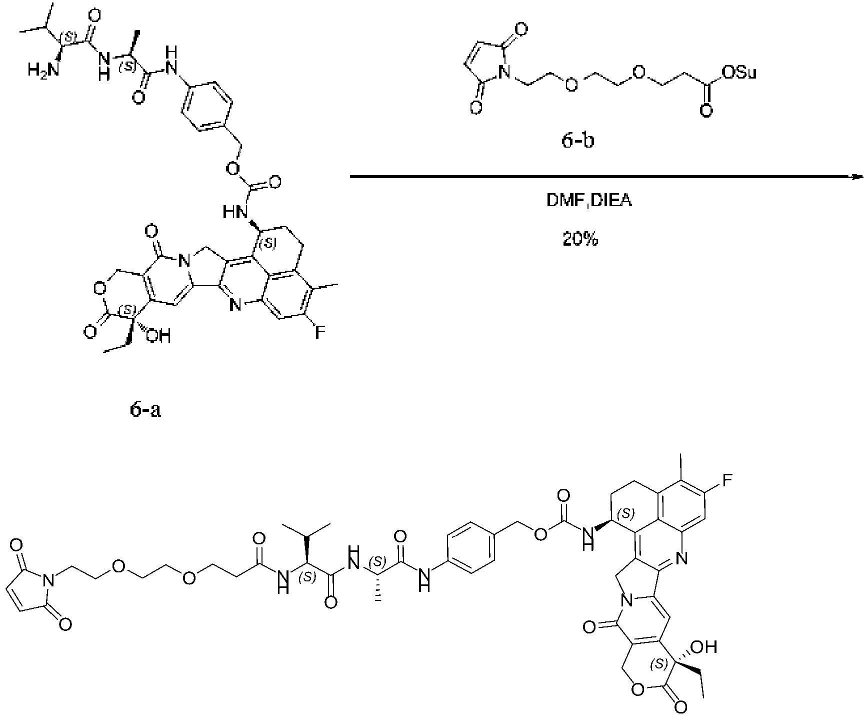

Compound 6-a (480 mg, 0.64 mmol) was added to a 100-mL three-necked flask at room temperature, and DMF (20 mL) was added for dissolution, followed by the addition of compound 6-b (237.2 mg, 0.67 mmol). The reaction system was cooled to 0°C, and DIEA (237.2 mg, 1.28 mmol) was slowly added dropwise. The reaction system was stirred at 0°C for 2 h. After the reaction was completed, as detected by LC-MS, acetic acid (0.5 mL) was added to the reaction liquid. The mixture was first subjected to reversed-phase separation (TFA/MeCN) and lyophilization. After further preparative purification and lyophilization, a yellow solid (compound 6, 127 mg, yield: 20%) was obtained.

NMR: ¹H NMR (400 MHz, DMSO) δ 9.95 (s, 1H), 8.19 (d, *J =* 6.8 Hz, 1H), 8.08 (d, *J =* 8.8 Hz, 1H), 7.88 (d, *J* = 8.8 Hz, 1H), 7.79 (d, *J =* 10.8 Hz, 1H), 7.62 (d, *J =* 8.4 Hz, 2H), 7.39 (d, *J =* 8.4 Hz, 2H), 7.33 (s, 1H), 7.04 (s, 2H), 5.47 (s, 2H), 5.30 (d, *J =* 4.4 Hz, 3H), 5.10 (s, 2H), 4.44-4.36 (m, 1H), 4.25-4.19 (m, 1H), 3.58 (dd, *J* = 11.2, 5.6 Hz, 5H), 3.52 (d, *J =* 5.2 Hz, 3H), 3.36-3.07 (m, 3H), 2.49-2.36 (m, 5H), 2.20 (d, *J =* 13.2 Hz, 2H), 2.04-1.85 (m, 3H), 1.32 (d, *J =* 7.2 Hz, 3H), 0.92-0.84 (m, 9H).

### Example 7. Preparation of Triptolide (Compound 2)/Exatecan (Compound 1) Double-Toxin ADC (ADC-1)

The samples were added according to the following reaction system to enable the final concentration of the antibody (sacituzumab) to be 10 mg/mL, the final concentration of MnCl₂ to be 10 mmol/L, the final concentration of Tris-HCl (pH 7.5) to be 10 mmol/L, and the final concentration of UDP-GalNAz to be 5 mmol/L. GalT1 was added at a ratio of 5 mg of GalT1/1 g of mAb to 50 mg of GalT1/1 g of mAb. The mixture was shaken in a constant-temperature shaker at 30°C for ≥12 h to complete the azidation reaction of the antibody. Substances, including UDP-GalNAz, GalT1, and Mn²⁺, introduced during the azidation reaction were removed via chromatography. Compound 1 was added according to the following reaction system to enable the final concentration of the azidated antibody to be 10 mg/mL, the final concentration of DMSO to be 10%, and the molar ratio of compound 1 to the antibody to be 5-15. The mixture was shaken in a constant-temperature shaker at 30°C for ≥12 h to complete glycosyl conjugation between the antibody and the toxin. TCEP was directly added to the above reaction liquid for antibody reduction at a molar ratio of TCEP to the antibody of 5. The mixture was shaken in a constant-temperature shaker at 30°C for 2 h for antibody reduction. Compound 2 was added to complete sulfhydryl conjugation, with the molar ratio of compound 2 to the antibody being 5. The mixture was stirred at 2-8°C for 2 h to complete sulfhydryl conjugation. Substances, including TCEP, DMSO, compound 1, and compound 2, introduced during the process were removed via concentration and buffer exchange. The sample was exchanged into a 4.29 g/L MES buffer (pH 6.5). Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, 2 µL of 1 mol/L DTT was added, and ultrapure water was supplemented to make a final volume of 100 µL. The mixture was reduced at room temperature for 30 min and then directly detected by LC-MS. After normalization according to mass spectrometry signal intensities, the average DAR value of the sample was calculated. The DAR value of triptolide was 3.7, and the DAR value of exatecan was 3.6.

### Example 8. Preparation of Exatecan (Compound 7)/Triptolide (Compound 3) Double-Toxin ADC (ADC-2)

The samples were added according to the following reaction system to enable the final concentration of the antibody (sacituzumab) to be 10 mg/mL, the final concentration of MnCl₂ to be 10 mmol/L, the final concentration of Tris-HCl (pH 7.5) to be 10 mmol/L, and the final concentration of UDP-GalNAz to be 5 mmol/L. GalT1 was added at a ratio of 5 mg of GalT1/1 g of mAb to 50 mg of GalT1/1 g of mAb. The mixture was shaken in a constant-temperature shaker at 30°C for ≥12 h to complete the azidation reaction of the antibody. Substances, including UDP-GalNAz, GalT1, and Mn²⁺, introduced during the azidation reaction were removed via chromatography. Compound 3 was added according to the following reaction system to enable the final concentration of the azidated antibody to be 10 mg/mL, the final concentration of DMSO to be 10%, and the molar ratio of compound 3 to the antibody to be 5-15. The mixture was shaken in a constant-temperature shaker at 30°C for ≥12 h to complete glycosyl conjugation between the antibody and the toxin. TCEP was directly added to the above reaction liquid for antibody reduction at a molar ratio of TCEP to the antibody of 4.5. The mixture was shaken in a constant-temperature shaker at 30°C for 2 h for antibody reduction. Compound 7 was added to complete sulfhydryl conjugation, with the molar ratio of compound 7 to the antibody being 5. The mixture was stirred at 2-8°C for 2 h to complete sulfhydryl conjugation. Substances, including TCEP, DMSO, compound 3, and compound 7, introduced during the process were removed via concentration and buffer exchange. The sample was exchanged into a 4.29 g/L MES buffer (pH 6.5), and the protein concentration was adjusted to 20 mg/mL. Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, 2 µL of 1 mol/L DTT was added, and ultrapure water was supplemented to make a final volume of 100 µL. The mixture was reduced at room temperature for 30 min and then directly detected by LC-MS. After normalization according to mass spectrometry signal intensities, the average DAR value of the sample was calculated. The DAR value of exatecan was 4.2, and the DAR value of triptolide was 3.7.

### Example 9. Preparation of Triptolide (Compound 4)/Exatecan (Compound 1) Double-Toxin ADC (ADC-3)

The samples were added according to the following reaction system to enable the final concentration of the antibody (sacituzumab) to be 10 mg/mL, the final concentration of MnCl₂ to be 10 mmol/L, the final concentration of Tris-HCl (pH 7.5) to be 10 mmol/L, and the final concentration of UDP-GalNAz to be 5 mmol/L. GalT1 was added at a ratio of 5 mg of GalT1/1 g of mAb to 50 mg of GalT1/1 g of mAb. The mixture was shaken in a constant-temperature shaker at 30°C for ≥12 h to complete the azidation reaction of the antibody. Substances, including UDP-GalNAz, GalT1, and Mn²⁺, introduced during the azidation reaction were removed via chromatography. Compound 1 was added according to the following reaction system to enable the final concentration of the azidated antibody to be 10 mg/mL, the final concentration of DMSO to be 10%, and the molar ratio of compound 1 to the antibody to be 5-15. The mixture was shaken in a constant-temperature shaker at 30°C for ≥12 h to complete glycosyl conjugation between the antibody and the toxin. TCEP was directly added to the above reaction liquid for antibody reduction at a molar ratio of TCEP to the antibody of 5. The mixture was shaken in a constant-temperature shaker at 30°C for 2 h for antibody reduction. Compound 4 was added to complete sulfhydryl conjugation, with the molar ratio of compound 4 to the antibody being 5. The mixture was stirred at 2-8°C for 2 h to complete sulfhydryl conjugation. Substances, including TCEP, DMSO, compound 1, and compound 4, introduced during the process were removed via concentration and buffer exchange. The sample was exchanged into a 4.29 g/L MES buffer (pH 6.5). Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, 2 µL of 1 mol/L DTT was added, and ultrapure water was supplemented to make a final volume of 100 µL. The mixture was reduced at room temperature for 30 min and then directly detected by LC-MS. After normalization according to mass spectrometry signal intensities, the average DAR value of the sample was calculated. The DAR value of triptolide was 4.5, and the DAR value of exatecan was 3.8.

### Example 10. Preparation of Exatecan (Compound 6)/Triptolide (Compound 3) Double-Toxin ADC (ADC-4)

The samples were added according to the following reaction system to enable the final concentration of the antibody (sacituzumab) to be 10 mg/mL, the final concentration of MnCl₂ to be 10 mmol/L, the final concentration of Tris-HCl (pH 7.5) to be 10 mmol/L, and the final concentration of UDP-GalNAz to be 5 mmol/L. GalT1 was added at a ratio of 5 mg of GalT1/1 g of mAb to 50 mg of GalT1/1 g of mAb. The mixture was shaken in a constant-temperature shaker at 30°C for ≥12 h to complete the azidation reaction of the antibody. Substances, including UDP-GalNAz, GalT1, and Mn²⁺, introduced during the azidation reaction were removed via chromatography. Compound 3 was added according to the following reaction system to enable the final concentration of the azidated antibody to be 10 mg/mL, the final concentration of DMSO to be 10%, and the molar ratio of compound 3 to the antibody to be 5-15. The mixture was shaken in a constant-temperature shaker at 30°C for ≥12 h to complete glycosyl conjugation between the antibody and the toxin. TCEP was directly added to the above reaction liquid for antibody reduction at a molar ratio of TCEP to the antibody of 4.5. The mixture was shaken in a constant-temperature shaker at 30°C for 2 h for antibody reduction. Compound 6 was added to complete sulfhydryl conjugation, with the molar ratio of compound 6 to the antibody being 5. The mixture was stirred at 2-8°C for 2 h to complete sulfhydryl conjugation. Substances, including TCEP, DMSO, compound 3, and compound 6, introduced during the process were removed via concentration and buffer exchange. The sample was exchanged into a 4.29 g/L MES buffer (pH 6.5), and the protein concentration was adjusted to 20 mg/mL. Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, 2 µL of 1 mol/L DTT was added, and ultrapure water was supplemented to make a final volume of 100 µL. The mixture was reduced at room temperature for 30 min and then directly detected by LC-MS. After normalization according to mass spectrometry signal intensities, the average DAR value of the sample was calculated. The DAR value of exatecan was 3.7, and the DAR value of triptolide was 3.6.

### Example 11. Preparation of Exatecan (Compound 1) ADC (ADC-5)

The samples were added according to the following reaction system to enable the final concentration of the antibody (sacituzumab) to be 10 mg/mL, the final concentration of MnCl₂ to be 10 mmol/L, the final concentration of Tris-HCl (pH 7.5) to be 10 mmol/L, and the final concentration of UDP-GalNAz to be 5 mmol/L. GalT1 was added at a ratio of 5 mg of GalT1/1 g of mAb to 50 mg of GalT1/1 g of mAb. The mixture was shaken in a constant-temperature shaker at 30°C for ≥12 h to complete the azidation reaction of the antibody. Substances, including UDP-GalNAz, GalT1, and Mn²⁺, introduced during the azidation reaction were removed via chromatography. Compound 1 was added according to the following reaction system to enable the final concentration of the azidated antibody to be 10 mg/mL, the final concentration of DMSO to be 10%, and the molar ratio of compound 1 to the antibody to be 5-15. The mixture was shaken in a constant-temperature shaker at 30°C for ≥12 h to complete glycosyl conjugation between the antibody and the toxin. Substances, including DMSO and compound 1, introduced during the process were removed via concentration and buffer exchange. The sample was exchanged into a 4.29 g/L MES buffer (pH 6.5), and the protein concentration was adjusted to 20 mg/mL. Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, and ultrapure water was supplemented to make a final volume of 100 µL. The mixture was then directly detected by LC-MS. After normalization according to mass spectrometry signal intensities, the average DAR value of the sample was calculated. The DAR value of exatecan was 3.7.

### Example 12

The expression of HSP70 is upregulated in many tumors, which can cause tumor progression and resistance to chemotherapy. The relative expression level of HSP70 was used to investigate the inhibition effect of triptolide on the mRNA transcription level.

Experimental method: HCT-15 (human colorectal cancer) cells were grown in T75 to 90% confluency, digested with trypsin, and passaged into a 6-well plate. After overnight culture at 37°C, the cells were treated with Dxd (100 nM), Exatecan (100 nM), Triptolide (100 nM), Triptolide + Dxd (100 nM + 100 nM), or Triptolide + Exatecan (100 nM + 100 nM). The untreated group served as CTRL. After 24 h, the culture medium was discarded, and total RNA was extracted with Trizol at 500 µL/well. Following reverse transcription, real-time quantitative PCR was performed to detect the expression levels of HSP70 and ABAC1 with 18S as the internal reference. The primer sequences are shown as follows: ABAC1 F: ATGGCTACATGAGAGCGGAG (SEQ ID NO: 21); ABAC1 R: CGTTGCACCTCTCTGGTCC (SEQ ID NO: 22); HSP70 F: ACCAAGCAGACGCAGATCTTC (SEQ ID NO: 23); HSP70 R: CGCCCTCGTACACCTGGAT (SEQ ID NO: 24); 18S F: CTCGCTCCTCTCCCACTTG (SEQ ID NO: 25); 18S R: TGACCGGGTTGG TTTTGATC (SEQ ID NO: 26).

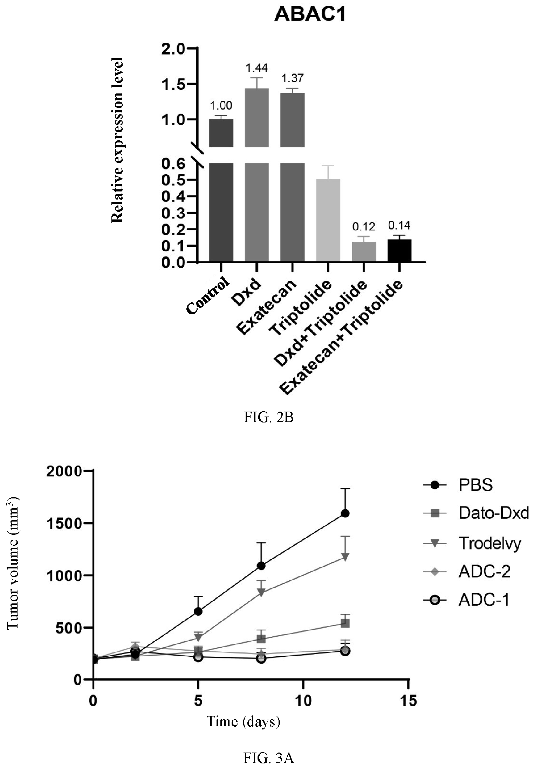

The results are shown in FIGs. 2A and 2B, and in FIG. 2A, the ordinate represents HSP70. HSP70 expression was significantly upregulated in HCT-15 cells upon stimulation with 100 nM Dxd or Exatecan, while HSP70 mRNA was significantly reduced after treatment with an equivalent molar concentration of Triptolide, indicating that triptolide is capable of inhibiting mRNA synthesis.

In FIG. 2B, the ordinate represents the expression level of the efflux pump gene ABAC1. As can be seen from the figure, Triptolide significantly reduced the expression of the efflux pump gene ABAC1 in HCT-15, resulting in enhanced sensitivity of cells to Dxd or Exatecan.

The combination of Triptolide and Exatecan resulted in significantly upregulated expression of HSP70 and ABAC1, demonstrating the synergy of the combination of triptolide and exatecan in anti-apoptotic protection and drug resistance.

### Example 13. Experiment on Efficacy in Human Colorectal Cancer Model (COLO205 Cells)

### Experimental Animals

NCG mice were purchased from GemPharmatech (Chengdu) Co., Ltd.

### Animal Modeling

COLO205 cells were resuspended in a RPMI 1640 culture medium or DMEM culture medium (total volume: 0.1 mL/mouse). The hair on the right anterior dorsal region of the experimental NCG mice was removed, followed by subcutaneous inoculation of 6 × 10⁶ to 1 × 10⁷ cells.

### Administration Regimen

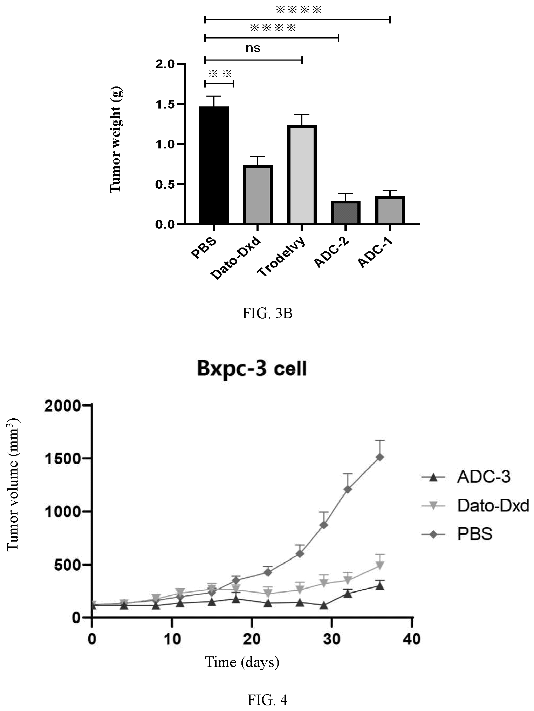

7 days after tumor inoculation, test samples (negative control: a PBS solution; test drugs: ADC-1 and ADC-2; positive controls: Dato-Dxd and Trodelvy) were administered to the mice by intravenous injection at an administration dose of 5 mg/kg, with 6 animals in each group. After the start of administration, the body weight and tumor size of the mice were measured twice a week. Calculation formula for tumor volume: Tumor volume (mm³) = 1/2 × (L × W²) (where L represents the long diameter and W represents the short diameter). The results of changes in the tumor volume are shown in FIG. 3A. After the experiment was completed, the mice were sacrificed, and the tumors were dissected and weighed. The results are shown in FIG. 3B (* indicates P < 0.05, ** indicates P < 0.01, *** indicates P < 0.001, and **** indicates P < 0.0001).

### Example 14. Tumor Inhibition Effect in Human Pancreatic Cancer Model (Bxpc-3 Cells)

### Experimental Animals

NCG mice were purchased from GemPharmatech (Chengdu) Co., Ltd.

### Animal Modeling

Bxpc-3 cells were resuspended in a RPMI 1640 culture medium or DMEM culture medium (total volume: 0.1 mL/mouse). The hair on the right anterior dorsal region of the experimental NCG mice was removed, followed by subcutaneous inoculation of 6 × 10⁶ to 1 × 10⁷ cells.

### Administration Regimen

17 days after tumor inoculation, test samples (negative control: a PBS solution; test drug: ADC-3; positive control: Dato-Dxd) were administered to the mice by intravenous injection at an administration dose of 5 mg/kg, with 6 animals in each group. On day 28 after inoculation, i.e., on day 11 after the first administration, a second administration was performed at a dose of 10 mg/kg. After the start of administration, the body weight and tumor size of the mice were measured twice a week. Calculation formula for tumor volume: Tumor volume (mm³) = 1/2 × (L × W²) (where L represents the long diameter and W represents the short diameter). The results of changes in the tumor volume are shown in FIG. 4.

### Example 15. Tumor Inhibition Effect in Human Colorectal Cancer Drug-Resistant Model (HCT-15-TROP2 Cells)

### Experimental Animals

NCG mice were purchased from GemPharmatech (Chengdu) Co., Ltd.

### Animal Modeling

HCT-15-TROP2 cells were resuspended in a RPMI 1640 culture medium or DMEM culture medium (total volume: 0.1 mL/mouse). The hair on the right anterior dorsal region of the experimental NCG mice was removed, followed by subcutaneous inoculation of 6 × 10⁶ to 1 × 10⁷ cells.

### Administration Regimen

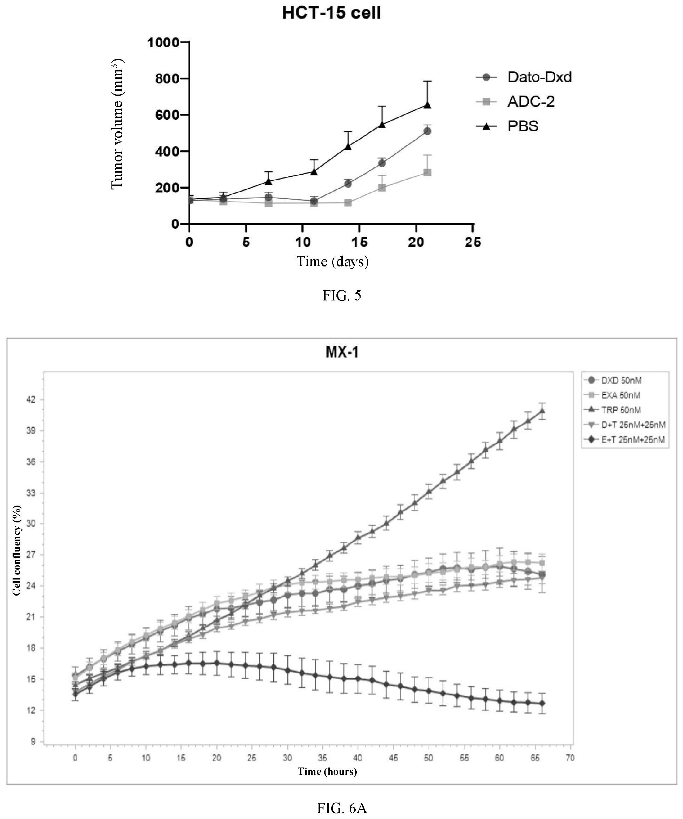

10 days after tumor inoculation, test samples (negative control: a PBS solution; test drug: ADC-2; positive control: Dato-Dxd) were administered to the mice by intravenous injection at an administration dose of 5 mg/kg, with 6 animals in each group. After the start of administration, the body weight and tumor size of the mice were measured twice a week. Calculation formula for tumor volume: Tumor volume (mm³) = 1/2 × (L × W²) (where L represents the long diameter and W represents the short diameter). The results of changes in the tumor volume are shown in FIG. 5.

### Example 16

This example was intended to investigate whether Triptolide and Exatecan, used alone or in combination at a ratio of 1:1, exhibited significant killing against tumor cells.

### Cell Preparation

MX-1 cells (human breast cancer cells), WiDr cells (human colorectal cancer cells), MFE-280 cells (human endometrial cancer cells), HeLa cells (human cervical cancer cells), HuH-7 cells (human liver cancer cells), NUGC-4 cells (human gastric cancer cells), and Calu-6 cells (human anaplastic carcinoma cells) in the logarithmic growth phase were taken, digested with 0.25% Trypsin-EDTA, resuspended in a RPMI 1640 culture medium containing 10% FBS, counted via trypan blue staining, and adjusted to a cell density of 1 × 10⁶ cells/mL. The cells were seeded into a 96-well cell culture plate at 100 µL/well, and adherent culture was performed for 4 h in an incubator at 37 °C with 5% CO₂.

### Drug Preparation:

Drug group 1: dilution of small-molecule toxin Dxd: Dxd was serially diluted 2-fold starting from 100 nM in RPMI 1640 medium containing 10% FBS, resulting in 5 concentrations in total, with a blank control included. The concentrations were 100 nM, 50 nM, 25 nM, 12.5 nM, and 6.25 nM.

Drug group 2: dilution of small-molecule toxin Exatecan (abbreviated as EXA): Exatecan was serially diluted 2-fold starting from 100 nM in RPMI 1640 medium containing 10% FBS, resulting in 5 concentrations in total, with a blank control included. The concentrations were 100 nM, 50 nM, 25 nM, 12.5 nM, and 6.25 nM.

Drug group 3: dilution of small-molecule toxin Triptolide (abbreviated as TRP): Triptolide was serially diluted 2-fold starting from 100 nM in RPMI 1640 medium containing 10% FBS, resulting in 5 concentrations in total, with a blank control included. The concentrations were 100 nM, 50 nM, 25 nM, 12.5 nM, and 6.25 nM.

Drug group 4: dilution of small-molecule toxins Dxd + Triptolide (abbreviated as DXD + TRP): An equal-ratio mixed solution of 100 nM Dxd + 100 nM Triptolide (i.e., a mixed solution of 2 mL of 100 nM Dxd + 2 mL of 100 nM Triptolide) was subjected to 2-fold serial dilution for 4 gradients, starting from an initial concentration of 50 nM, with a RPMI 1640 culture medium containing 10% FBS, resulting in 5 concentration gradients in total, with a blank control included. The concentrations were 50 nM, 25 nM, 12.5 nM, 6.25 nM, and 3.125 nM.

Drug group 5: dilution of small-molecule toxins Exatecan + Triptolide (abbreviated as EXA + TRP): An equal-ratio mixed solution of 100 nM Exatecan + 100 nM Triptolide (i.e., a mixed solution of 2 mL of 100 nM Exatecan + 2 mL of 100 nM Triptolide) was subjected to 2-fold serial dilution for 4 gradients, starting from an initial concentration of 50 nM, with a RPMI 1640 culture medium containing 10% FBS, resulting in 5 concentration gradients in total, with a blank control included. The concentrations were 50 nM, 25 nM, 12.5 nM, 6.25 nM, and 3.125 nM.

### Sample Addition:

100 µL of each of the diluted samples of Dxd, Exatecan, Triptolide, Dxd + Triptolide, and Exatecan + Triptolide at different concentration gradients was transferred to culture plates seeded with MX-1, WiDr, MFE-280, HeLa, HuH-7, NUGC-4, and Calu-6 cells, with 2 replicate wells set. There were 5 sample groups in total: the single Dxd group, the single Exatecan group, the single Triptolide group, the Dxd + Triptolide group, and the Exatecan + Triptolide group. The final concentrations of the single Dxd group were 50 nM, 25 nM, 12.5 nM, 6.25 nM, and 3.125 nM; the final concentrations of the single Exatecan group were 50 nM, 25 nM, 12.5 nM, 6.25 nM, and 3.125 nM; the final concentrations of the single Triptolide group were 50 nM, 25 nM, 12.5 nM, 6.25 nM, and 3.125 nM; the final concentrations of the Dxd + Triptolide group and the Exatecan + Triptolide group were 25 nM, 12.5 nM, 6.25 nM, 3.125 nM, and 1.562 nM.

### Data Acquisition

After sample addition, the cell plates were placed in an Incucyte for continuous imaging every 2 h. Continuous imaging was performed, and data were collected to analyze cell confluency, so as to evaluate the killing ability of the drugs on the cells.

### Experimental Results

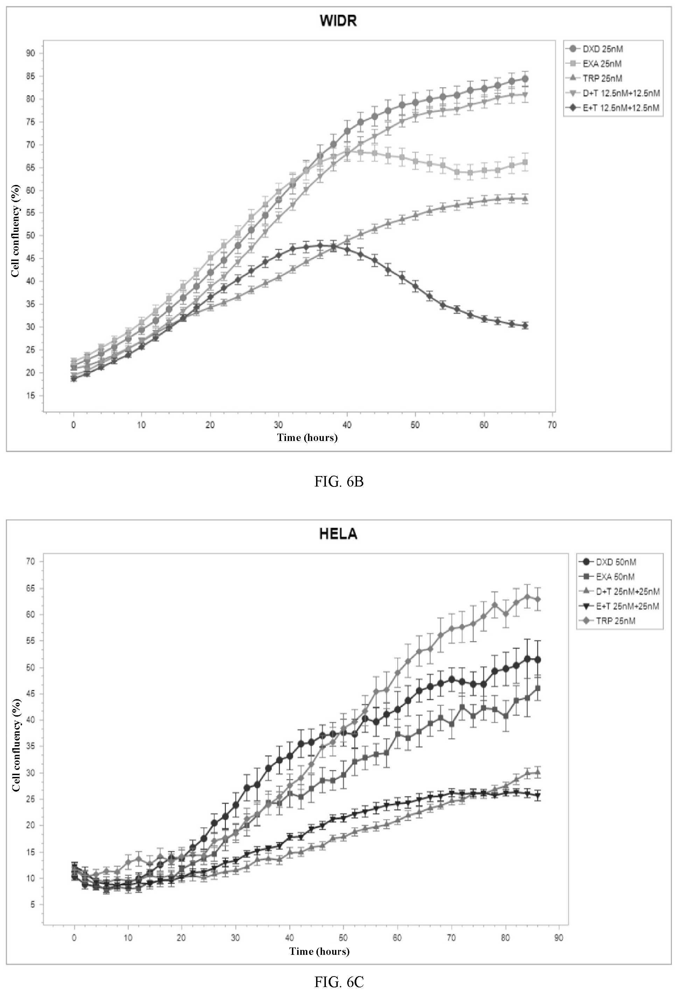

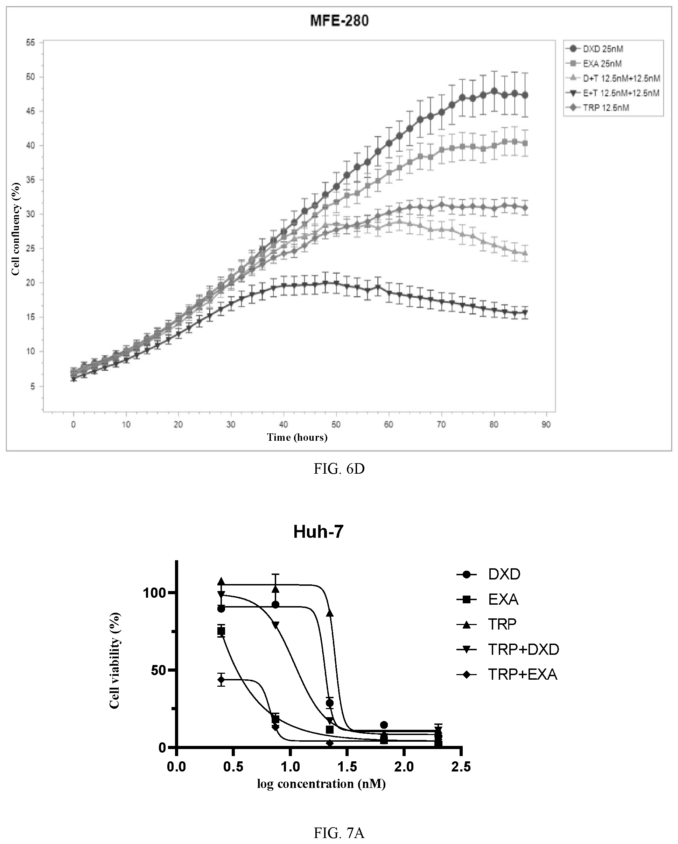

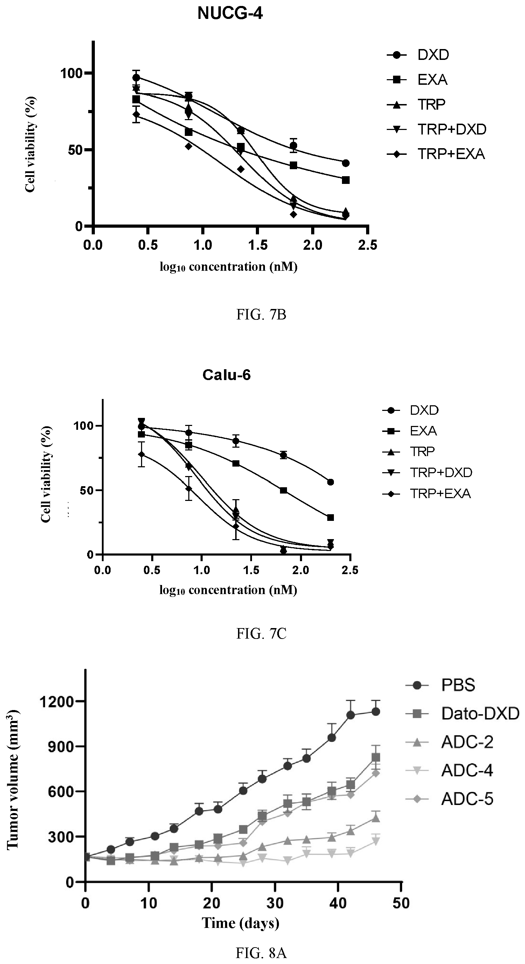

The confluency of MX-1 cells, WIDR cells, HeLa cells, and MFE-280 cells is shown in FIGs. 6A-6D, respectively. The viabilities of HuH-7 cells, NUGC-4 cells, and Calu-6 cells are shown in FIGs. 7A-7C, respectively. It can be seen from the results that the groups of Dxd or Exatecan in combination with Triptolide achieved more significant cell killing compared to the groups of Dxd, Exatecan, or Triptolide used alone.

### Example 17. Tumor Inhibition Effect in Human Colorectal Cancer Drug-Resistant Model (HCT-15-TROP2 Cells)

### Experimental Animals

NCG mice were purchased from GemPharmatech (Chengdu) Co., Ltd.

### Animal Modeling

HCT-15-Trop2 cells were resuspended in a RPMI 1640 culture medium or DMEM culture medium (total volume: 0.1 mL/mouse). The hair on the right anterior dorsal region of the experimental NCG mice was removed, followed by subcutaneous inoculation of 1 × 10⁷ cells.

### Administration Regimen

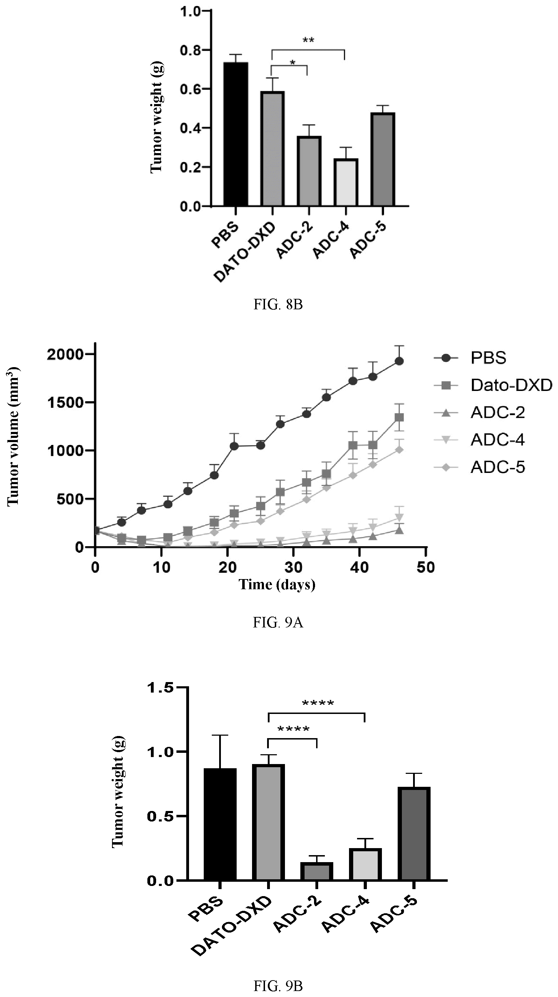

Test samples (negative control: a PBS solution; test drugs: ADC-2, ADC-4, and ADC-5; positive control: Dato-Dxd) were administered to the animals (7 animals per group) once every two weeks via tail vein injection. The day of administration was recorded as D0, and a total of three administrations were given (Day 0, Day 14, and Day 29) at an administration dose of 5 mg/kg. After the start of administration, the body weight and tumor size of the mice were measured twice a week. Calculation formula for tumor volume: Tumor volume (mm³) = 1/2 × (L × W²) (where L represents the long diameter and W represents the short diameter). The results of changes in the tumor volume are shown in FIG. 8A. After the experiment was completed, the mice were sacrificed, and the tumors were dissected and weighed. The results are shown in FIG. 8B (* indicates P < 0.05, ** indicates P < 0.01, *** indicates P < 0.001, and **** indicates P < 0.0001).

### Example 18. Tumor Inhibition Effect in Human Lung Cancer NCI-H2170 Cell Model

### Experimental Animals

NCG mice were purchased from GemPharmatech (Chengdu) Co., Ltd.

### Animal Modeling

NCI-H2170 cells were resuspended in a RPMI 1640 culture medium or DMEM culture medium (total volume: 0.1 mL/mouse). The hair on the right anterior dorsal region of the experimental NCG mice was removed, followed by subcutaneous inoculation of 1 × 10⁷ cells.

### Administration Regimen

Test samples (negative control: a PBS solution; test drugs: ADC-2, ADC-4, and ADC-5; positive control: Dato-Dxd) were administered to the animals (6 animals per group) via tail vein injection. The day of administration was recorded as D0, and a single administration was given (Day 0) at an administration dose of 5 mg/kg. After the start of administration, the body weight and tumor size of the mice were measured twice a week. Calculation formula for tumor volume: Tumor volume (mm³) = 1/2 × (L × W²) (where L represents the long diameter and W represents the short diameter). The results of changes in the tumor volume are shown in FIG. 9A. After the experiment was completed, the mice were sacrificed, and the tumors were dissected and weighed. The results are shown in FIG. 9B (* indicates P < 0.05, ** indicates P < 0.01, *** indicates P < 0.001, and **** indicates P < 0.0001).

### Example 19. Preparation of Exatecan (Compound 6) ADC (ADC-6)

The TCEP was directly added to the protein solution at a molar ratio of TCEP to the antibody (sacituzumab) of 4:1 to reduce the antibody. The mixture was shaken in a constant-temperature shaker at 30°C for 2 h for antibody reduction. Compound 6 was added to complete sulfhydryl conjugation, with the molar ratio of compound 6 to the antibody being 5:1. The mixture was stirred at 2-8°C for 2 h to complete sulfhydryl toxin conjugation. Substances, including DMSO and compound 6, introduced during the process were removed via concentration and buffer exchange. The sample was exchanged into a 4.29 g/L MES buffer (pH 6.5), and the protein concentration was adjusted to 20 mg/mL. Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, and ultrapure water was supplemented to make a final volume of 100 µL. The mixture was then directly detected by LC-MS. After normalization according to mass spectrometry signal intensities, the average DAR value of the sample was calculated. The DAR value of exatecan was 3.9.

### Example 20. Preparation of Exatecan (Compound 6) ADC (ADC-7)

The TCEP was directly added to the protein solution at a molar ratio of TCEP to the antibody (sacituzumab) of 8:1 to reduce the antibody. The mixture was shaken in a constant-temperature shaker at 30 °C for 2 h for antibody reduction. Compound 6 was added to complete sulfhydryl conjugation, with the molar ratio of compound 6 to the antibody being 10:1. The mixture was stirred at 2-8°C for 2 h to complete sulfhydryl toxin conjugation. Substances, including DMSO and compound 6, introduced during the process were removed via concentration and buffer exchange. The sample was exchanged into a 4.29 g/L MES buffer (pH 6.5), and the protein concentration was adjusted to 20 mg/mL. Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, and ultrapure water was supplemented to make a final volume of 100 µL. The mixture was then directly detected by LC-MS. After normalization according to mass spectrometry signal intensities, the average DAR value of the sample was calculated. The DAR value of exatecan was 7.9.

### Example 21. Preparation of Triptolide (Compound 3) ADC (ADC-8)

The samples were added according to the following reaction system to enable the final concentration of the antibody (sacituzumab) to be 10 mg/mL, the final concentration of MnCl₂ to be 10 mmol/L, the final concentration of Tris-HCl (pH 7.5) to be 10 mmol/L, and the final concentration of UDP-GalNAz to be 5 mmol/L. GalT1 was added at a ratio of 5 mg of GalT1/1 g of mAb to 50 mg of GalT1/1 g of mAb. The mixture was shaken in a constant-temperature shaker at 30°C for ≥12 h to complete the azidation reaction of the antibody. Substances, including UDP-GalNAz, GalT1, and Mn²⁺, introduced during the azidation reaction were removed via chromatography. Compound 3 was added according to the following reaction system to enable the final concentration of the azidated antibody to be 10 mg/mL, the final concentration of DMSO to be 10%, and the molar ratio of compound 3 to the antibody to be 5-15. The mixture was shaken in a constant-temperature shaker at 30°C for ≥12 h to complete glycosyl conjugation between the antibody and the toxin. Substances, including DMSO and compound 3, introduced during the process were removed via concentration and buffer exchange. The sample was exchanged into a 4.29 g/L MES buffer (pH 6.5), and the protein concentration was adjusted to 20 mg/mL. Trehalose and polysorbate 80 (II) were supplemented to enable the final concentration of trehalose to be 8.56 g/L and the final concentration of polysorbate 80 (II) to be 0.1 g/L. Thus, the sample was prepared. 100 µg of the sample was taken, and ultrapure water was supplemented to make a final volume of 100 µL. The mixture was then directly detected by LC-MS. After normalization according to mass spectrometry signal intensities, the average DAR value of the sample was calculated. The DAR value of triptolide was 3.7.

### Example 22. Animal Efficacy Experiment on NCI-H292 CDX Model

### Experimental Animals

NKG mice were purchased from Cyagen Biosciences Co., Ltd.

### Animal Modeling

NCI-H292 cells were resuspended in a RPMI 1640 culture medium or DMEM culture medium at a density of 1×10⁸ cells/mL. The hair on the right anterior dorsal region of the experimental mice was removed, followed by subcutaneous inoculation of 0.1 mL of cell suspension/mouse. The inoculation density was 1 × 10⁷ NCI-H292 cells/mouse, and there were 8 animals in each group.

### Administration Regimen

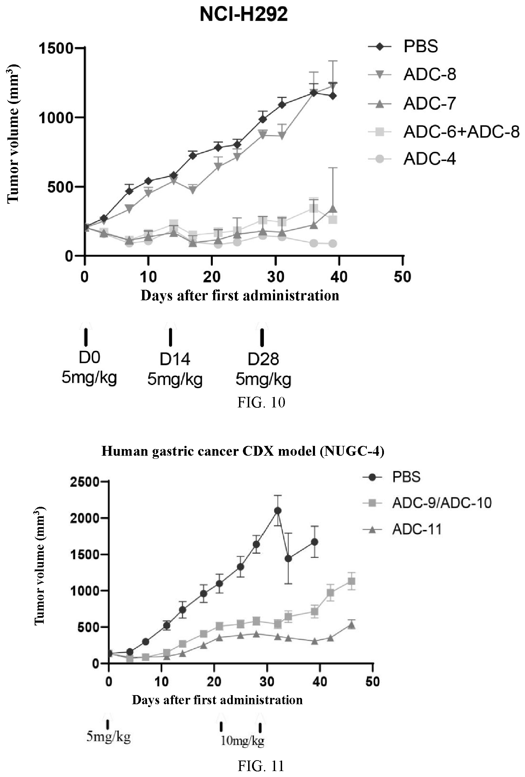

Test samples (negative control: a PBS solution; test drugs: the ADC8 group, the ADC-7 group, the ADC8 + ADC6 group, and the ADC-4 group) were administered to the animals via tail vein injection. The day of administration was recorded as D0, and a total of three administrations were given (Day 0, Day 14, and Day 28). The administration doses were as follows: the ADC8 group (5 mg/kg), the ADC-7 group (5 mg/kg), the ADC8 + ADC6 group (2.5 mg/kg + 2.5 mg/kg), and the ADC-4 group (5 mg/kg). After the start of administration, the body weight and tumor size of the mice were measured twice a week. Calculation formula for tumor volume: Tumor volume (mm³) = 1/2 × (L × W²) (where L represents the long diameter and W represents the short diameter). The results of changes in the tumor volume are shown in FIG. 10. On D43, the observation was stopped, and the experiment was terminated. The mortality of the mice was counted. The results showed that in the ADC-4 group, 5 mice survived and 3 died; in the ADC-7 group, 7 mice died, and in the PBS control group, 7 mice died. The results are shown in the table below.

As can be seen from the results, the efficacy of the ADC8 + ADC6 (triptolide-ADC and exatecan-ADC) combination group was similar to that of the ADC-7 and ADC-4 groups after the first two administrations. After the third administration, the ADC-4 group showed the best efficacy, and based on the statistics of mouse death time and number, it also exhibited the best safety.

**Table 1**

| Group | Number of dead mice (number of surviving mice)/mouse | | | | |
|---|---|---|---|---|---|
| | Day 0 | Day 10 | Day 20 | Day 30 | Day 42 |
| Negative control group | 0 (8) | 0 (8) | 0 (8) | 1 (7) | 7 (1) |
| ADC-7 group | 0 (8) | 0 (8) | 1 (7) | 2 (6) | 7 (1) |
| ADC-4 group | 0 (8) | 0 (8) | 0 (8) | 1 (7) | 3 (5) |

### Example 23. Preparation of Dxd-ADC (ADC-9)

This preparation process included antibody reduction → sulfhydryl conjugation → concentration and buffer exchange. The antibody (patritumab) was reduced with TCEP. TCEP was added at a molar ratio of TCEP to the antibody of 4:1. The reduction reaction was carried out under shaking in a constant-temperature shaker at 22°C for a reaction time of 2 h. After the antibody reduction reaction was completed, the small-molecule toxin (compound 8) bearing a maleimide linker was added to the antibody for sulfhydryl conjugation. The small-molecule payload (Dxd) was added at a molar ratio of the small molecule to the antibody of 6:1. The sulfhydryl conjugation reaction was carried out under shaking in a constant-temperature shaker at 22°C for a reaction time of 2 h. Subsequently, impurities, including DMSO, TCEP, and small-molecule payload, introduced during the process were removed via concentration and buffer exchange. After normalization according to mass spectrometry signal intensities, the average DAR value of the sample was calculated. The DAR value of Dxd was 5.63.

### Example 24. Preparation of Dxd-ADC (ADC-10)

This preparation process included antibody reduction → sulfhydryl conjugation → concentration and buffer exchange. The antibody (patritumab) was reduced with TCEP. TCEP was added at a molar ratio of TCEP to the antibody of 20:1. The reduction reaction was carried out under shaking in a constant-temperature shaker at 22°C for a reaction time of 2 h. After the antibody reduction reaction was completed, the small-molecule toxin (compound 8) bearing a maleimide linker was added to the antibody for sulfhydryl conjugation. The small-molecule payload (Dxd) was added at a molar ratio of the small molecule to the antibody of 20:1. The sulfhydryl conjugation reaction was carried out under shaking in a constant-temperature shaker at 22°C for a reaction time of 2 h. Subsequently, impurities, including DMSO, TCEP, and small-molecule payload, introduced during the process were removed via concentration and buffer exchange. After normalization according to mass spectrometry signal intensities, the average DAR value of the sample was calculated. The DAR value of Dxd was 7.93.

### Example 25. Preparation of Exatecan (Compound 6)/Triptolide (Compound 3) Double-Toxin ADC (ADC-11)

The preparation process included antibody azidation → protein A affinity chromatography → glycosyl conjugation → antibody reduction → sulfhydryl conjugation → concentration and buffer exchange. The samples were added according to the following reaction system to enable the final concentration of the antibody (patritumab) to be 10 mg/mL, the final concentration of Tris-HCl (pH 7.5) to be 10 mmol/L, and the final concentration of UDP-GalNAz to be 5 mmol/L, and GalT1 was added at a mass ratio of GalT1 to the antibody (patritumab) of 40:1. The azidation reaction of the antibody was carried out under shaking in a constant-temperature shaker at 22°C for a reaction time of 14-16 h. Subsequently, impurities including GalT1, UDP-GalNAz, and Mn²⁺ added during the azidation reaction were removed via protein A affinity chromatography. Then, the antibody bearing an azide group and the small-molecule payload (compound 3) bearing a DBCO linker were added at a molar ratio of the small-molecule payload (compound 3) to the antibody of 7.5:1 to perform a click chemistry reaction. The glycosyl conjugation reaction of the antibody was carried out under shaking in a constant-temperature shaker at 22°C for a reaction time of 14-16 h, completing the glycosyl conjugation of the antibody to the small-molecule payload (compound 3). The glycosyl-conjugated antibody was reduced with TCEP. TCEP was added at a molar ratio of TCEP to the antibody of 3.7:1. The reduction reaction was carried out under shaking in a constant-temperature shaker at 22°C for a reaction time of 2 h. After the antibody reduction reaction was completed, the small molecule (compound 6) bearing a maleimide linker was added to the antibody for sulfhydryl conjugation. The small-molecule payload (compound 6) was added at a molar ratio of the small molecule to the antibody of 6:1. The sulfhydryl conjugation reaction was carried out under shaking in a constant-temperature shaker at 22°C for a reaction time of 2 h. Finally, impurities, including DMSO, TCEP, and small-molecule payload, introduced during the process were removed via concentration and buffer exchange. After normalization according to mass spectrometry signal intensities, the average DAR value of the sample was calculated. The DAR value of exatecan was 3.89, and the DAR value of triptolide was 3.78.

### Example 26. Preparation of Triptolide ADC (ADC-12)

The preparation process included antibody azidation → protein A affinity chromatography → glycosyl conjugation → concentration and buffer exchange. The samples were added according to the following reaction system to enable the final concentration of the antibody (patritumab) to be 10 mg/mL, the final concentration of Tris-HCl (pH 7.5) to be 10 mmol/L, and the final concentration of UDP-GalNAz to be 5 mmol/L, and GalT1 was added at a mass ratio of GalT1 to the antibody (patritumab) of 40:1. The azidation reaction of the antibody was carried out under shaking in a constant-temperature shaker at 22°C for a reaction time of 14-16 h. Subsequently, impurities including GalT1, UDP-GalNAz, and Mn²⁺ added during the azidation reaction were removed via protein A affinity chromatography. Then, the antibody bearing an azide group and the small-molecule payload (compound 3) bearing a DBCO linker were added at a molar ratio of the small-molecule payload (compound 3) to the antibody of 5:1 to perform a click chemistry reaction. The glycosyl conjugation reaction of the antibody was carried out under shaking in a constant-temperature shaker at 22°C for a reaction time of 14-16 h, completing the glycosyl conjugation of the antibody to the small-molecule payload (compound 3). Finally, impurities, including DMSO, TCEP, and small-molecule payload, introduced during the process were removed via concentration and buffer exchange. After normalization according to mass spectrometry signal intensities, the average DAR value of the sample was calculated. The DAR value of triptolide was 3.9.

### Example 27. Efficacy in Subcutaneous Tumor-Bearing Model of NUGC-4 Human Gastric Cancer Cells in NCG Mice

### Experimental Animals

NCG mice were purchased from GemPharmatech (Chengdu) Co., Ltd.

### Animal Modeling

NUGC-4 cells were cultured in a culture solution containing RPMI 1640 + 10% FBS + 1% P/S. NUGC-4 cells in the exponential growth phase were collected, resuspended in a RPMI 1640 serum-free medium, and adjusted to a cell density of 1 × 10⁸ cells/mL. The cells were aliquoted into 1.5-mL EP tubes at 1 mL/tube and kept on ice for later use. The cells were resuspended in a RPMI 1640 serum-free medium (total volume: 0.1 mL/mouse). The hair on the right anterior dorsal region of the experimental mice was removed, followed by subcutaneous inoculation of 1 × 10⁷ NUGC-4 cells. There were 8 animals in each group.

### Administration Regimen

Test samples (negative control: a PBS solution; test drugs: the ADC-9 group or ADC-10, and the ADC-11 group) were administered to the animals via tail vein injection. The day of administration was recorded as Day 0, and a total of three administrations were given (for the ADC-9/ADC-10 group, ADC-9 was administered on Day 0, and ADC-10 was administered on Day 21 and Day 28). The administration doses were as follows: 5 mg/kg was administered on Day 0, and 10 mg/kg was administered on Day 21 and Day 28. After the start of administration, the tumor size of the mice was measured twice a week. Calculation formula for tumor volume: Tumor volume (mm³) = 1/2 × (L × W²) (where L represents the long diameter and W represents the short diameter). The results of changes in the tumor volume are shown in FIG. 11.

### Example 28. Efficacy in Subcutaneous Tumor-Bearing Model of Colon Cancer COLO205 Cells

### Experimental Animals

C-NKG mice were purchased from Cyagen Biosciences Co., Ltd.

### Animal Modeling

COLO205 cells were cultured in a culture solution containing RPMI 1640 + 10% FBS. COLO205 cells in the exponential growth phase were collected, resuspended in a RPMI 1640 serum-free medium, and adjusted to a cell density of 1 × 10⁸ cells/mL. The cells were aliquoted into 1.5-mL EP tubes at 1 mL/tube and kept on ice for later use. The hair on the right anterior dorsal region of the experimental mice was removed, followed by subcutaneous inoculation of 0.1 mL of cell suspension/mouse. The inoculation density was 1 × 10⁷ NCI-H2170 cells/mouse. There were 8 animals in each group.

### Administration Regimen

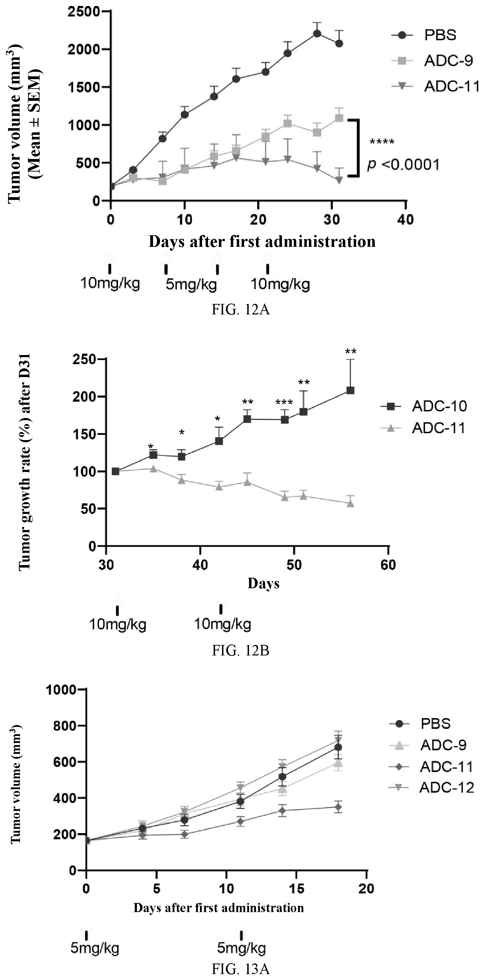

Test samples (negative control: a PBS solution; test drugs: the ADC9 group and the ADC11 group) were administered to the animals via tail vein injection. The day of administration was recorded as D0, and a total of four administrations were given (Day 0, Day 7, Day 14, and Day 21). The administration doses were as follows: 10 mg/kg was administered on Day 0, 5 mg/kg was administered on Day 7 and Day 14, and 10 mg/kg was administered on Day 21. After the start of administration, the tumor size of the mice was measured twice a week. Calculation formula for tumor volume: Tumor volume (mm³) = 1/2 × (L × W²) (where L represents the long diameter and W represents the short diameter). The results of changes in the tumor volume are shown in FIG. 12A.

On Day 31 after administration, the animals in the ADC9 group were evenly divided into two groups, which were administered ADC-10 and ADC-11, respectively, at a dose of 10 mg/kg. The changes in the tumor volume are shown in FIG. 12B. As can be seen from the figure, the tumors had developed resistance to ADC-9. After grouping and administration, the ADC-11 group was able to inhibit tumor growth, exhibiting a significant inhibition effect on tumor growth, whereas the ADC-10 group remained unable to inhibit tumor growth.

### Example 29. Efficacy in Subcutaneous Tumor-Bearing Model of Lung Squamous Cell Carcinoma NCI-H2170 Cells

### Experimental Animals

C-NKG mice were purchased from Cyagen Biosciences Co., Ltd.

### Animal Modeling

NCI-H2170 cells were cultured in a culture solution containing RPMI 1640 + 20% FBS. NCI-H2170 cells in the exponential growth phase were collected, resuspended in a RPMI 1640 serum-free medium, and adjusted to a cell density of 1 × 10⁸ cells/mL. The cells were aliquoted into 1.5-mL EP tubes at 1 mL/tube and kept on ice for later use. The hair on the right anterior dorsal region of the experimental mice was removed, followed by subcutaneous inoculation of 0.1 mL of cell suspension/mouse. The inoculation density was 1 × 10⁷ NCI-H2170 cells/mouse. There were 8 animals in each group.

### Administration Regimen

Test samples (negative control: a PBS solution; test drugs: the ADC-9 group, the ADC-11 group, and ADC-12) were administered to the animals via tail vein injection. The day of administration was recorded as D0, and a total of 2 administrations were given (Day 0 and Day 10). The administration dose was 5 mg/kg. After the start of administration, the tumor size of the mice was measured twice a week. Calculation formula for tumor volume: Tumor volume (mm³) = 1/2 × (L × W²) (where L represents the long diameter and W represents the short diameter). The results of changes in the tumor volume are shown in FIG. 13A.

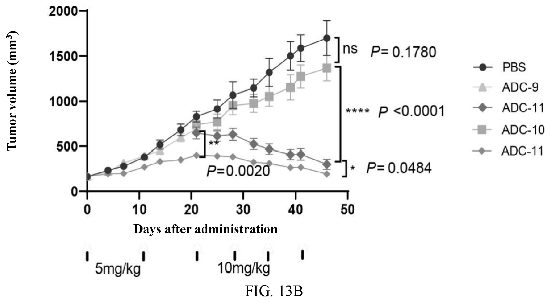

On Day 21 after the first administration, the 16 mice in the original ADC-9 group were evenly divided into two groups based on tumor volume, which were administered ADC-10 and ADC-11, respectively, at 10 mg/kg once a week. The PBS group and the original ADC-11 group also continued to receive administration at 10 mg/kg once a week. The changes in the tumor volume are shown in FIG. 13B. As can be seen from the figure, under the once-weekly administration regimen, ADC-10 showed no significant inhibition effect on tumor growth, whereas the ADC-11 group was able to significantly reduce tumor size.

## Claims

1. Use of a combination of triptolide and a camptothecin drug in preparing a medicament for treating a tumor disease.

2. The use according to claim 1, wherein triptolide and the camptothecin drug are used together as toxins in an antibody-drug conjugate.

3. An antibody-drug conjugate comprising double toxins, wherein the antibody-drug conjugate has a formula of: 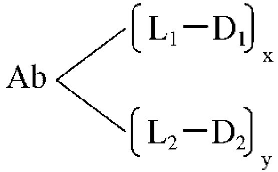
wherein Ab is an antibody or an antigen-binding fragment thereof;
D₁ and D₂ are selected from the group consisting of triptolide and a camptothecin drug, and D₁ and D₂ are different;
L₁ and L₂ are linking units;
x and y are 0-8.

4. The antibody-drug conjugate according to claim 3, wherein L₁ is linked to Ab via a sulfhydryl or amide group.

5. The antibody-drug conjugate according to claim 3 or 4, wherein L₂ is linked to Ab via glycosyl.

6. The antibody-drug conjugate according to any one of claims 3 to 5, wherein:
L₁ has a structure represented by [H₁-L₁ₐ-L_{1b}-L_{1c}-D₁]ₓ;
L₂ has a structure represented by [H₂-L₂ₐ-L_{2b}-L_{2c}-D₂]_{y};
H₁ and H₂ are linker moieties for Ab or groups capable of reacting with Ab;
L₁ₐ is a linking unit that links H₁ and L_{1b}, and L₂ₐ is a linking unit that links H₂ and L_{2b};
L_{1b} is a linker that links L₁ₐ and L_{1c}, and L_{2b} is a linker that links L₂ₐ and L_{2c};
L_{1c} is a spacer between L_{1b} and D₁, and L_{2c} is a spacer between L_{2b} and D₂.

7. The antibody-drug conjugate according to claim 6, wherein H₁ and H₂ are selected from the group consisting of: 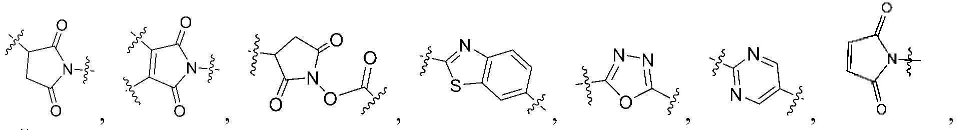 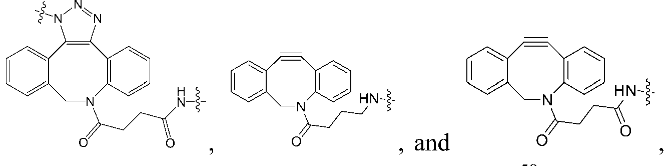 wherein 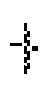 represents a linking site.

8. The antibody-drug conjugate according to claim 7, wherein H₁ is selected from the group consisting of: 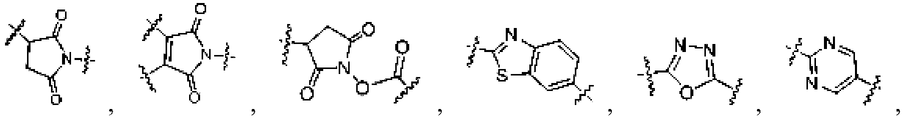 and 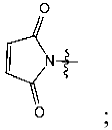 preferably, H₁ is

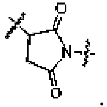

9. The antibody-drug conjugate according to claim 7, wherein H₂ is selected from the group consisting of:

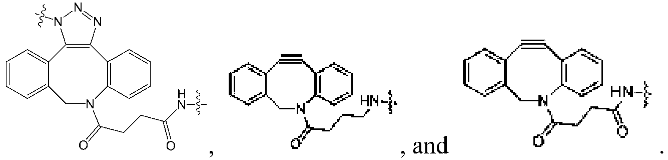

10. The antibody-drug conjugate according to any one of claims 6 to 9, wherein L₁ₐ or L₂ₐ comprises -L_{d}-C(O)-, wherein L_{d} is selected from the group consisting of optionally substituted alkylene, an optionally substituted polyethylene glycol group, optionally substituted alkenylene, optionally substituted alkynylene, optionally substituted aliphatic cyclylene, optionally substituted aliphatic heterocyclylene, optionally substituted arylene, optionally substituted heteroarylene, and a combination thereof; preferably, L_{d} is selected from the group consisting of optionally substituted C₁₋₃₀ alkylene, an optionally substituted polyethylene glycol group, optionally substituted C₂₋₃₀ alkenylene, optionally substituted C₂₋₃₀ alkynylene, optionally substituted C₃₋₃₀ aliphatic cyclylene, optionally substituted C₁₋₃₀ aliphatic heterocyclylene, optionally substituted C₆₋₃₀ arylene, optionally substituted C₅₋₃₀ heteroarylene, and a combination thereof.

11. The antibody-drug conjugate according to claim 10, wherein L_{d} is selected from the group consisting of -(CH₂)m-, -(PEG)n-, and -(CH₂)ₘ-(PEG)ₙ-(CH₂)_{z}-, wherein m, n, and z are integers of 0-10, and preferably, m, n, and z are integers of 0-8.

12. The antibody-drug conjugate according to any one of claims 6 to 11, wherein L_{1b} or L_{2b} is a cleavable linker or a non-cleavable linker.

13. The antibody-drug conjugate according to claim 12, wherein L_{1b} or L_{2b} is a cleavable peptide chain consisting of 2-10 amino acids; preferably, L_{1b} or L_{2b} is selected from the group consisting of Gly-Gly-Phe-Gly, Val-Cit, Val-Ala, Val-Lys, Val-Arg, AcLys-Val-Cit, AcLys-Val-Ala, Phe-Cit, Phe-Ala, Phe-Lys, Phe-Arg, Glu-Val-Ala, Glu-Val-Cit, Ala-Lys, Leu-Cit, Ile-Cit, Trp-Cit, Ala-Ala-Asn, Ala-Ala-Ala, Ala-Asn, and Lys; preferably, L_{1b} or L_{2b} is selected from the group consisting of Gly-Gly-Phe-Gly, Val-Cit, Val-Ala, Val-Lys, Phe-Cit, Phe-Ala, Phe-Lys, and Ala-Ala-Asn.

14. The antibody-drug conjugate according to any one of claims 6 to 13, wherein L_{1c} or L_{2c} is selected from the group consisting of: 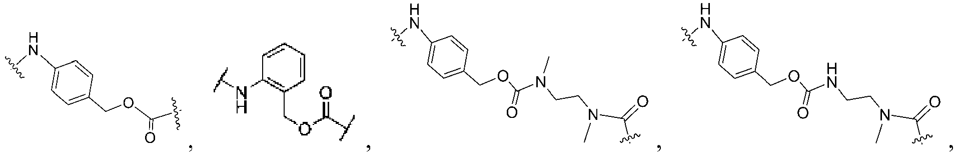 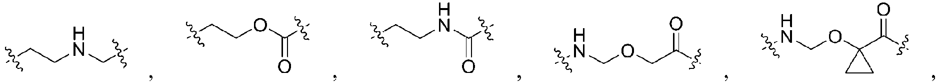 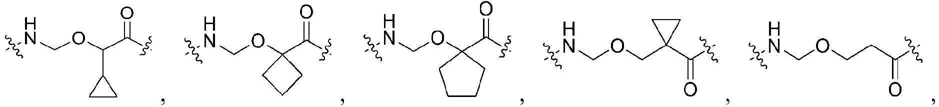 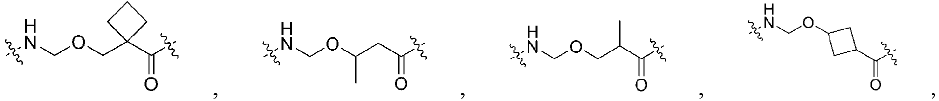 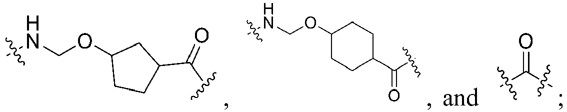 preferably, L_{1c} is selected from the group consisting of 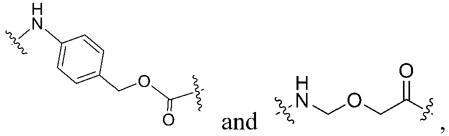 wherein 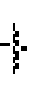 represents a linking site.

15. The antibody-drug conjugate according to any one of claims 3 to 14, wherein L₁-D₁ is 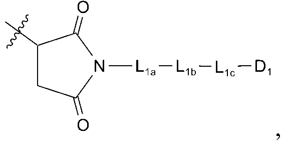 wherein
L₁ₐ is -(CH₂)ₘ₁-C(O)- or -(PEG)ₙ₁-(CH₂)_{z1}-C(O)-, wherein m₁, n₁, and z₁ are integers of 2-8;
L_{1b} is Gly-Gly-Phe-Gly, Val-Cit, or Val-Ala;
L_{1c} is 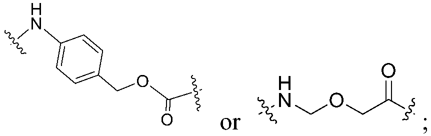
D₁ is triptolide or a camptothecin drug.

16. The antibody-drug conjugate according to any one of claims 3 to 15, wherein L₂-D₂ is 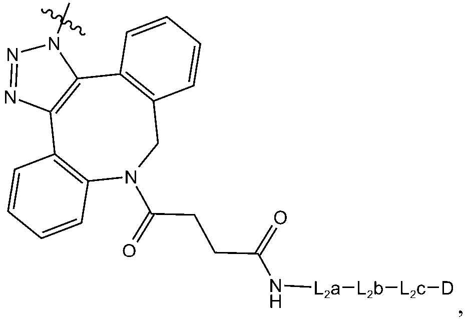 wherein:
L₂ₐ is -(PEG)ₙ₂-(CH₂)_{z2}-C(O)-, wherein n₂ and z₂ are integers of 2-8;
L_{2b} is Gly-Gly-Phe-Gly, Val-Cit, or Val-Ala;
L_{2c} is 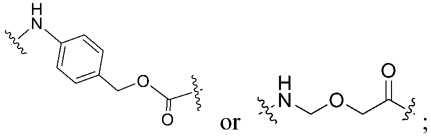
D₂ is triptolide or a camptothecin drug.

17. The antibody-drug conjugate according to claim 15, wherein L₁ₐ is -(CH₂)ₘ₁-C(O)-, wherein m₁ is an integer of 2-6, preferably 5; or L₁ₐ is -(PEG)ₙ₁-(CH₂)_{z1}-C(O)-, wherein n₁ and z₁ are integers of 2-6, and preferably, n₁ and z₁ are 2.

18. The antibody-drug conjugate according to claim 16, wherein L₂ₐ is -(PEG)ₙ₂-(CH₂)_{z2}-C(O)-, wherein n₂ and z₂ are integers of 2-6; or L₁ₐ is -(PEG)ₙ₁-(CH₂)_{z1}-C(O)-, wherein n₁ and z₁ are integers of 2-6; preferably, n₂ is an integer of 4-6, and z₂ is 2.

19. The antibody-drug conjugate according to claim 3, wherein D₁ is triptolide, and D₂ is a camptothecin drug.

20. The antibody-drug conjugate according to claim 3, wherein D₁ is a camptothecin drug, and D₂ is triptolide.

21. The antibody-drug conjugate according to any one of claims 3 to 20, wherein the camptothecin drug is camptothecin, exatecan, topotecan, SN38, or a derivative thereof; preferably, the camptothecin drug is exatecan.

22. The antibody-drug conjugate according to claim 3, wherein x is selected from 2-8, preferably 3-5; y is selected from 3-4.

23. The antibody-drug conjugate according to any one of claims 3 to 14, wherein L₁-D₁ is selected from the group consisting of:

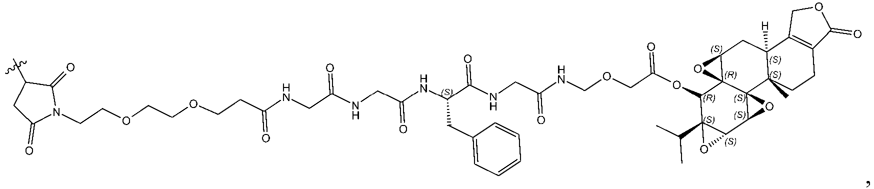

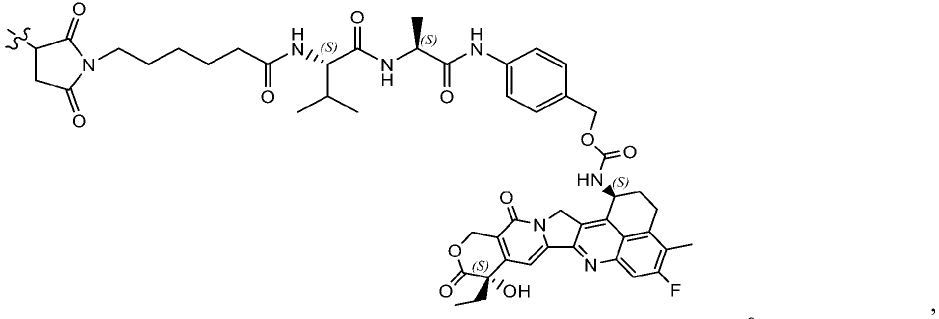

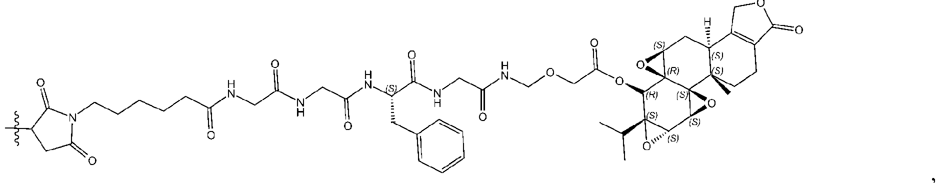

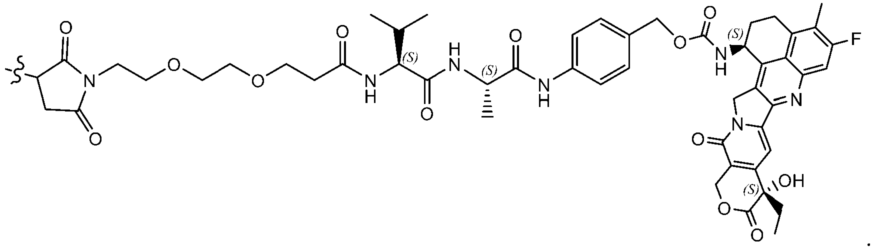

24. The antibody-drug conjugate according to any one of claims 3 to 14, wherein L₂-D₂ is selected from the group consisting of:

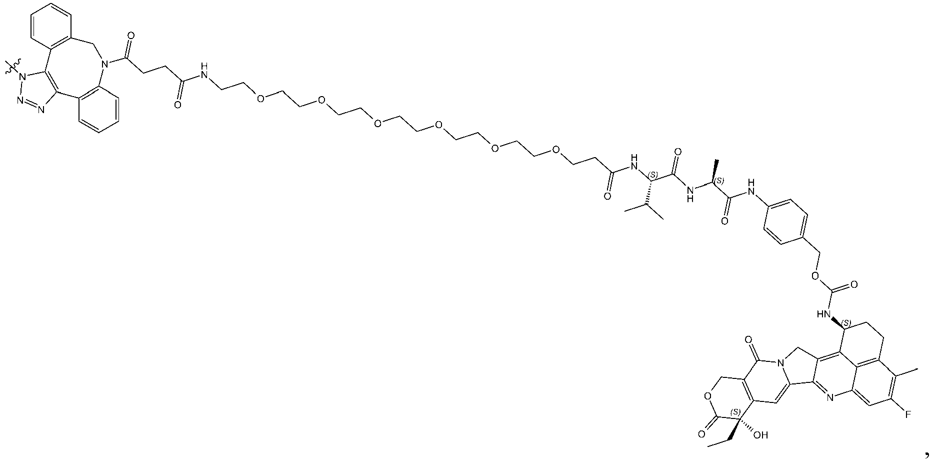

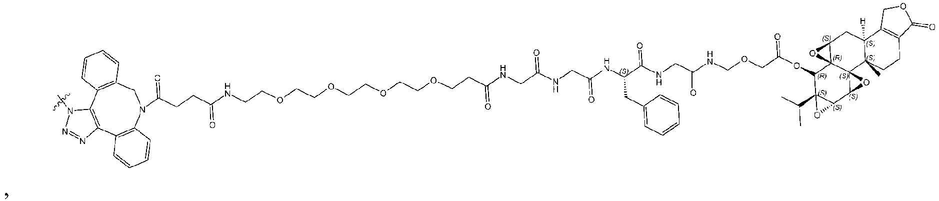

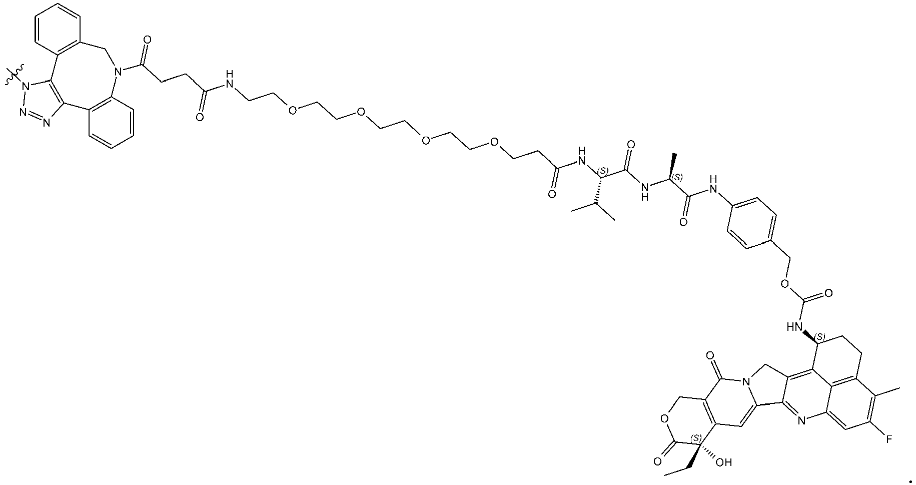

25. The antibody-drug conjugate according to any one of claims 3 to 14, wherein L₁-D₁ is: 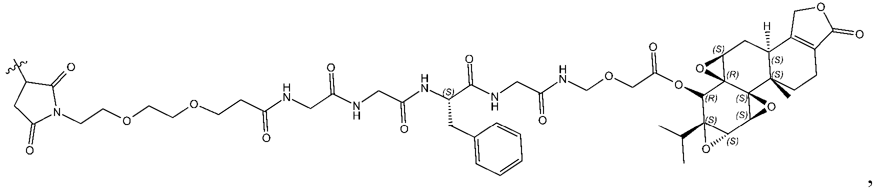 and
L₂-D₂ is: 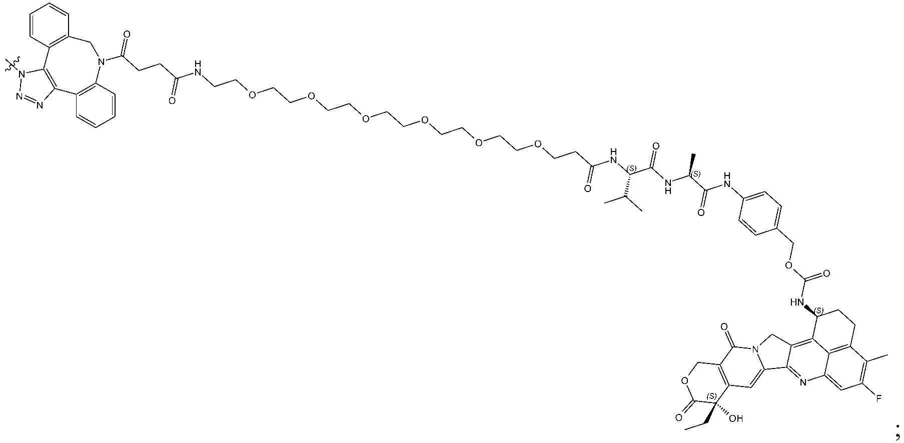
or L₁-D₁ is: 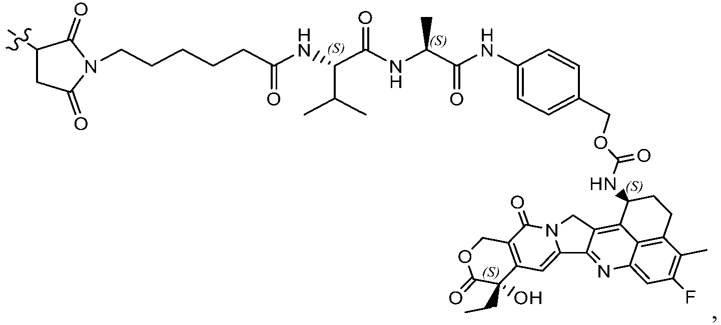 and
L₂-D₂ is: 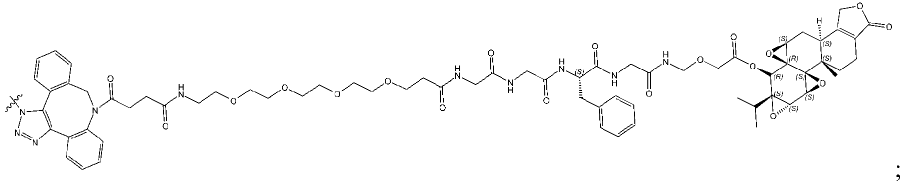
or L₁-D₁ is: 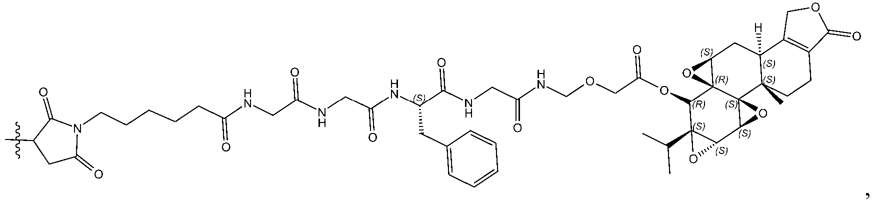 and
L₂-D₂ is: 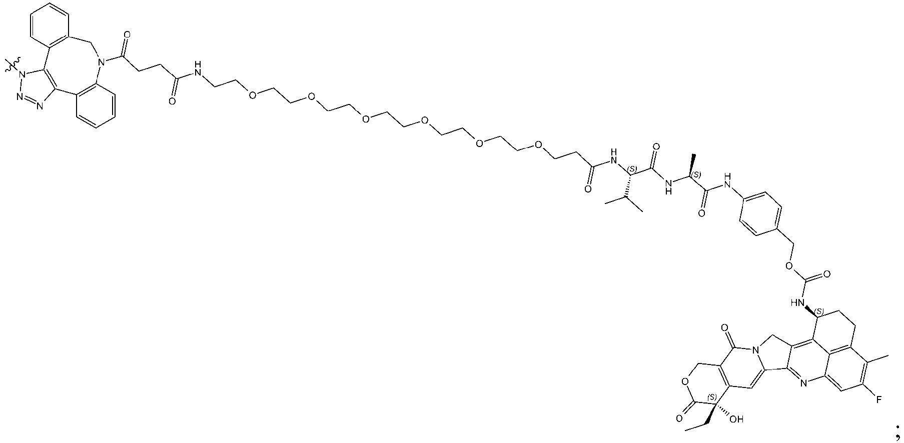
or L₁-D₁ is: 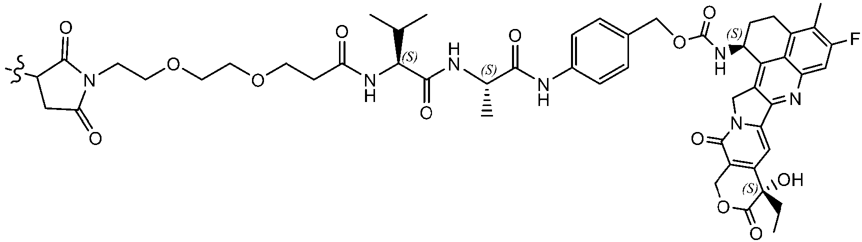 and
L₂-D₂ is:

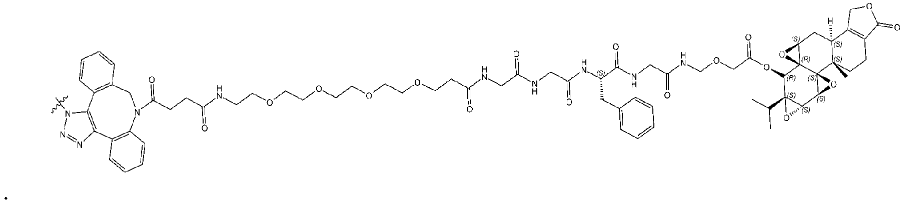

26. The antibody-drug conjugate according to any one of claims 3 to 25, wherein L₂ is linked to the antibody via an oligosaccharide.

27. The antibody-drug conjugate according to any one of claims 3 to 26, wherein the antibody or the antigen-binding fragment binds to one or more selected from the following: carbonic anhydrase IX, α-fetoprotein, α-actinin, A3, A33, ART 4, B7, B7H3, B7H4, BAGE, a BrE3 antigen, CA125, CAMEL, CAP", CASP-8/m, CCL19, CCL21, CD1, CDla.CD2, CD3, CD4, CD5, CD8, CD11A, CD14, CD15, CD16, CD18, CD19, CD20, CD21, CD22, CD23, CD24, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4, CDKN2A, HIF-Iα, colon-specific antigen p (CSAp), CEA, CEACAM5, CEACAM6, oMet, DAM, EGFR, EGFRvIII, cMet, EGP-1 (Trop-2), EGP-2, ELF2-M, Ep-CAM, Her2, Her3, Claudin 18.2, ROR1, ROR2, dll3, marcl7, a fibroblast growth factor (FGF), Flt-1, Flt-3, a folate receptor, a G250 antigen, GAGE, gp100, GRO-β, HLA-DR.HML24, HMGB-1, HSP70-2M., IGF-1R, IGR1R, MAGE, MAGE-3, MART-1, MART-2, NY-ESO-1, TRAG3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, a mucin in pancreatic cancer, a PD-1 receptor, a PD-L1 receptor, a placental growth factor, PLAGL2, prostatic acid phosphatase, PSA, PRAME, PSMA, P1GF, ILGF, ILGF-1R, RS5, RANTES, T101, SAGE, S100, survivin, survivin-2B, TAG, TAG-72, tenascin, a TRAIL receptor, a Tn antigen, ED-B, WT-1, and a 17-1A antigen; preferably, the antibody or the antigen-binding fragment binds to one or more selected from the group consisting of Her2, Her3, B7H3, Claudin 18.2, DLL-3, and EGP-1 (Trop-2); and
preferably, the antibody or the antigen-binding fragment is selected from the group consisting of epratuzumab, veltuzumab, sacituzumab, patritumab, trastuzumab, pertuzumab, abciximab, alemtuzumab, cetuximab, gemtuzumab, ibritumomab, panitumumab, rituximab, tositumomab, abagovomab, atlizumab, benralizumab, obinutuzumab, basiliximab, dadizumab, efalizumab, muromomab, natlizumab, omalizumab, gaiitenemmab, solanezumab, tisotumab, brentuximab, inotuzumab, moxetumomab, polatuzumab, enfortumab, belantamab, cetuximab, loncastuximab, daratumumab, nimotuzumab, zolbetuximab, omburtamab, and rovalpituzumab; more preferably, the antibody or the antigen-binding fragment is selected from the group consisting of sacituzumab, daratumumab, zolbetuximab, omburtamab, patritumab, and rovalpituzumab.

28. The antibody-drug conjugate according to any one of claims 3 to 27, wherein the antibody or the antigen-binding fragment comprises a heavy chain variable region and a light chain variable region, wherein the heavy chain variable region comprises three complementarity determining regions HCDR1-3, which comprise the amino acid sequences set forth in SEQ ID NOs: 1-3, respectively, and the light chain variable region comprises three complementarity determining regions LCDR1-3, which comprise the amino acid sequences set forth in SEQ ID NOs: 4-6, respectively; preferably, the heavy chain variable region of the antibody or the antigen-binding fragment thereof comprises the amino acid sequence set forth in SEQ ID NO: 7, and the light chain variable region comprises the amino acid sequence set forth in SEQ ID NO: 8; more preferably, a heavy chain of the antibody or the antigen-binding fragment thereof comprises the amino acid sequence set forth in SEQ ID NO: 9, and a light chain comprises the amino acid sequence set forth in SEQ ID NO: 10.

29. The antibody-drug conjugate according to any one of claims 3 to 27, wherein the antibody or the antigen-binding fragment comprises a heavy chain variable region and a light chain variable region, wherein the heavy chain variable region comprises three complementarity determining regions HCDR1-3, which comprise the amino acid sequences set forth in SEQ ID NOs: 11-13, respectively, and the light chain variable region comprises three complementarity determining regions LCDR1-3, which comprise the amino acid sequences set forth in SEQ ID NOs: 14-16, respectively; preferably, the heavy chain variable region of the antibody or the antigen-binding fragment thereof comprises the amino acid sequence set forth in SEQ ID NO: 17, and the light chain variable region comprises the amino acid sequence set forth in SEQ ID NO: 18; more preferably, a heavy chain of the antibody or the antigen-binding fragment thereof comprises the amino acid sequence set forth in SEQ ID NO: 19, and a light chain comprises the amino acid sequence set forth in SEQ ID NO: 20.

30. The antibody-drug conjugate according to any one of claims 3 to 29, wherein x + y ≥ 4, preferably, 4 ≤ x + y ≤ 9, and more preferably, 7 ≤ x + y ≤ 8.

31. A pharmaceutical composition, comprising the antibody-drug conjugate according to any one of claims 3 to 30, and a pharmaceutically acceptable carrier, excipient, or diluent.

32. Use of the antibody-drug conjugate according to any one of claims 3 to 30 in preparing a medicament for treating and/or preventing a tumor; preferably, the tumor is selected from tumors associated with the expression of one or more of the following molecules: Trop-2, Her2, Her3, B7H3, Claudin 18.2, CD30, CD38, CD33, CD70, and EGFR; preferably, the tumor comprises a solid tumor or a hematological tumor; more preferably, the tumor is selected from the group consisting of: lung cancer, kidney cancer, urethral cancer, colorectal cancer, prostate cancer, glioblastoma multiforme, ovarian cancer, pancreatic cancer, breast cancer, melanoma, liver cancer, bladder cancer, gastric cancer, leukemia, malignant lymphoma, cervical cancer, squamous cell carcinoma, penile cancer, esophageal cancer, and salivary gland cancer.

33. The antibody-drug conjugate according to any one of claims 3 to 30 for use in treating a tumor disease.

34. The antibody-drug conjugate according to claim 33, wherein triptolide and a camptothecin drug are used together as toxins in the antibody-drug conjugate.

35. A method for treating a tumor disease in an individual, comprising administering to the individual the antibody-drug conjugate according to any one of claims 3 to 30 or the pharmaceutical composition according to claim 31; preferably, the tumor is selected from tumors associated with the expression of one or more of the following molecules: Trop-2, Her2, Her3, B7H3, Claudin 18.2, CD30, CD38, CD33, CD70, and EGFR; preferably, the tumor comprises a solid tumor or a hematological tumor; more preferably, the tumor is selected from the group consisting of: lung cancer, kidney cancer, urethral cancer, colorectal cancer, prostate cancer, glioblastoma multiforme, ovarian cancer, pancreatic cancer, breast cancer, melanoma, liver cancer, bladder cancer, gastric cancer, leukemia, malignant lymphoma, cervical cancer, squamous cell carcinoma, penile cancer, esophageal cancer, and salivary gland cancer.
